# EUROPEAN PATENT SPECIFICATION

(11) **EP 4 648 772 B1**
(45) Date of publication and mention of the grant of the patent: **08.07.2026**
(21) Application number: 24701462.4
(22) Date of filing: 12.01.2024
(51) Int. Cl.: A61K 31/437, A61K 31/415, A61P 35/00, A61P 43/00

(54) **P38MAP KINASE INHIBITORS FOR USE IN THE ALLEVIATION OF CANCER IMMUNOTHERAPY-ASSOCIATED CYTOKINE RELEASE SYNDROME**
P38MAP-KINASE-INHIBITOREN ZUR VERWENDUNG BEI DER LINDERUNG DES MIT EINER KREBSIMMUNTHERAPIE ASSOZIIERTEN ZYTOKINFREISETZUNGSSYNDROMS
INHIBITEURS DE LA P38MAP KINASE POUR L'UTILISATION D'ATTÉNUATION DU SYNDROME DE LIBÉRATION DE CYTOKINE ASSOCIÉ À L'IMMUNOTHÉRAPIE ANTICANCÉREUSE

(30) Priority: 13.01.2023 GB 202300510; 13.01.2023 GB 202300512; 01.11.2023 GB 202316713; 01.11.2023 GB 202316714; 01.11.2023 GB 202316715
(43) Date of publication of application: 19.11.2025
(73) Proprietor: Poolbeg Pharma (UK) Limited, London E14 5NR (GB)
(72) Inventor: BUCKLEY, Brendan, London E1 2AX (GB); TREMBLE, Liam, London E1 2AX (GB)
(74) Representative: Abel & Imray LLP
(86) International application number: PCT/GB2024/050081
(87) International publication number: WO 2024/150014

(56) References cited:
- WO-A1-2019/122909
- WO-A1-2021/022186
- WO-A1-2021/195475
- SEARLE E: "Polb 001, an Oral Broad-Spectrum Anti-Inflammatory with the Potential to Prevent Cytokine Release Syndrome (CRS)", EMBASE, ELSEVIER SCIENCE PUBLISHERS, AMSTERDAM, NL, 28 November 2023 (2023-11-28), XP002811250
- PORTER DAVID ET AL: "Grading of cytokine release syndrome associated with the CAR T cell therapy tisagenlecleucel", JOURNAL OF HEMATOLOGY & ONCOLOGY, vol. 11, no. 1, 1 December 2018 (2018-12-01), XP093102069, Retrieved from the Internet <URL:https://jhoonline.biomedcentral.com/counter/pdf/10.1186/s13045-018-0571-y.pdf> DOI: 10.1186/s13045-018-0571-y
- TVEDT TOR HENRIK ANDERSON ET AL: "Cytokine Release Syndrome in the Immunotherapy of Hematological Malignancies: The Biology behind and Possible Clinical Consequences", JOURNAL OF CLINICAL MEDICINE, vol. 10, no. 21, 1 January 2021 (2021-01-01), pages 5190, XP093049778, Retrieved from the Internet <URL:https://www.ncbi.nlm.nih.gov/pmc/articles/PMC8585070/pdf/jcm-10-05190.pdf> DOI: 10.3390/jcm10215190
- DATABASE EMBASE [online] ELSEVIER SCIENCE PUBLISHERS, AMSTERDAM, NL; 28 November 2023 (2023-11-28), SEARLE E: "Polb 001, an Oral Broad-Spectrum Anti-Inflammatory with the Potential to Prevent Cytokine Release Syndrome (CRS)", XP002811250, Database accession no. EMB-002028765491
- SEARLE E: "Polb 001, an Oral Broad-Spectrum Anti-Inflammatory with the Potential to Prevent Cytokine Release Syndrome (CRS)", BLOOD 20231128 ELSEVIER B.V. NLD, vol. 142, no. Supplement 1, 28 November 2023 (2023-11-28), pages 2093 CONF 20231209 to 20231212 San Diego CA - 65th ASH, ISSN: 0006-4971

## Description

This application claims the benefit of: UK Patent Application No. 2300510.1 filed on 13 January 2023; UK Patent Application No. 2300512.7 filed on 13 January 2023; UK Patent Application No. 2316713.3 filed on 1 November 2023; UK Patent Application No. 2316714.1 filed on 1 November 2023; and UK Patent Application No. 2316715.8 filed on 1 November 2023.

### Field of the Disclosure

Cytokine release syndrome ("CRS") is a common side effect of cancer immunotherapy. The present disclosure concerns products and methods for preventing or reducing the severity of signs or symptoms of cancer immunotherapy-associated CRS. More particularly, but not exclusively, the disclosure concerns a p38 MAP kinase inhibitor for use in preventing or reducing the severity of signs or symptoms of cancer immunotherapy-associated CRS in a human patient, wherein the p38 MAPK inhibitor is administered prior to the onset of CRS. Also disclosed are related products, including pharmaceutical compositions and kits and related methods.

### Background of the Disclosure

In certain cancer immunotherapies, efficacy may depend upon the survival, function and phenotype of one or more populations of T cells in the patient. This may especially be the case for therapies which employ genetically engineered (or otherwise modified or cultured) T cells. Such a dependency is thought to be implicated in, for example, tumour infiltrating lymphocyte ("TIL") therapy, T cell checkpoint modulator therapy and chimeric antigen receptor ("CAR") T cell therapy.

Illustrating this, CAR T cell therapy relies on genetic modification of T cells to recognise, target and destroy cancer cells. T cells are harvested (often from the same patient, but T cells may alternatively be harvested from a healthy donor), genetically engineered to express CARs with specificity for cancer cell antigens (e.g., anti-CD19 or anti-BCMA CARs) and administered by infusion to a cancer patient ("adoptive transfer"). CAR T cells are then trafficked, via the circulation, to recognise antigenexpressing (e.g., CD19-expressing or BCMA-expressing) cancer cells. The survival, function and phenotype of the infused CAR T cells may thus drive therapeutic effects.

Meanwhile, in other kinds of cancer immunotherapy, the survival and memory function of particular T cells is thought not to be essential to the provision of desired therapeutic effects and turnover of a T cell population is not expected to result in total loss of therapeutic efficacy. Nevertheless, for the therapy to work, there must generally exist a population of T cells having adequate function and preferably a phenotype of benefit to the therapy concerned. This may especially be the case for T cell-engaging therapies, such for example as bispecific T-cell engager (BiTE) therapy, bifunctional checkpoint-inhibitory T cell engager (CiTE) therapy, simultaneous multiple interaction T cell engager (SMITe) therapy, therapy with other bispecific antibodies, trispecific killer engager (TriKE) therapy or therapy with other immunostimulatory molecules able to induce cytokine release.

Other cancer immunotherapies exist which, although not causally dependent on T cell survival, function and phenotype, may nevertheless benefit from enhanced T cell activity. Examples include adoptive immunotherapy with unmodified or genetically engineered natural killer (NK) cells and cancer vaccines.

The survival, function and phenotype of T cells may be modulated by four distinct signals. The first of these (commonly known as "signal 1") involves antigen recognition. Signal 1 is antigen-specific and occurs via stimulation of a T cell receptor ("TCR") upon binding to antigenic peptide complexed with a major histocompatibility complex ("MHC") and results in changes to the intracellular apparatus of the TCR. The second signal ("signal 2") involves co-stimulation and/or co-inhibition. Namely, signal 2 occurs via co-stimulatory signalling by co-stimulatory receptors on T cells with their corresponding ligands on antigen-presenting cells ("APCs"; namely, monocytes and dendritic cells); and/or co-inhibitory signalling by co-inhibitory receptors on T cells with their corresponding ligands on APCs.

Signal 2 may affect T cell polarisation. It will be understood that as used herein, the term "T cell polarisation" refers to the specialisation of T cells into sub-sets of T cells (phenotypes) which are restricted to producing a certain pattern or patterns of cytokines; such, for example, as a T1 pattern or a T2 pattern. When naive or resting memory T cells differentiate into effector T cells, they may produce a broad range of cytokines in a T0 pattern. When, for example, T cells are polarised so as to be restricted to a T1 pattern or a T2 pattern, the T1 pattern of cytokines may include interleukin (IL)-12 and interferon (IFN)y, while the T2 pattern of cytokines may include IL-4. Where signal 2 is not provided following signal 1, the result may be T cell unresponsiveness ("anergy").

A number of additional signals may further modulate the activity of T cells during or after activation. Thus, "signal 3" involves cytokines, such as interferon-α, IFNγ, IL-4, IL-10 and IL-12, and other extracellular factors *in vivo.* Signal 3 may ensure that T cells can behave in concert with their environment. Similarly to signal 2, signal 3 may affect T cell polarisation. Thus, signal 3 may involve cytokine-mediated differentiation and expansion of T cells.

Meanwhile, "signal 4" may involve post-activation events *in vivo* and may control the duration of the T cell response, having an impact on post-activation T cell phenotype, persistence and function. "Signal 4", although less conventional a term (in particular, less well established in the art than signal 1 or 2) is coined in recognition of the important phenomena involved. Signal 4 may, for example, involve cytokines, such as IFN-I, inducing monocyte-derived cells to provide a survival checkpoint for T cell activation .

The function of T cells may depend, *inter alia,* on the environmental stimuli provided thereto, such as signal 3 and signal 4, as described above. Signals 3 and 4 may contribute to T cell survival, expansion and antigen-specific cell-mediated cell death.

Once cytotoxic T cells recognise cognate antigen, they release perforin, granzymes and inflammatory cytokines. By way of example, the release of such cytokines by CAR T cells in CAR T cell therapy, is thought to cause a high level of pyroptotic cancer cell death (Liu et al., 2020, Sci. Immunol. 5:43). At the time of writing, CAR T cell therapy is approved by the US Food and Drug Administration as standard of care for some forms of aggressive, relapsed or refractory non-Hodgkin lymphoma, including: diffuse large B cell lymphoma, primary mediastinal B-cell lymphoma, high grade B-cell lymphoma, transformed follicular lymphoma and mantle cell lymphoma. CAR T-cell therapies are also approved for the treatment of B-cell acute lymphoblastic leukaemia and multiple myeloma. Further CAR-T therapies for the treatment of additional cancers are under review, including for haematological malignancies and solid tumours.

Efforts have been made further to potentiate the functionality of CAR T cells. In one study (Gurusamy et al., 2020, Cancer Cell 37, 818-833), an *in vitro* monoculture of CAR T cells was subjected to knockout of *Mapk14* encoding p38α kinase. *Mapk14* KO cells exhibited an increase in cell expansion and CD62L expression and a decrease in reactive oxygen species and γH2AX accumulation. The authors then attempted to mimic the phenotypic effects of genetic KO of *Mapk14* in a reversible manner in order to confine inhibition of p38 to *in vitro* expansion, using the p38 MAP kinase inhibitor Doramapimod/BIRB796. This was found, similarly, to promote desirable CAR T cell phenotypes *in vitro.* The Doramapimod was washed out before adoptive transfer.

Cancer immunotherapy, such, for example, as CAR T cell therapy or infusion of T cell-engaging agents, may result in significant toxicities, including CRS. CRS may clinically manifest from a day to several weeks after administration of the cancer immunotherapy. CRS may present with a variety of symptoms ranging from mild, flu-like symptoms to severe, life-threatening manifestations of an exaggerated systemic inflammatory response. In severe cases, and despite therapeutic intervention, CRS may lead to death (Shimabukuro-Vornhagen et al., 2018, J. Immunother. Cancer, 15 6(1):56).

CRS is thought to arise from on-target effects of CAR T cell therapy (Morris et al., 2022, Nat. Rev. Immunol. 22, 85-96). Monocytes may be activated by cytokines (such as IL-6 and/or TNFα) produced by T cells involved in cancer cell destruction. Additionally or alternatively, monocytes may be activated by contact-dependent engagement of T cell-expressed CD40 ligand with monocyte-expressed CD40. Additionally or alternatively, monocytes may be activated by damage-associated molecular patterns ("DAMPs") which are released by dying cancer cells and recognizable by pattern-recognition receptors ("PRRs") on monocytes.

Activated monocytes represent a major source of secreted cytokines (such as interleukin 6 (IL-6)) *in vivo.* Cytokines secreted by monocytes may signal to a range of non-immune cells, especially endothelial cells. The activation of endothelial cells can lead to vascular leakage, hypotension and further cytokine (such as interleukin 6 (IL-6) and interleukin 8 (IL-8)) secretion, thus resulting in amplification of the patient's inflammatory response.

Cytokines may attract circulating monocytes, leading to an accumulation of activated monocytes nearby to interactions between T cells and cancer cells, thus amplifying the inflammatory loop. Furthermore, the T cells may themselves overflow or redistribute, exacerbating an uncontrolled immune response.

In one study, the supernatants of an anti-CD19 CAR T cell/Nalm6-luc co-culture were used to stimulate endothelial (HUVEC) cells, and the p38 MAPK inhibitor SB203580 was applied to investigate the inhibition of endothelial activation (Chen et al., 2021, Front. Immunol., 12:623610).

Meanwhile, WO 2021/022186 A1 and WO 2021/195475 A1 (both to Aclaris Therapeutics Inc.) disclose methods of modulating a p38 MAP kinase-mediated function in a patient and it is postulated that in some embodiments, the methods could be used to treat CRS.

Current treatment of CRS is reactive, requiring evidence of the onset of the syndrome before treatment initiation. In those patients who develop CRS, physicians may employ a number of treatments. Tocilizumab (an IL-6 receptor antagonist) for example, optionally in combination with corticosteroids, has been indicated for the treatment of symptoms that follow onset of CRS. In patients refractory to tocilizumab and corticosteroids, anakinra (an IL-1 receptor antagonist) may be used.

At the time of writing, prophylaxis of CRS is currently not usual practice and there remains an urgent need for treatments to prevent cancer immunotherapy-associated CRS.

It would be advantageous for a therapy for CRS to be clinically tolerable (i.e., have no or low risk of side effects). Preferably, it would not lead to ablation of core immune function, in cancer immunotherapy patients already at risk of viral and bacterial infections. Preferably, it would be provided in a form suitable for outpatient administration.

The present disclosure seeks to alleviate one or more of these and other problems.

### Summary of the Disclosure

The invention is set out in the appended set of claims.

In a first aspect of the present disclosure, there is provided a p38 MAP kinase inhibitor for use in preventing or reducing the severity of signs or symptoms of cancer immunotherapy-associated cytokine release syndrome ("CRS") in a human patient, wherein the p38 MAPK inhibitor is administered prior to the onset of CRS.

As supported by Examples 5 and 6 (especially, Example 6) below, administration of a p38 MAPK inhibitor to a human patient prior to the onset of CRS may prevent or reduce the severity of signs or symptoms of cancer immunotherapy-associated CRS. Connectedly, the p38 MAPK inhibitor may inhibit cytokine release by all three of T cells, monocytes and endothelial cells in the environment of cancer cells (especially, IL-6 release by T cells, IL-6 release by monocytes and IL-6 release by endothelial cells; as supported by Examples 3, 4 and 6 hereinbelow). Thus, the p38 MAPK inhibitor suitably inhibits IL-6 release by T cells, inhibits IL-6 release by monocytes and inhibits IL-6 release by endothelial cells.

Surprisingly, when the p38 MAPK inhibitor is administered to a patient prior to the onset of CRS, it may potentiate T cells in the patient, and may do so in the presence of cancer cells. The p38 MAPK inhibitor may enhance T cells' anti-cancer efficacy; or may, at the least, maintain T cells' anti-cancer efficacy. In particular, the p38 MAPK inhibitor may increase the number of circulating T cells (see, especially, Example 5 below); promote or allow maintenance of T cell-induced cancer cell death (see, especially, Example 6 below); and/or reduce markers of exhaustion (especially, TIM3 and/or LAG3) in T cells (see, especially, Example 3 below). A reduction in markers of exhaustion may be interpreted as prolonging the anti-cancer efficacy of T cells; it may additionally or alternatively be linked to T cell polarisation, thus to "signal 2" as described above. Meanwhile, retention (or improvement) of T cell proliferation and anti-cancer efficacy may each be linked to modulation of "signal 4" as described above. Thus, the p38 MAPK inhibitor may allow maintenance of, or may enhance, the anti-cancer action of immunotherapy, in addition to preventing or reducing the severity of signs or symptoms of cancer immunotherapy-associated CRS. The p38 MAP kinase inhibitor may surprisingly have these effects when administered directly *(in vivo)* to a subject prior to the onset of CRS in that subject, as required by the present disclosure and as modelled, for instance, in Example 6 described herein.

Accordingly, the p38 MAPK inhibitor may prolong the (duration of the) anti-cancer efficacy of T cells. The p38 MAPK inhibitor may help to maintain or increase the strength of the anti-cancer efficacy of T cells. The p38 MAPK inhibitor may prolong the (duration of the) anti-cancer efficacy of T cells and help to maintain or increase the strength of the anti-cancer efficacy of T cells.

Most suitably, the p38 MAPK inhibitor may maintain or increase the anti-cancer efficacy of T cells.

As used herein, the term "cancer immunotherapy" refers to therapeutic stimulation of the immune system to treat cancer. Cancer immunotherapy is thought to enhance the natural capacity of the immune system to recognise and destroy cancer cells and may enhance immune cell memory functions, thereby preventing or reducing the risk of cancer recurrence.

As the term is used herein, cancer immunotherapy may especially comprehend one or more of: therapies which employ genetically engineered (or otherwise modified or cultured) T cells; immune cell-engaging therapies (especially BiTEs and other bispecific antibodies); and other therapies such as natural killer ("NK") cell therapy, cancer vaccines and T cell checkpoint modulators (especially, T cell checkpoint inhibitors). These therapies may benefit from modulation by a p38 MAPK inhibitor of one or more of T cell survival, phenotype and function. Additionally or alternatively, these therapies may benefit from modulation by a p38 MAPK inhibitor of the activation of monocytes and/or endothelial cells, particularly those in proximity to cancer cells targeted by the immune system, especially cancer cells targeted by T cells.

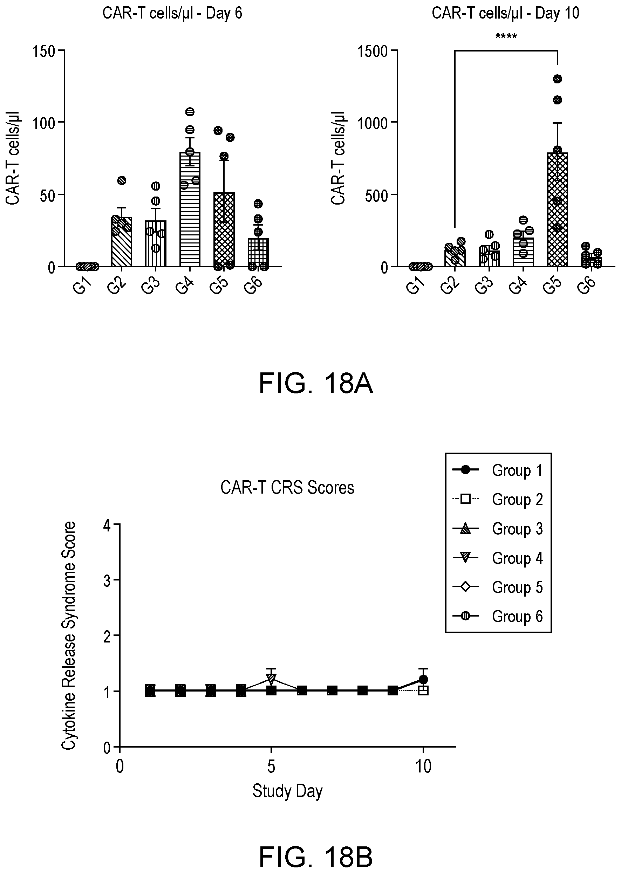

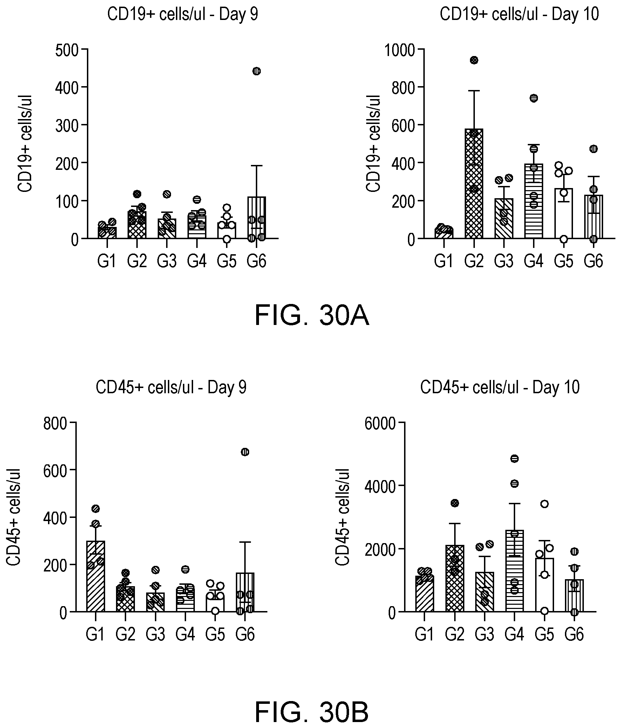

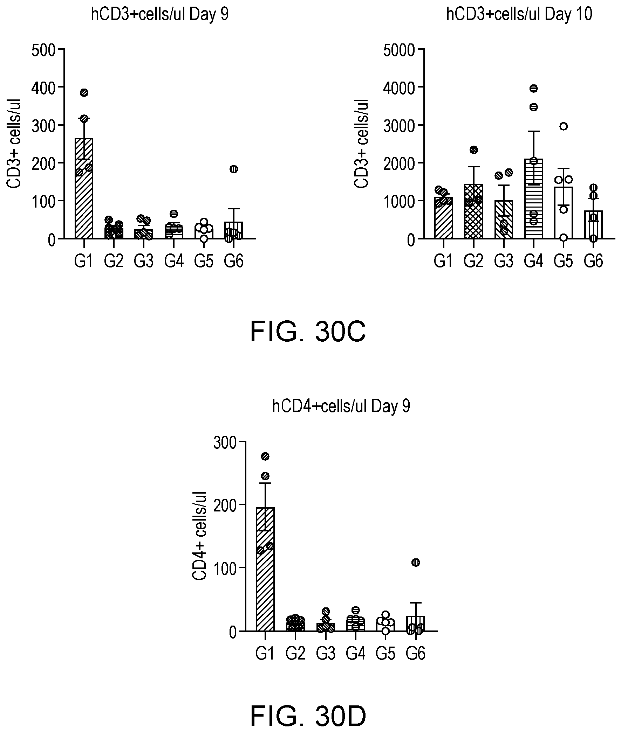

In accordance with the present disclosure, cancer immunotherapy may preferably comprise or consist of adoptive T cells or otherwise implicate T cells. It will be appreciated that a cancer immunotherapy which is said to "otherwise implicate T cells" may not necessarily be limited to a T cell-engaging therapy (such as BiTE, CiTE, SMITe or TriKE) but rather may comprehend any cancer immunotherapy wherein T cells are implicated. The cancer immunotherapy may therefore be one which is enhanced (or, at the least, not significantly impaired) through one or more of the following effects:
(i) reduced pro-inflammatory cytokine release by T cells (especially, reduced IL-6 release by T cells);
(ii) reduced pro-inflammatory cytokine release by monocytes (especially, reduced IL-6 release by monocytes);
(iii) reduced pro-inflammatory cytokine release by endothelial cells (especially, reduced IL-6 release by endothelial cells; and, optionally, reduced IL-8 release by endothelial cells);
(iv) modulation of T cell signals 3 and/or 4 (especially, signal 4);
(v) modulation of T cell polarisation and/or reduction of T cell exhaustion (especially, reduced TIM3 and/or reduced LAG3 on T cells' surface);
(vi) maintained or increased T cell anti-cancer cell efficacy; and
(vii) maintained or increased proliferation of T cells,
especially maintained or increased T cell anti-cancer cell efficacy, as evidenced by the Examples hereinbelow. In particular, Example 2 provides evidence of reduction of TIM3 and LAG3 (markers of exhaustion) on the surface of CAR T cells upon administration of a p38 MAPK inhibitor to CAR T cells in the presence of (in a co-culture with) cancer cells. A reduction in markers of exhaustion may be interpreted as prolonging the anti-cancer efficacy of T cells; it may additionally or alternatively be linked to T cell polarisation, thus to "signal 2" as described above. Three structurally distinct p38 MAPK inhibitors are shown to have this effect (all of which are selective for p38, implying no or minimal off-target effects). Example 2 also evidences a reduction in TNFα secretion by CAR T cells upon p38 MAPK inhibition; Example 6 evidences a reduction in a range of cytokines, including IL-6, understood to encompass those released by T cells, in addition to those released by monocytes and endothelial cells. Example 5 shows maintained CAR T cell efficacy upon p38 MAPK inhibition; by Day 10 of the described murine study, p38 MAPK inhibitor UR-13870 administered twice daily at 25 mg/kg significantly enhanced the number of CAR T cells in circulation (see **FIG.** 18A). Furthermore, Example 6 shows a downward trend in mean CD19+ cancer cells per ml following treatment with UR-13870 (compared to CD28 stimulation only) at days 9 and 10 (especially, day 10) of the described study, indicating potentiation of T cells/ increased T cell anti-cancer activity (see **FIG.** 30A). Meanwhile, Examples 3 and 4 evidence reduced IL-6 release by monocytes and endothelial cells; and reduced IL-8 release by endothelial cells, when treated with p38 MAPK inhibitors in the face of insult by supernatant from a cancer cell and CAR T cell co-culture, suggesting a beneficial effect on key mediators of CRS.

For some uses of the p38 MAP kinase inhibitor in accordance with the present disclosure, the cancer immunotherapy may comprehend a therapy mediated by T cell effector functions and memory; especially, therapies with engineered T cells (such, for example, as CAR T cell therapy and TCR therapy), with otherwise modified or cultured T cells (such, for example, as TIL therapy), or with T cell checkpoint modulators.

For some uses of the p38 MAP kinase inhibitor in accordance with the present disclosure, the cancer immunotherapy of the present disclosure may be a therapy mediated by T cell effector functions (but not, however, especially dependent on T cell memory); such, for example, as a T cell-engaging therapy, such as bispecific T-cell engager (BiTE) therapy, therapy with other bispecific antibodies, bifunctional checkpoint-inhibitory T cell engager (CiTE) therapy, simultaneous multiple interaction T cell engager (SMITe) therapy or trispecific killer engager (TriKE) therapy, or therapy with other immunostimulatory molecules able to induce cytokine release; especially BiTE therapy.

Thus, the cancer immunotherapy of the present disclosure may suitably be a T cell-engaging cancer immunotherapy (especially, BiTE therapy or therapy with other bispecific antibodies). This may be advantageous for the reason that the p38 MAP kinase inhibitor may benefit T cells as described herein, such as by increasing their level of anti-cancer efficacy; and/or prolonging the time period during which they display anti-cancer efficacy. The p38 MAP kinase inhibitor may surprisingly have these effects when administered directly (*in vivo*) to a subject prior to the onset of CRS in that subject, as required by the present disclosure and as modelled, for instance, in Example 6 described herein.

For some uses of the p38 MAP kinase inhibitor in accordance with the present disclosure, the cancer immunotherapy of the present disclosure may be a therapy which is not obviously dependent on T cell effector functions; nor on T cell memory. Nevertheless, improvement of T cell function may benefit. For example, the cancer immunotherapy may comprise CAR NK therapy, or a cancer vaccine.

For some uses of the p38 MAP kinase inhibitor in accordance with the present disclosure, the cancer immunotherapy may be a therapy which may benefit from modulation, by a p38 MAP kinase inhibitor, of signal 3 described hereinabove and elsewhere, such for example as: a T cell-engaging therapy, especially a bispecific T-cell engager (BiTE) therapy (or therapy with another bispecific antibody), a CAR NK therapy, a cancer vaccine, or a T cell checkpoint modulator; or a therapy with engineered T cells (such, for example, as CAR T cell therapy and TCR therapy) or with otherwise modified or cultured T cells (such, for example, as TIL therapy).

For some uses of the p38 MAP kinase inhibitor in accordance with the present disclosure, the cancer immunotherapy of the present disclosure may be a therapy which may benefit from modulation, by a p38 MAP kinase inhibitor, of signal 4 described hereinabove and elsewhere, such for example as: a T cell-engaging therapy, especially a bispecific T-cell engager (BiTE) therapy (or therapy with another bispecific antibody), CAR NK therapy, a cancer vaccine, or a T cell checkpoint modulator; or a therapy with engineered T cells (such, for example, as CAR T cell therapy and TCR therapy) or with otherwise modified or cultured T cells (such, for example, as TIL therapy).

For some uses of the p38 MAP kinase inhibitor in accordance with the present disclosure, the cancer immunotherapy may especially be therapy comprising the administration of genetically engineered (or otherwise modified or cultured) T cells. As used herein, depending on the context, the term "therapy comprising the administration of genetically engineered T cells" may refer to one or both of CAR T cell therapy and TCR therapy; or to a broader group of therapies encompassing CAR T cell therapy and TCR therapy. Meanwhile, "therapy comprising the administration of otherwise modified or cultured T cells" may refer to tumour-infiltrating lymphocyte ("TIL") therapy, or to a broader group of therapies encompassing TIL therapy.

The T cells described herein may be PD-1, CTLA4, TIM-3, TIGIT, CD3- or CD28-expressing T cells. CD28 may provide co-stimulatory signals for T cell activation and survival. CD28 may play a role in signals 1, 2, 3 and/or 4, described herein. CD28 may play a role in the CRS described herein.

Optionally, the cancer immunotherapy may be combination cancer immunotherapy, for example by means of CAR T cell therapy in combination with one or more checkpoint inhibitors, such as one or more anti-PD-1 antibodies (such as nivolumab, pembrolizumab or cemiplimab), or one or more anti-PD-L1 antibodies (such as atezolizumab, avelumab or durvalumab), or one or more CTLA-4 antibodies (such as ipilimumab or tremelimumab).

By "CART cell therapy" herein is meant therapy for the treatment of a cancer patient in which T cells, having been harvested (optionally although not necessarily harvested from the same cancer patient, as T cells may be harvested from a healthy donor) and genetically engineered *in vitro* to express CARs with specificity for relevant cancer cell antigens (and, optionally although not necessarily, proliferated *in vitro*) are administered to the cancer patient by adoptive transfer. Suitably, CAR T cell therapy may be implemented in treatment of a CD19-expressing malignancy or a BCMA-expressing malignancy. See, e.g., Morris et al., Cytokine release syndrome and associated neurotoxicity in cancer immunotherapy, Nat. Rev. Immunol., 2022, (22) 85-96. Thus, CAR T cell therapy may be autologous (in that the T cells are harvested from the same cancer patient). Alternatively, CAR T cell therapy may be allogeneic ("off the shelf") using T cells harvested from a healthy donor and modified to express CARs. In allogeneic CAR T cell therapy, the T cells may optionally be further modified, for example via genome editing technologies such as CRISPR/Cas9 or base editing, to prevent graft-vs.-host disease and host allorejection.

Similarly, TCR therapy involves genetic engineering of T cells, followed by their adoptive transfer to a patient in need thereof. While CAR T cells are capable of recognising and binding to naturally occurring antigens on the surface of cancer cells, in TCR therapy T cells are genetically engineered to express receptors which bind to major histocompatibility complex (MHC) proteins. However, the manufacturing process for T cells for use in TCR therapy may otherwise be analogous to that described hereinabove in respect of CAR T cells.

TCR therapy may thus be used herein to refer to therapy for the treatment of a cancer patient in which T cells, having been harvested (optionally although not necessarily harvested from the same cancer patient, as T cells may be harvested from a healthy donor) and genetically engineered *in vitro* to express engineered T cell receptors configured to recognise MHC-presented polypeptide fragments molecules (and, optionally although not necessarily, proliferated *in vitro*) are administered to the cancer patient by adoptive transfer.

In certain preferred embodiments, the cancer immunotherapy is CAR T cell therapy or TCR therapy.

By "tumour-infiltrating lymphocytes" ("TILs") herein is meant white blood cells that leave the bloodstream and migrate towards a solid tumour. TILs may be found within a tumour, as well as within the tumour stroma. The abundance and phenotype of TILs may vary with tumour type and stage. TILs may especially include T cells.

By "TIL therapy" herein is meant adoptive T cell transfer therapy of a patient in need thereof, wherein TILs, especially T cells, which may have originated from the patient or from another individual, are administered to the patient. Typically, therefore, TILs from a resected tumour are expanded *in vitro.* Several TIL cultures may be established separately before being assayed for satisfactory tumour recognition function. Once selected, the satisfactory TILs may be expanded over the course of a few weeks; typically using IL-2 as a general growth factor. Following a further TIL cell selection step, TIL lines having the best tumour recognition may be further expanded in a "rapid expansion protocol" (REP), which uses anti-CD3 activation for a period of a few weeks. Finally, the post-REP TILs are infused into the patient.

For some uses of the p38 MAP kinase inhibitor in accordance with the present disclosure, the cancer immunotherapy may be a T cell-engaging therapy, such for example as bispecific T-cell engager (BiTE) therapy or therapy with another bispecific antibody, bifunctional checkpoint-inhibitory T cell engager (CiTE) therapy, simultaneous multiple interaction T cell engager (SMITe) therapy or trispecific killer engager (TriKE) therapy.

The term "BiTE" as used herein means a bispecific monoclonal antibody for use as an anti-cancer drug. Particularly, a BiTE may be a fusion protein comprising two single-chain variable fragments ("scFvs") of different antibodies, or amino acid sequences from four different genes, on a single peptide chain. One of the scFvs may be configured to bind to T cells (especially, via a CD3 receptor); the other may be configured to bind to a tumour cell.

The term bispecific antibody as used herein means an antibody that can bind to two different types of antigen simultaneously. Various bispecific antibodies are known in the art, including BiTEs.

For some uses of the p38 MAP kinase inhibitor in accordance with the present disclosure, the cancer immunotherapy may be natural killer ("NK") cell therapy, a cancer vaccine or a T cell checkpoint modulatory therapy.

The term "NK cell therapy" may be used herein interchangeably with "CAR NK cell therapy". Like CAR T cells, CAR NK cells are genetically engineered to encode CARs that recognise tumour antigens. The manufacturing process for CAR NK cells is broadly analogous to that described hereinabove for CAR T cells.

By "CAR NK cell therapy" herein is meant therapy for the treatment of a cancer patient in which natural killer cells, having been harvested (optionally although not necessarily harvested from the same cancer patient, as NK cells may be harvested from a healthy donor) and genetically engineered *in vitro* to express CARs (and, optionally although not necessarily, proliferated *in vitro*) are administered to the cancer patient by adoptive transfer.

The term "cancer vaccine", as used herein, means one or more tumour antigen vaccines, whether autologous or allogeneic. A cancer vaccine may, for example, comprise: one or more cancer cell proteins and/or one or more fragments (peptide) thereof; one or more whole cells (such as tumour cells or dendritic cells); one or more nucleic acids; and/or one or more virus-based vaccines (encompassing oncolytic viruses).

As used herein, the term "T cell checkpoint modulator" has its normal meaning in the medical arts and means a T cell checkpoint inhibitor that is configured to block or otherwise antagonise tumour cell inhibitory immune checkpoint proteins from binding with partner proteins, or a T cell checkpoint stimulator, that is configured to enhance signalling for otherwise agonise tumour cell costimulatory checkpoint proteins.

The term "cancer immunotherapy-induced cytokine release syndrome" ("CRS"), as used herein, refers to an uncontrolled systemic inflammatory response that is triggered by cancer immunotherapy.

It is thought that the p38 MAP kinase inhibitor may attenuate the uncontrolled systemic inflammatory response that may result from cancer immunotherapy in some patients, rather than ablate it, with the objective of blunting the damaging effects of the out-of-control inflammation, whilst preserving its protective and disease pro-resolution effects, such as anti-cancer effects. Total suppression of inflammation would be likely to be deleterious, whereas attenuation of the explosive process of uncontrolled systemic inflammation should provide protection against the damaging effects caused by excess inflammatory responses whilst preserving essential innate defence activities. The p38 MAP kinase inhibitor for use in the methods of the present disclosure therefore aims to attenuate rather than ablate components in their entirety.

The population of patients undergoing cancer immunotherapy may be or comprise an especially sensitive patient population, for whom total suppression of essential innate defence activities would be especially deleterious. They may be an at-risk population for whom the ablation of core immune function would render them especially vulnerable to viral and/or bacterial infection. For these patients, reducing or preventing vulnerability to viral and/or bacterial infection is of significant concern. By attenuating the immune response without ablating it, the risk of significant viral and/or bacterial complications resulting from a suppressed immune system may be mitigated. Such infection could lead a physician to delay or discontinue the cancer immunotherapy; thus, by mitigating the risk of significant viral and/or bacterial complications, the risk of delay or discontinuation of the cancer immunotherapy is reduced; and the likelihood of anti-cancer efficacy is enhanced.

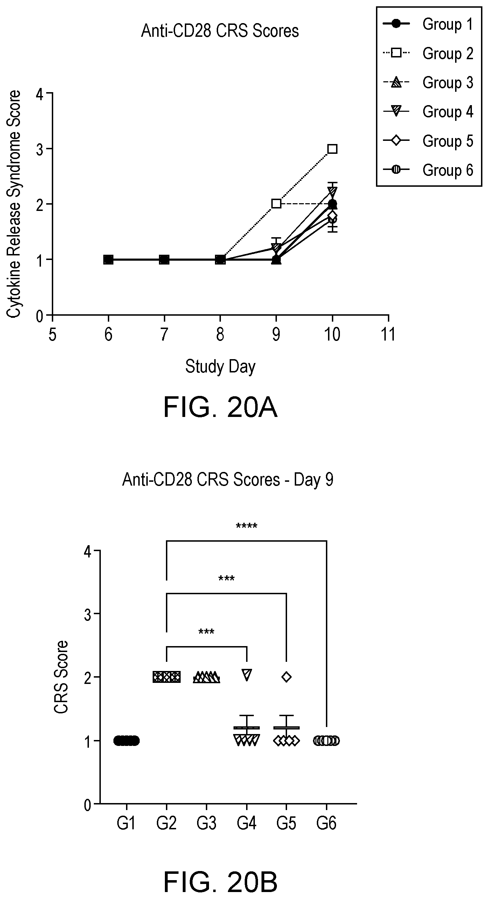

Cancer immunotherapy-induced CRS may present with a range of symptoms and signs. The symptoms and signs of CRS may vary, from mild, flu-like symptoms to severe life-threatening manifestations of an exaggerated inflammatory response. Examples 5 and 6 herein describe murine experiments in which symptoms of CRS modelled in mice were recorded. In particular in Example 6, administration of a p38 MAPK inhibitor prior to the onset of CRS was found to counteract CRS symptoms, as evidenced by a reduction in CRS score (see **FIGs. 20A-20C****).**

The onset of CRS may vary. The onset of CRS may be within 5, 4, 3, 2 or 1 day following administration of the cancer immunotherapy. Symptoms or signs of CRS may be observed within a day after administration of the cancer immunotherapy.

The "onset" of CRS, as the term is used herein, may refer to the start of one or more detectable symptoms or signs of CRS. A detectable symptom or sign may refer to a symptom or sign that is detectable by a physician.

Yet the onset of CRS may be later, such, for example, as a few weeks after administration of the cancer immunotherapy. Thus, the onset of CRS may occur after at least 10, 20, 30, 40 or 50 days have elapsed from administration of the cancer immunotherapy.

By way of example, TCR-engineered cells, T cells, NK cells or genetically engineered immune cells, which are able to replicate, may remain present in the body for an extended period, such for example as for up to 6 months or up to 12 months or longer. Thus, the cells may trigger CRS even after several weeks have passed following infusion. Thus, the onset of CRS may occur after about 1 week following administration of cancer immunotherapy, such for example as in a range of from about 1 week to 6 weeks following administration of cancer immunotherapy.

Mild symptoms or signs of CRS may be observed at first, for example within a matter of days after the onset of CRS (such, for example, as within 1 or 2 days after the onset of CRS).

Mild symptoms of CRS may include fever, fatigue, headache, rash, arthralgia, and myalgia, especially fever. As used herein, the term fever may refer to a core body temperature exceeding 38 °C. Respiratory symptoms may be common in patients with CRS. Mild respiratory symptoms and signs may include cough and tachypnoea (respiratory rate ≥ 30 for ages ≥ 12 years, rate ≥ 40 for ages 6 to 12 years, rate ≥ 45 for ages 3 to 6 years, rate ≥ 50 for ages 1 to 3 years).

The signs or symptoms of CRS to be prevented or reduced may be signs or symptoms of CRS of grade 1, graded in accordance with American Society for Transplantation and Cellular Therapy Consensus Guidelines (2019) as set out in **Table 5** hereinbelow. Thus, the patient may have symptoms that comprise or consist of fever.

Preventing or reducing the severity of signs or symptoms of cancer immunotherapy-associated CRS in a human patient may accordingly be or comprise preventing (or reducing the severity of) signs or symptoms of CRS of grade 1. Preventing or reducing the severity of signs or symptoms of cancer immunotherapy-associated CRS in a human patient may be or comprise preventing (or reducing the risk of) signs or symptoms of CRS of grade 1 progressing to signs or symptoms of CRS of grade 2. These effects may especially be of benefit where the cancer immunotherapy is or comprises T-cell engaging cancer immunotherapy, most especially BiTE therapy or therapy with one or more other bispecific antibodies. They may especially be of benefit to enable the early discharge of a cancer immunotherapy patient from hospital; and/or to limit their stay in hospital to about 1-3 days. Prophylaxis of grade 1 CRS, or prevention (or reducing the risk of) progression of grade 1 CRS to grade 2 CRS, in patients undergoing BiTE therapy or therapy with one or more other bispecific antibodies, is therefore especially contemplated herein (such as to enable their discharge from hospital, or even to enable all their treatment to be conducted outside a hospital setting).

Thus, preventing or reducing the severity of signs or symptoms of cancer immunotherapy-associated CRS in a human patient may be or comprise primary prophylaxis (by administration of the p38 MAP kinase inhibitor prior to the onset of CRS in any form, such as before administration of the cancer immunotherapy) and/or secondary prophylaxis (by administration of the p38 MAP kinase inhibitor when a patient has already received cancer immunotherapy, such as when the patient is displaying symptoms or signs of grade 1 CRS).

More serious symptoms or signs of CRS may be observed after the onset of mild symptoms or signs; nonetheless, the more serious symptoms or signs may be observed quite soon after the onset of CRS, such, for example, as within a matter of days or weeks after the onset of CRS (such, for example, as within 2, 3, 4, 5, 6, 15 or 25 after the onset of CRS).

Preventing or reducing the severity of signs or symptoms of cancer immunotherapy-associated CRS in a human patient may be or comprise preventing (or reducing the severity of) signs or symptoms of CRS of grade 2; or preventing (or reducing the risk of) signs or symptoms of CRS of grade 2 progressing to signs or symptoms of CRS of grade 3 (or grade 4). These effects may especially be of benefit where the cancer immunotherapy is or comprises CAR T cell therapy.

Symptoms and signs of CRS, especially if left untreated, may last for a week or more after the onset of CRS, for example, for up to 10 days, up to 20 days, or up to 50 days after the onset of CRS.

Serious symptoms of CRS may include hypotension. As used herein, hypotension may refer to a blood pressure of less than about 90/60 mmHg. Serious signs of CRS may include high fever. As used herein, the term high fever may refer to a core body temperature exceeding 40 °C. Especially, serious symptoms and signs of CRS may include both hypotension and high fever.

Serious respiratory symptoms and signs of CRS may include one or more of acute respiratory distress syndrome (ARDS) with dyspnea, hypoxemia (the patient may have an arterial oxygen saturation of ≤ 92% on room air by a transcutaneous method) and bilateral opacities on chest X-ray. ARDS owing to CRS may require mechanical ventilation.

Serious symptoms and signs of CRS may include circulatory shock, especially vasopressor-requiring circulatory shock. Further or alternatively, they may include vascular leakage, which may be accompanied by peripheral and pulmonary oedema; and, in some cases, disseminated intravascular coagulation.

Serious symptoms and signs of CRS may include dysfunction of one or more organs. Thus, serious symptoms and signs of CRS may include symptoms and signs of renal failure. Serious symptoms and signs of CRS may include symptoms and signs of cardiac dysfunction, for example as observed by reduced ejection fraction on echocardiography or Multi-Gated Acquisition (MUGA) scan. The symptoms and signs of CRS may progress to those of multi-organ system failure.

CRS may be marked by one or more of cytopenia (a lower-than-normal count of one or more types of blood cells) elevated creatinine and liver enzymes, deranged coagulation parameters, and a higher-than-normal level of C-reactive protein.

In especially severe cases, CRS may lead to death.

The symptoms and signs of CRS may differ from one patient to another. There may be a "first dose effect" wherein a patient undergoing therapy comprising multiple administrations of cancer immunotherapy over a period of time nonetheless only experiences severe symptoms and signs of CRS following their first administration thereof. A higher cancer burden may be a strong predictor of CRS, especially of severe CRS. A higher administered dose of cancer immunotherapy may be a strong predictor of CRS, especially of severe CRS. Children (as used herein, this may refer to those of age 12 or below) may experience more severe CRS than adults.

It will be understood that symptoms and signs of CRS in this context may be taken to mean symptoms and signs induced primarily by CRS, as opposed to any other cause.

In especially severe cases, CRS can be accompanied by clinical signs and laboratory abnormalities that resemble hemophagocytic lymphohistiocytosis (HLH) or macrophage activation syndrome (MAS). However, CRS induced by cancer immunotherapy is distinct from HLH and MAS.

CRS is also distinct from other conditions. Thus, CRS is distinct (at least in respect of its initiating factors) from sepsis. CRS is similarly distinct from tumour lysis syndrome.

By "reducing the severity of signs or symptoms of cancer immunotherapy-associated CRS in a human patient" herein is meant attenuating one or more of the above-described symptoms or signs of CRS. It may include, for example, preventing or reducing the severity of fever. It may include delaying the onset of fever. It may include preventing one or more of the serious symptoms and signs of CRS described herein, especially dysfunction of one or more organs. It may include preventing death.

"Preventing signs or symptoms of cancer immunotherapy-associated CRS in a human patient" may refer herein to averting one or more of the above-described symptoms and signs of CRS. It may include preventing fever. It may include preventing one or more of the serious symptoms and signs of CRS described herein, especially dysfunction of one or more organs. It may include preventing death.

"Preventing signs or symptoms of cancer immunotherapy-associated CRS in a human patient" may refer herein to reducing the risk of the patient suffering signs or symptoms of cancer immunotherapy-associated CRS, such for example as one or more of the above-described signs or symptoms. It may include reducing the risk of fever. It may include reducing the risk of one or more of the serious symptoms and signs of CRS described herein, especially dysfunction of one or more organs. It may include reducing the risk of death.

"Preventing signs or symptoms of cancer immunotherapy-associated CRS in a human patient" may refer herein to reducing prophylactically the risk of the patient suffering signs or symptoms of cancer immunotherapy-associated CRS, such for example as one or more of the above-described signs or symptoms. It may include reducing the risk of fever. It may include reducing the risk of one or more of the serious symptoms and signs of CRS described herein, especially dysfunction of one or more organs. It may include reducing the risk of death.

In one aspect, preventing or reducing the severity of signs or symptoms of cancer immunotherapy-associated CRS may be defined by reference to the mechanism of CRS induction by cancer immunotherapy. It is thought that cytokines produced by T cells (e.g., TNFα; and/or IL-6; especially, IL-6) in the presence of cancer cells as well as damage associated molecular patterns (DAMPs) from dead cancer cells attract circulating monocytes. The monocytes then release pro-inflammatory mediators (e.g., IL-6). In turn, this is thought to trigger release of pro-inflammatory mediators (e.g., IL-6; and IL-8; especially, IL-6) from nearby endothelial cells. Furthermore, T cells may overflow or redistribute systemically, causing monocyte and endothelial activation in diverse areas. Thus, one or more of the above-described symptoms and signs of CRS, including fever followed by hypotension, hypoxia and/or organ dysfunction, may be considered as a sequela or sequelae of hyperinduction of cytokines near to interactions between T cells and cancer cells, which may involve undue release of pro-inflammatory mediators from monocytes nearby. It may further involve undue release of pro-inflammatory mediators from proximate endothelial cells. This activity on the part of endothelial cells, also known as "endothelial activation," may lead to vascular leakage, hence hypotension (and further cytokine release).

Accordingly, prevention or attenuation of hyperinduction of cytokines near to interactions between T cells and cancer cells, i.e. prevention or attenuation of one or both of: (i) pro-inflammatory mediator release by monocytes; and (ii) pro-inflammatory mediator release by endothelial cells proximate the monocytes, may be effective to prevent or at least reduce the symptoms and signs of cancer immunotherapy-associated CRS and the risk of death.

Thus, according to the present disclosure, the p38 MAP kinase inhibitor may suitably inhibit the release of pro-inflammatory mediators from monocytes *in vivo.* In some implementations, the pro-inflammatory mediators include one or more of: IFNγ, IL-1β, IL-2, IL-6, IL-8, IL-10, IL-12, IL-18, CCL2, CCL5, CXCL10, MCP-1, MIP-1β, GM-CSF, VEGF and TNFα; most especially IL-6.

Monocytes may be responsible for releasing a substantial proportion of the cytokines (especially, IL-6) which are produced nearby to interactions between cancer cells and T cells. Thus, by targeting cytokine (especially, IL-6) release by monocytes, the p38 MAPK inhibitor may remove or weaken an important step in a chain of cellular signalling, or cytokine feedback loop, which otherwise would lead to CRS.

The terms monocyte and macrophage may be used interchangeably herein. It will be appreciated by those skilled in the art that monocytes typically develop into macrophages when recruited, via extravasation, from blood vessels to other tissues. When monocytes are attracted towards interacting T cells and cancer cells within the lumen of a blood vessel, they may exhibit characteristics and/or behaviours typically associated with macrophages; a non-limiting example being the release of pro-inflammatory mediators, including one or more of: IFNγ, IL-1β, IL-2, IL-6, IL-8, IL-10, IL-12, IL-18, CCL2, CCL5, CXCL10, MCP-1, MIP-1β, GM-CSF, VEGF and TNFα; most especially IL-6. It will also be appreciated that the terms "cytokine" and "pro-inflammatory mediator" may be used interchangeably herein.

Meanwhile, the term cancer may typically be used herein to refer to a blood cancer, such, for example, as a leukaemia, a lymphoma, or a myeloma. For example, a blood cancer may be: acute myeloid leukaemia (AML); chronic myeloid leukaemia (CML); acute lymphoblastic leukaemia (ALL); chronic lymphocytic leukaemia (CLL); non-Hodgkin lymphoma (NHL); Hodgkin lymphoma (HL); or myeloma. Thus, in some implementations, the cancer may be B cell or T cell acute lymphoblastic leukaemia; or large cell lymphoma. Especially, the cancer may be aggressive, relapsed or refractory non-Hodgkin lymphoma, including: diffuse large B cell lymphoma, primary mediastinal B-cell lymphoma, high grade B-cell lymphoma, transformed follicular lymphoma, mantle cell lymphoma and peripheral T cell lymphoma.

The cancer may be a CD19-expressing malignancy (e.g., a CD19-expressing refractory or relapsed B cell malignancy). The cancer may be a BMCA-expressing malignancy (e.g., a BCMA-expressing myeloma). The cancer may be a uveal melanoma.

It will be appreciated that the term cancer may also be used herein to refer to a solid tumour, in which case the term cancer cell may be used interchangeably with the term tumour cell. A cancer having one or more solid tumours may, for example, include a sarcoma; a carcinoma; a carcinosarcoma; or a lymphoma. A solid tumour may, for example, be found in the prostate, breast, lung, oesophagus, stomach, small intestine, pancreas, colon and/or rectum, central nervous system, urinary bladder, thyroid, kidney, uterine corpus, oral cavity, larynx, pharynx or ovary.

In some embodiments, the cancer may be a skin cancer.

In some embodiments, the p38 MAP kinase inhibitor may inhibit the release of pro-inflammatory mediators from endothelial cells *in vivo* (another major source of cytokines). Optionally, the pro-inflammatory mediators may include one or more of: IL-1β, IL-2, IL-6, IL-8, IL-10, CCL2, CCL5, CXCL10, IL-18, TNFα, MCP-1, MIP-1β, IP10 and GM-CSF; especially IL-6 and IL-8, most especially IL-6.

In some embodiments, the p38 MAP kinase inhibitor may inhibit the upregulation of one or more proteins (such, for example, as E-selectin, VCAM1 and/or ICAM1) on the surface of endothelial cells, especially ICAM1.

The p38 MAP kinase inhibitor may suitably inhibit the release of pro-inflammatory mediators from monocytes *in vivo* and also inhibit the release of pro-inflammatory mediators from endothelial cells *in vivo.*

The p38 MAP kinase inhibitor may suitably inhibit the release of IL-6 from monocytes and inhibit the release of IL-6 from endothelial cells *in vivo.* The p38 MAP kinase inhibitor may most suitably inhibit the release of IL-6 from T cells, inhibit the release of IL-6 from monocytes and inhibit the release of IL-6 from endothelial cells (*in vivo*; thus, it will be understood that this effect may occur when the T cells, monocytes and endothelial cells are all present; and it is advantageous for it to occur in the presence of cancer cells).

Especially, the p38 MAP kinase inhibitor, by inhibiting the release of pro-inflammatory mediators from monocytes *in vivo,* may prevent signalling from the monocytes to endothelial cells, meaning that endothelial cell activation is decreased and endothelial cells release fewer pro-inflammatory mediators (or even that endothelial cells are never activated and do not themselves release pro-inflammatory mediators).

Any treatments for the prophylaxis of CRS should desirably avoid significant attenuation of the anti-cancer effects of the cancer immunotherapy. By way of a representative but non-limiting example, T cells in genetically engineered T cell therapies should desirably retain their anti-cancer phenotype for a practically useful duration. More desirable would be a medicinal product for the prophylaxis of CRS which would also potentiate cancer immunotherapies.

The p38 MAPK inhibitor may suitably inhibit the release of cytokines (e.g., TNFα, see Example 3 below; and especially IL-6, see Example 6 below) from T cells *in vivo,* such, for example, as in the presence of cancer cells. The p38 MAPK inhibitor may suitably inhibit the release of cytokines from T cells subsequent to their having undergone antigen recognition and may consequently alter signal 3 or signal 4 described herein. In this way, the p38 MAPK inhibitor may prevent or reduce the risk of a cytokine feedback loop being started by T cells and may thus prevent or reduce monocyte recruitment and activation by T cells and cancer cells.

The p38 MAPK inhibitor may be effective to prevent or attenuate CRS without ablating the anti-cancer cell efficacy of the cancer immunotherapy. Advantageously, in some implementations, the p38 MAPK inhibitor may be effective to prevent or attenuate CRS while also increasing the anti-cancer cell efficacy of the cancer immunotherapy (such, for example, as in the presence of cancer cells). Example 2 provides evidence of reduction of TIM3 and LAG3 (markers of exhaustion) on the surface of CAR T cells upon administration of a p38 MAPK inhibitor to CAR T cells in the presence of (in a co-culture with) cancer cells. A reduction in markers of exhaustion may be interpreted as prolonging the anti-cancer efficacy of T cells; it may additionally or alternatively be linked to T cell polarisation, thus to "signal 2" as described above. Similarly, Example 5 shows no reduction in CAR T cell efficacy upon p38 MAPK inhibition; by Day 10 of the described murine study, p38 MAPK inhibitor UR-13870 administered twice daily at 25 mg/kg significantly enhanced the number of CAR T cells in circulation (see **FIG.** 18A). Furthermore, Example 6 shows a downward trend in mean CD19+ cancer cells per ml following treatment with UR-13870 (compared to CD28 stimulation only) at days 9 and 10 (especially, day 10) of the described study, indicating potentiation of T cells/ increased T cell anti-cancer activity (see **FIG.** 30A). Retention (or improvement) of T cell proliferation and anti-cancer efficacy may each be linked to modulation of "signal 4" as described above.

The p38 MAPK inhibitor is effective to prevent or reduce the severity of signs or symptoms of CRS in a human patient. The p38 MAP kinase inhibitor may prevent or reduce the severity of signs or symptoms of CRS while the anti-cancer cell efficacy of the cancer immunotherapy is maintained (suitably, in the presence of cancer cells). The p38 MAPK inhibitor may prevent or reduce the severity of signs or symptoms of CRS in a human patient without unduly attenuating anti-cancer cell efficacy of the cancer immunotherapy in the presence of cancer cells.

Advantageously, the p38 MAPK inhibitor may increase the anti-cancer cell efficacy of the cancer immunotherapy (*in vivo*). Advantageously, the p38 MAPK inhibitor may increase anti-cancer cell efficacy of the cancer immunotherapy in the presence of cancer cells. These effects may suitably be obtained by administration of the p38 MAPK inhibitor in a dosage amount as described herein.

As used herein, the term "anti-cancer cell efficacy" means the ability of a cancer immunotherapy to contribute to a reduction in cancer burden of a patient. T cells may assist in the attainment of such a reduction in cancer burden of a patient. Thus, suitably, the p38 MAP kinase inhibitor may increase one or more of the following in T cells: cancer cell-directed cytotoxicity, in vivo expansion, IFNγ production, durability of antigen specific-response, viability and memory phenotype (e.g. cell surface markers); and/or to reduce one or more of the following in T cells: oxidative stress markers (e.g. iNOS) and genomic stress markers (e.g. γH2AX). The p38 MAPK inhibitor may increase one or more of the following in CAR T cells: cancer cell-directed cytotoxicity, in vivo expansion, IFNγ production, durability of antigen specific-response, viability and memory phenotype (e.g. cell surface markers); and/or to reduce one or more of the following in CAR T cells: oxidative stress markers (e.g. iNOS) and genomic stress markers (e.g. γH2AX).

Suitably, not unduly attenuating the anti-cancer cell efficacy of the cancer immunotherapy in the presence of cancer cells is or comprises not unduly attenuating the anti-cancer cell efficacy of T cells in the presence of cancer cells. Suitably, increasing the anti-cancer cell efficacy of the cancer immunotherapy is or comprises increasing the anti-cancer cell efficacy of T cells. Increasing the anti-cancer cell efficacy of the cancer immunotherapy in the presence of cancer cells may be or comprise increasing the anti-cancer cell efficacy of T cells in the presence of cancer cells.

The p38 MAPK inhibitor may prevent or reduce T cell exhaustion (such, for example, as evidenced by reduction of TIM3 and LAG3 on the surface of the T cells). Suitably, therefore, the p38 MAPK inhibitor may prevent or reduce T cell exhaustion. As used herein, "T cell exhaustion" may refer to T cells incapable of causing cancer cell death; such T cells are non-functional and will have a transcriptional state distinct from that of functional T cells. Thus, the p38 MAPK inhibitor may prolong the anti-cancer cell efficacy of the T cells. It will be appreciated that the p38 MAPK inhibitor may be administered to the human patient in a dosage amount that is effective to do so. A reduction in markers of exhaustion may be interpreted as prolonging the anti-cancer efficacy of T cells; it may additionally or alternatively be linked to T cell polarisation, thus to "signal 2" as described above. Suitably, therefore, the p38 MAPK inhibitor may modulate T cell polarisation. Thus, the p38 MAPK inhibitor may be administered in a dosage amount that is effective to modulate T cell polarisation; and/or to prevent or reduce T cell exhaustion.

It is thought that signals 3 and 4 of T cell activation, as described herein, may be modulated by external factors. It is thought that the p38 MAPK inhibitor may modulate signals 3 and 4 in a manner which is not significantly deleterious to T cells, and which may, in some embodiments, have a beneficial effect on the T cells' anti-cancer cell efficacy.

In some embodiments, by reducing the secretion of cytokines (especially, IL-6) by monocytes and/or endothelial cells, signal 3 may be modulated. Signal 4 may be modulated. Both signals 3 and 4 may be modulated.

Accordingly, in some embodiments, the p38 MAPK inhibitor may modulate T cell polarisation. The p38 MAPK inhibitor may modulate T cell specialisation into sub-sets of T cells which are restricted to producing a certain pattern or patterns of cytokines.

The p38 MAPK inhibitor may modulate the development of T cell effector functions, such as T cell mediated tumour cell death. T cells may develop stronger effector functions. Additionally or alternatively, a larger protective T cell memory population may be formed as a result of p38 MAPK inhibition.

Meanwhile, in some embodiments, the p38 MAPK inhibitor may modulate post-cancer immunotherapy administration events *in vivo* and may modulate the duration of T cell response in the therapy, having an impact on subsequent T cell phenotype and function.

In some embodiments, the p38 MAPK inhibitor does not reduce *in vivo* proliferation of non-anergic T cells. In some embodiments, the p38 MAPK inhibitor does not reduce *in vivo* longevity of non-anergic T cells. In some embodiments, the p38 MAPK inhibitor does not reduce *in vivo* proliferation of non-anergic T cells and does not reduce *in vivo* longevity of non-anergic T cells.

In some embodiments, the p38 MAPK inhibitor increases *in vivo* proliferation of non-anergic T cells. In some embodiments, the p38 MAPK inhibitor increases *in vivo* longevity of non-anergic T cells. In some embodiments, the p38 MAPK inhibitor increases *in vivo* proliferation of non-anergic T cells and also increases *in vivo* longevity of non-anergic T cells.

There may be a smaller proportion of T cells that survive long term (such, for example, as for 3, 6, 9 or 12 months or more) yet are anergic.

Meanwhile, maintained or increased T cell proliferation, persistence and/or longevity notwithstanding, reduced monocyte and/or endothelial secretion of cytokines by means of a p38 MAPK inhibitor may lead to the prevention or reduction of severity of signs or symptoms of cancer immunotherapy-associated CRS. Thus, even when the anti-cancer cell efficacy of the cancer immunotherapy remains the same or is increased, which might otherwise be a predictor of CRS (similarly to the manner in which a higher dose of cancer immunotherapy has been thought to be linked to a greater risk and/or increased severity of CRS) signs and symptoms of CRS may be prevented, or their severity reduced.

Thus it may be advantageous to establish p38 MAPK inhibition *in vivo* by or during early stages of cancer immunotherapy, such as by the time of administration of cancer immunotherapy, or shortly thereafter, e.g. within 5, 4, 3, 2 or 1 day after administration of cancer immunotherapy. In this way, the p38 MAPK inhibitor may shape initial factors, during T cell activation and/or expansion associated with the early stages of cancer immunotherapy, which may influence the effectiveness of the anti-cancer therapy; especially signals 3 and 4.

Summarising, in broad terms, inter-related beneficial effects of the use of a p38 MAPK inhibitor in accordance with the present disclosure may include, but are not necessarily limited to, one or more of:
(i) reduced pro-inflammatory cytokine release by T cells (especially, reduced TNFα release by T cells);
(ii) reduced pro-inflammatory cytokine release by monocytes (especially, reduced IL-6 release by monocytes);
(iii) reduced pro-inflammatory cytokine release by endothelial cells (especially, reduced IL-6 release by endothelial cells; and, optionally, reduced IL-8 release by endothelial cells);
(iv) modulation of T cell signals 3 and/or 4 (especially, signal 4);
(v) modulation of T cell polarisation and/or reduction of T cell exhaustion (especially, reduced TIM3 and/or reduced LAG3 on T cells' surface;
(vi) maintained or increased T cell anti-cancer cell efficacy; and
(vii) maintained or increased proliferation of T cells.

Most especially, beneficial effects may pertain to maintaining or increasing T cell anti-cancer cell efficacy.

It will be appreciated that p38 MAP kinase exists in various isoforms, including an α isoform, a β isoform, a γ isoform and a δ isoform. In some embodiments, the p38 MAP kinase inhibitor may be an inhibitor of one or more of p38α MAP kinase, p38β MAP kinase, p38γ MAP kinase and p38δ MAP kinase; such, for example, as an inhibitor of p38α MAP kinase and/or an inhibitor of p38 β MAP kinase.

Without prejudice to the generality of the present disclosure, mentions of the use of a p38 MAP kinase inhibitor above and elsewhere herein specifically comprehend use of at least one active agent of the kind described below.

Thus, suitably, the p38 MAP kinase inhibitor may be of Formula I below, or a pharmaceutically acceptable salt or solvate thereof: 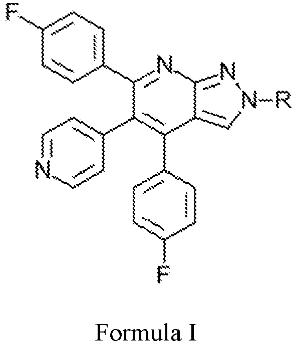 wherein R is C₁₋₃alkyl, optionally substituted by one or more halo, NR¹R² or hydroxyl, and R¹ and R² are independently H, halo or C₁₋₃alkyl, optionally substituted by one or more F.

For example, R may be optionally substituted by one, two or three of halo, NR¹R² or hydroxyl; or may be optionally substituted by one or two of halo, NR¹R² or hydroxyl; or may be optionally substituted by one of halo, NR¹R² or hydroxyl. When R is optionally substituted by more than one (for example two or three) halo, NR¹R² and/or hydroxyl, those substituents may be independently selected from that list.

When R¹ and/or R² is a C₁₋₃ alkyl optionally substituted by one or more F, each C₁₋₃ alkyl may, for example, be optionally substituted by one, two, or three F; be optionally substituted by one or two F; be optionally substituted by one or three F; be optionally substituted by one F; or be optionally substituted by three F.

In some embodiments, R is methyl or ethyl. In another embodiment, R may be propyl.

R may be substituted by one or more fluoride atoms.

In some embodiments, R may be substituted by hydroxy. For example, R may be ω-substituted alkyl, i.e. ω-hydroxyalkyl. Thus, in one embodiment, R may be 3-propanol.

Suitably R¹ and R² may be independently selected from H and CH₃.

R may be C₁₋₃ alkyl, which is substituted by one, two or three of halo, NR₁R₂ or hydroxy.

In some embodiments, the p38 MAPK inhibitor may be of Formula II or a pharmaceutically acceptable salt or solvate thereof:

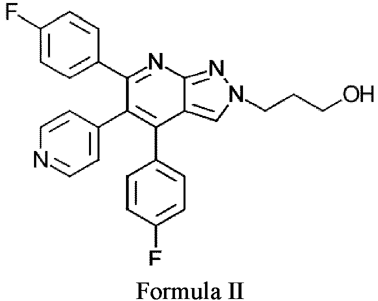

The compound of Formula II, and the synthesis of the compound, are disclosed in WO 2004/076450 A1 (see Example 18). Reference is also made to WO 2018/007788 A1 which discloses p38 MAP kinase inhibitors for use in the treatment or prevention of severe influenza in a human patient; and WO 2019/122909 A1 which discloses p38 MAP kinase inhibitors of Formula I for use in the treatment or prevention of hypercytokinemia in a human patient.

WO 2021/022186 and WO 2021/195475 disclose the use of p38 MAP kinase inhibitors in the treatment of various diseases including CRS. TVEDT TOR HENRIK ANDERSON ET AL: "Cytokine Release Syndrome in the Immunotherapy of Hematological Malignancies: The Biology behind and Possible Clinical Consequences", JOURNAL OF CLINICAL MEDICINE, vol. 10, no. 21, 1 January 2021, relates to cancer immunotherapy-associated CRS and treatment options of the latter, but does not disclose the use of p38 MAP kinase inhibitors.

The compound of Formula II may be especially suitable for oral administration, which is advantageous for its ease of delivery (for example, ease of delivery outside a hospital setting, in contrast to other routes of administration, such as intravenous administration, which may typically require a hospital context). It may especially be administered orally BID, such as about 5 to about 1000 mg BID, especially about 5 to about 200 mg BID, such as about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195 or 200 mg BID; especially, about 70 or about 150 mg BID.

The compound of Formula II may be especially suitable for oral administration at about 5 to about 1000 mg BID (especially, about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195 or 200 mg BID; most especially, about 70 or about 150 mg BID), beginning about 4 days to about 2 hours before administration of the cancer immunotherapy (the cancer immunotherapy may suitably administered as a single dose, e.g. by infusion) up to about 10 days after administration of the cancer immunotherapy.

The compound of Formula II may be tolerable (i.e., have low or now side effects or toxicity), especially when administered acutely (such as in a dosage regime described herein, such as a regime lasting for a matter of days; as opposed to a regime lasting for months or years).

Taken together, (i) the attenuation of an uncontrolled systemic inflammatory response, without ablating core immune function, in an at-risk group of patients; (ii) the convenient oral dosage regime; and (iii) the tolerability of the compound of Formula II, may be especially beneficial for patients undergoing cancer immunotherapy, for whom it is desirable to provide a way to reduce or prevent the severity of signs or symptoms of cancer immunotherapy-associated CRS, without ablating core immune function thus without risking viral and/or bacterial complications; without requiring continuous monitoring and/or administration of the compound in a hospital setting (achieved by oral dosing, rather than, e.g., IV dosing); and without causing deleterious side effects.

In some embodiments, the p38 MAP kinase inhibitor may be or include: 2-(4-chlorophenyl)-4-(fluorophenyl)-5-pyridin-4- yl-l,2-dihydropyrazol-3-one; 4-[4-(4-fluorophenyl)-1-(3-phenylpropyl)-5-(4-pyridinyl)-1H-imidazol-2-yl]-3-butyn-1-ol; (2R)-2-[[(2R)-2-amino-5-(diaminomethylideneamino)pentanoyl]amino]-N-[(2R)-1-[[(2R)-1-[[(2R)-1-[[(2R)-1-[[(2R)-1-[[(2R)-1-[[2-[[(2R)-1-amino-3-(4-hydroxyphenyl)-1-oxopropan-2-yl]amino]-2-oxoethyl]amino]-1-oxohexan-2-yl]amino]-1-oxohexan-2-yl]amino]-1-oxohexan-2-yl]amino]-5-(diaminomethylideneamino)-1-oxopentan-2-yl]amino]-1-oxohexan-2-yl]amino]-1-oxohexan-2-yl]hexanamide; 2-[6-chloro-5-[(2R,5S)-4-[(4-fluorophenyl)methyl]-2,5-dimethylpiperazine-1-carbonyl]-1-methylindol-3-yl]-N,N-dimethyl-2-oxoacetamide; 6-[(6R)-2-(4-fluorophenyl)-6-(hydroxymethyl)-4,5,6,7-tetrahydropyrazolo[1,5-a]pyrimidin-3-yl]-2-(2-methylphenyl)pyridazin-3-one; 4-[3-[4-(4-fluorophenyl)-5-pyridin-4-ylimidazol-1-yl]propyl]morpholine, 4-[5-(4-fluorophenyl)-3-piperidin-4-ylimidazol-4-yl]pyrimidin-2-amine; 4-[5-(4-fluorophenyl)-3-piperidin-4-ylimidazol-4-yl]pyridine; 4-[4-(4-fluorophenyl)-2-(4-methylsulfinylphenyl)-1H-imidazol-5-yl]pyridine; 4-[4-(4-fluorophenyl)-5-(2-methoxypyrimidin-4-yl)imidazol-1-yl]cyclohexan-1-ol; 4-[5-(4-fluorophenyl)-3-piperidin-4-ylimidazol-4-yl]-2-methoxypyrimidine; 6-(N-carbamoyl-2,6-difluoroanilino)-2-(2,4-difluorophenyl)pyridine-3-carboxamide; 5-(2,6-dichlorophenyl)-2-(2,4-difluorophenyl)sulfanylpyrimido[1,6-b]pyridazin-6-one; 2-[[(2S)-2-amino-3-phenylpropyl]amino]-3-methyl-5-naphthalen-2-yl-6-pyridin-4-ylpyrimidin-4-one;1-[5-tert-butyl-2-(4-methylphenyl)pyrazol-3-yl]-3-[4-(2-morpholin-4-ylethoxy)naphthalen-1-yl]urea; 6-(2,4-difluorophenoxy)-2-(1,5-dihydroxypentan-3-ylamino)-8-methylpyrido[2,3-d]pyrimidin-7-one; 1-[7-(4-fluorophenyl)-8-pyridin-4-yl-3,4-dihydro-1H-pyrazolo[5,1-c][1,2,4]triazin-2-yl]-2-phenylethane-1,2-dione; 8-(2,6-difluorophenyl)-2-(1,3-dihydroxypropan-2-ylamino)-4-(4-fluoro-2-methylphenyl)pyrido[2,3-d]pyrimidin-7-one; 2-[4-(4-fluorophenyl)-5-(2-phenoxypyrimidin-4-yl)imidazol-1-yl]propane-1,3-diol; N,N'-bis[3,5-bis[(E)-N-(diaminomethylideneamino)-C-methylcarbonimidoyl]phenyl]decanediamide; [2-[4-(4-fluorophenyl)-5-pyridin-4-yl-1H-imidazol-2-yl]-5-methyl-1,3-dioxan-5-yl]-morpholin-4-ylmethanone;methanesulfonic acid; [5-amino-1-(4-fluorophenyl)pyrazol-4-yl]-[3-[(2S)-2,3-dihydroxypropoxy]phenyl]methanone; 2-(2-chloro-6-fluorophenyl)-N-[3-(4-fluorophenyl)-4-pyrimidin-4-yl-1,2-oxazol-5-yl]acetamide; [(2R,3S,4R,5R,6R)-5-[[2-(aminomethylideneamino)acetyl]-methylamino]-3-hydroxy-2-(hydroxymethyl)-6-[(7-hydroxy-5-methyl-4-oxo-3a,6,7,7a-tetrahydro-1H-imidazo[4,5-c]pyridin-2-yl)amino]oxan-4-yl] carbamate; 1-[5-tert-butyl-2-(4-methylphenyl)pyrazol-3-yl]-3-[[5-fluoro-2-[1-(2-hydroxyethyl)indazol-5-yl]oxyphenyl]methyl]urea; 5-(2,4-difluorophenoxy)-N-[2-(dimethylamino)ethyl]-1-(2-methylpropyl)indazole-6-carboxamide; N-cyclopropyl-3-fluoro-4-methyl-5-[3-[[1-[2-[2-(methylamino)ethoxy]phenyl]cyclopropyl]amino]-2-oxopyrazin-1-yl]benzamide; 3-[5-amino-4-(3-cyanobenzoyl)pyrazol-1-yl]-N-cyclopropyl-4 - methylbenzamide;4-[5-(cyclopropylcarbamoyl)-2-methylanilino]-5-methyl-N-propylpyrrolo[2,1-f][1,2,4]triazine-6-carboxamide; 4-[4-(4-fluorophenyl)-2-(4-methylsulfonylphenyl)-1H-imidazol-5-yl]pyridine; N-[4-[5-(4-fluorophenyl)-3-methyl-2-methylsulfinylimidazol-4-yl]pyridin-2-yl]acetamide; 1-(5-tert-butyl-2-phenylpyrazol-3-yl)-3-[2-fluoro-4-[(3-oxo-4H-pyrido[2,3-b]pyrazin-8-yl)oxy]phenyl]urea, 1-(5-tert-butyl-2-phenylpyrazol-3-yl)-3-[2-methylsulfanyl-4-[(3-oxo-4H-pyrido[2,3-b]pyrazin-8-yl)oxy]phenyl]urea; 4-(3,4-dichlorophenyl)-5-(4-pyridinyl)-2-thiazolamine dihydrochloride; 5-(2-chloroethyl)-4-methyl-1,3-thiazole;ethane-1,2-disulfonic acid; 2'-fluoro-N-(4-hydroxyphenyl)-[1,1'-biphenyl]-4-butanamide; [4-(2-amino-4-bromoanilino)-2-chlorophenyl]-(2-methylphenyl)methanone; (E)-3-[4-(imidazol-1-ylmethyl)phenyl]prop-2-enoic acid; 17alpha-ethynyl-5-androstene-3beta, 7beta, 17beta-triol; (Z)-6-amino-2-(3',5'-dibromo-4'-hydroxybenzylidene)-2H-benzo[b][1,4]oxazin-3(4H)-one; (4-benzylpiperidin-1-yl)-(2-methoxy-4-methylsulfanylphenyl)methanone; 6-[5-(cyclopropylcarbamoyl)-3-fluoro-2-methylphenyl]-N-(2,2-dimethylpropyl)pyridine-3-carboxamide; 5-[2-tert-butyl-4-(4-fluorophenyl)-1H-imidazol-5-yl]-3-(2,2-dimethylpropyl)imidazo[4,5-b]pyridin-2-amine-methanesulfonic acid; 4-[5-(4-fluorophenyl)-2-methylsulfanyl-1H-imidazol-4-yl]-N-(1-phenylethyl)pyridin-2-amine;2-(3,4-dihydroxyphenyl)-3-hydroxychromen-4-one; 1-[5-tert-butyl-2-(3-chloro-4-hydroxyphenyl)pyrazol-3-yl]-3-[[2-[[3-[2-(2-hydroxyethylsulfanyl)phenyl]-[1,2,4]triazolo[4,3-a]pyridin-6-yl]sulfanyl]phenyl]methyl]urea; 3-[3-bromo-4-[(2,4-difluorophenyl)methoxy]-6-methyl-2-oxopyridin-1-yl]-N,4-dimethylbenzamide; 5-[2-tert-butyl-4-(4-fluorophenyl)-1H-imidazol-5-yl]-3-(2,2-dimethylpropyl)imidazo[4,5-b]pyridin-2-amine; 4-[4-[[4-chloro-3-(trifluoromethyl)phenyl]carbamoylamino]-3-fluorophenoxy]-N-methylpyridine-2-carboxamide; [5-amino-1-(4-fluorophenyl)pyrazol-4-yl]-[3-[(2S)-2,3-dihydroxypropoxy]phenyl]methanone, 4-[4-(4-fluorophenyl)-5-pyridin-4-yl-1H-imidazol-2-yl]phenol; 4-[4-(4-fluorophenyl)-2-(4-methylsulfinylphenyl)-1H-imidazol-5-yl]pyridine hydrochloride; 4-[4-(4-fluorophenyl)-5-pyridin-4-yl-1H-imidazol-2-yl]phenol hydrochloride; 1-[4-[3-(4-chlorophenyl)-4-pyrimidin-4-yl-1H-pyrazol-5-yl]piperidin-1-yl]-2-hydroxyethanone; N,N'-bis[3,5-bis[(E)-N-(diaminomethylideneamino)-C-methylcarbonimidoyl]phenyl]decanediamide, 6-(4-fluorophenyl)-5-pyridin-4-yl-2,3-dihydroimidazo[2,1-b][1,3]thiazole; 2-[6-chloro-5-[4-[(4-fluorophenyl)methyl]piperidine-1-carbonyl]-1-methylindol-3-yl]-N,N-dimethyl-2-oxoacetamide; [5-amino-1-(4-fluorophenyl)pyrazol-4-yl]-[3-(2,3-dihydroxypropoxy)phenyl]methanone; N-[4-[2-ethyl-4-(3-methylphenyl)-1,3-thiazol-5-yl]pyridin-2-yl]benzamid; 4-[4-(6-methoxynaphthalen-2-yl)-2-(4-methylsulfinylphenyl)-1H-imidazol-5-yl]pyridine; 4,6-bis(p-fluorophenyl)-2-methyl-5-(4-pyridyl)-1,2,7-triaza-2H-indene; and 2-(3-phenyl-4,5-dihydro-1,2-oxazol-5-yl)acetic acid.

In some embodiments, the p38 MAP kinase inhibitor may be or include: 8-(2,6-difluorophenyl)-2-(1,3-dihydroxypropan-2-ylamino)-4-(4-fluoro-2-methylphenyl)pyrido[2,3-d]pyrimidin-7-one, (E)-3-[4-(imidazol-1-ylmethyl)phenyl]prop-2-enoic acid; 6-[5-(cyclopropylcarbamoyl)-3-fluoro-2-methylphenyl]-N-(2,2-dimethylpropyl)pyridine-3-carboxamide, 5-[(2-chloro-6-fluorophenyl)acetylamino]-3-(4-fluorophenyl)-4-(4-pyrimidinyl)isoxazole, 1-[5-tert-butyl-2-(3-chloro-4-hydroxyphenyl)pyrazol-3-yl]-3-[[2-[[3-[2-(2-hydroxyethylsulfanyl)phenyl]-[1,2,4]triazolo[4,3-a]pyridin-6-yl]sulfanyl]phenyl]methyl]urea, 3-[3-bromo-4-[(2,4-difluorophenyl)methoxy]-6-methyl-2-oxopyridin-1-yl]-N,4-dimethylbenzamide, 2-methoxy-1-{4-[(4-{3-[5-(tert-butyl)-2-(p-tolyl)-2H-pyrazol-3-yl]ureido}-1-naphthyloxy)methyl]-2-pyridylamino}-1-ethanone, 2-methoxy-1-[4-(4-{3-[5-(tert-butyl)-2-(p-tolyl)-2H-pyrazol-3-yl]ureido}-1-naphthyloxy)-2-pyridylamino]-1-ethanone, and 4,6-bis(p-fluorophenyl)-2-methyl-5-(4-pyridyl)-1,2,7-triaza-2H-indene.

In accordance with the present disclosure, the p38 MAPK inhibitor, which may optionally be at least one of the active agents described above, is administered prior to the onset of CRS. It will be understood that this does not preclude administration of the p38 MAPK inhibitor, e.g. continued administration, subsequent to the onset of CRS. Rather, continued administration subsequent to the onset of CRS is preferred. Administration of the p38 MAPK inhibitor may begin from about 4 days to about 2 hours prior to administration of cancer immunotherapy (suitably, the cancer immunotherapy is administered in one dose, e.g. by infusion); subsequently, the patient may continue said oral dosage regime with the p38 MAPK inhibitor, with the end-point of the oral dosage regime being at up to about 10 days following administration of the cancer immunotherapy.

The p38 MAPK inhibitor is administered to the human patient prior to the onset of CRS. The p38 MAPK inhibitor is administered directly to the human patient, prior to the onset of CRS (such, for example, administered intravenously, orally, or both). The p38 MAPK is not, for example, administered *in vitro* to a culture of T cells, the cells later being administered to the human patient. Preventing or reducing the severity of signs or symptoms of cancer immunotherapy-associated CRS in a human patient is by *in vivo* administration of the p38 MAPK inhibitor. Thus, the p38 MAPK inhibitor is administered *in vivo,* to the human patient, prior to the onset of CRS.

The timing of administration of the p38 MAPK inhibitor prior to the onset of CRS may be as modelled in the murine experiments described in Examples 5 and 6 herein.

The p38 MAPK inhibitor may be administered orally, intravenously, intraperitoneally, topically, transdermally, intramuscularly, subcutaneously, intranasally, sublingually, intrathecally, intraventricularly, intratumourally or by any other suitable route. In preferred embodiments, the p38 MAPK inhibitor may be administered intravenously, orally, or both (such, for example, as via an initial intravenous dose followed by a number of oral doses, as disclosed herein).

It is especially preferred to administer the p38 MAPK inhibitor (such as a compound of Formula II) orally. Suitably, oral administration may be convenient in an outpatient setting. This may reduce the cost of therapy of the patient, by reducing the need for hospitalisation or other in-patient monitoring. Suitably, the patient may begin an oral dosage regime (such as about 5 mg BID to about 1000 mg BID, especially about 50 mg BID to about 200 mg BID, such as about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195 or 200 mg BID, especially about 70 mg BID or about 150 mg BID, orally) of the p38 MAPK inhibitor (e.g., begin it in hospital), from about 4 days to about 2 hours prior to administration of cancer immunotherapy (e.g., still in hospital; suitably, the cancer immunotherapy is administered in one dose, e.g. by infusion); subsequently, the patient may (e.g., be discharged from hospital and) continue said oral dosage regime with the p38 MAPK inhibitor, with the end-point of the oral dosage regime being at up to about 10 days following administration of the cancer immunotherapy.

The p38 MAPK inhibitor may be administered prior to the administration of cancer immunotherapy to the patient. The p38 MAPK inhibitor may be administered contemporaneously with the administration of cancer immunotherapy to the patient. The p38 MAPK inhibitor may be administered after the administration of cancer immunotherapy to the patient, but prior to the onset of CRS.

The p38 MAPK inhibitor may be administered prior to the administration of cancer immunotherapy. Optionally, a dose of the p38 MAPK inhibitor may be administered (e.g., intravenously or orally) at a time in a range of from about 1 minute to about six hours, or from about 10 minutes to about 2 hours, or from about 30 minutes to about 6 hours, prior to the administration of cancer immunotherapy. For example, a dose of the p38 MAPK inhibitor may be administered intravenously about 30 minutes prior to the administration of cancer immunotherapy.

More preferably, administration of the p38 MAPK inhibitor may begin at a time in a range of from about 6 days to about 30 minutes, such as about 5 days to about 1 hour, especially about 4 days to about 2 hours, prior to the administration of cancer immunotherapy. Such administration of the p38 MAPK inhibitor is most suitably oral and BID, such as 10 to 1000 mg orally, BID. Such administration of the p38 MAPK inhibitor suitably continues (orally, BID) until an end-point of up to 15 days, such as up to 10 days, especially up to 7 days after the administration of cancer immunotherapy (i.e., and then stops).

In such embodiments, the p38 MAPK inhibitor may be effective to prevent a type 1 infusion-related hypersensitivity response or at least to reduce the severity of a type 1 infusion-related hypersensitivity response. The term "type 1 infusion-related hypersensitivity response" may be used herein to refer to an acute immune-mediated response to any intravenous administration of cancer immunotherapy. In contrast to cancer immunotherapy-induced CRS, the classic mechanism of type 1 immune-mediated hypersensitivity reactions may involve the formation of immunoglobulin E (IgE) antibodies in response to infusion. Subsequently, IgE binds to mast cells, which may lead to the release of numerous cytokines. Signs and symptoms may include pruritus, urticaria, fever, rigors/chills, diaphoresis, bronchospasm, and cardiovascular collapse.

Optionally, one or more doses of the p38 MAPK inhibitor may be administered repeatedly (e.g., intravenously or orally), especially orally BID; or the p38 MAPK inhibitor may be administered continuously, especially by intravenous infusion; preferably, beginning at a time in a range of from about 4 days to about 2 hours, such as about 3 days to about 1 day prior to administration of cancer immunotherapy, in such a way as to establish a steady state blood plasma concentration of the p38 MAPK inhibitor by the time of administration of cancer immunotherapy.

Establishing a steady state blood plasma concentration of the p38 MAPK inhibitor by the time of administration of cancer immunotherapy may enhance its prophylactic effects as described herein.

To establish a steady state plasma concentration of the p38 MAPK inhibitor by the time of administration of cancer immunotherapy, the p38 MAPK inhibitor may be administered orally repeatedly at a dosage of one or more doses per day (e.g. BID), beginning at a time in a range of from about 4 days to about 2 hours, such as about 3 days to about 1 day, prior to administration of cancer immunotherapy. Such dosage regime may continue to an end-point of up to about 15, 10 or especially 7 days after the administration of the cancer immunotherapy. Advantageously, a patient may be relatively healthy, conscious and able to receive the p38 MAPK inhibitor by oral administration at this stage in their personal treatment cycle. Until now, treatment of cancer immunotherapy-induced CRS, such as by using tocilizumab and steroids as described hereinabove, has been reactive rather than prophylactic; however, reactive treatment of a critically ill patient may be difficult to achieve using oral medication, thus requiring clinicians to resort to alternative modes of administration which typically are more laborious and/or invasive.

For some patients, to establish a steady state plasma concentration of the p38 MAPK inhibitor by the time of administration of cancer immunotherapy, the p38 MAPK inhibitor may be administered by intravenous infusion; preferably beginning at a time in a range of from about 3 days to about 1 day prior to administration of cancer immunotherapy. Intravenous infusion may be continuous or may occur at regular intervals in order to establish the steady state blood plasma concentration of the p38 MAPK inhibitor. Intravenous infusion may be especially appropriate when oral administration of the p38 MAPK inhibitor would be unsuitable for the patient in question, for example patients under intensive care.

Those skilled in the art will be familiar with suitable methods to calculate when a steady state blood plasma concentration of an active agent, such as a p38 MAP kinase inhibitor used in accordance with the present disclosure, has been achieved. Steady-state concentration (*C*ₛₛ) is defined as the time during which the concentration remains stable or consistent when the active agent is given repeatedly or continuously (IV infusion). The time to reach steady-state is a function of *T*_{½}*,* and is achieved when the rate of the active agent entering the systemic circulation equals the rate of elimination. For most active agents, the *C*ₛₛ is reached in approximately five half-lives. The time to reach steady-state is independent of dose size, dosing interval and number of doses. In case of multiple dosing, when an active agent is administered in a fixed dose at fixed intervals, the plasma concentration increases exponentially to a plateau or steady-state with a half time of increase that is equal to the *T*_{½} of the agent.

The steady state blood plasma concentration of the p38 MAPK inhibitor may be in a range of from about 1 to about 750 µg/L, about 5 to about 600 µg/L, about 10 to about 500 µg/L, about 25 to about 500 µg/L, about 50 to about 500 µg/L, or about 100 to about 400 µg/L.

Thus, the p38 MAPK inhibitor may especially be administered in accordance with the present disclosure, beginning about 4 days to about 2 hours, such as about 1 day to about 3 days prior to administration of cancer immunotherapy, in such a way as to establish a steady state blood plasma concentration of the p38 MAPK inhibitor in a range of from about 1 to about 750 µg/L by the time of administration of cancer immunotherapy. Especially, such administration of the p38 MAPK inhibitor may be orally with a repeated dose (e.g. BID) or by intravenous infusion. Most especially, the p38 MAPK inhibitor may be administered orally at 5 to 1000 mg BID beginning about 4 days to about 2 hours prior to administration of cancer immunotherapy, until an end-point of up to about 15, 10 or especially 7 days after administration of cancer immunotherapy; thus establishing a steady state blood plasma concentration of the p38 MAPK inhibitor prior to, during and for a suitable amount of time after administration of the cancer immunotherapy.

Additionally or alternatively, the p38 MAPK inhibitor may be administered (e.g. intravenously) contemporaneously with administration of cancer immunotherapy. Optionally, the p38 MAPK inhibitor and the cancer immunotherapy may be administered contemporaneously but in separate formulations and, optionally, via separate routes of administration. In some embodiments, the cancer immunotherapy and the p38 MAPK inhibitor may be administered contemporaneously in different formulations by two separate intravenous injections. In some embodiments, the cancer immunotherapy may be infused intravenously while, contemporaneously, the p38 MAPK inhibitor is administered orally. In some embodiments, the p38 MAPK inhibitor and the cancer immunotherapy may be administered in the same formulation, for example both may be administered by the same intravenous injection.

Additionally or alternatively, the p38 MAPK inhibitor may be administered after the administration of cancer immunotherapy to the patient but prior to the onset of CRS. The p38 MAPK inhibitor may be administered within 1, 2 or 3 days after the administration of cancer immunotherapy (still prior to the onset of CRS). As the risk of CRS is thought to be especially high around 2 weeks after administration of cancer immunotherapy, the p38 MAPK inhibitor may be administered within 1 or within 2 weeks after the administration of cancer immunotherapy, prior to the onset of CRS.

The p38 MAPK inhibitor may be administered in a dosage schedule of OD (once daily), BID (twice daily), TID (three times daily), QID (four times daily) or more often. For example, the p38 MAPK inhibitor may be administered from about once per day to about 6 times per day. Especially, the p38 MAPK inhibitor may be administered BID. In some embodiments, the p38 MAPK inhibitor may be administered in a dosage schedule of every other day, three times a week, twice a week, weekly or bi-weekly. Examples 5 and 6 described herein model BID dosing. It may especially be administered orally BID, such as about 5 to about 1000 mg BID, especially about 5 to about 200 mg BID, such as about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195 or 200 mg BID; especially, about 70 or about 150 mg BID.

The p38 MAPK inhibitor dosage schedule may include a "drug holiday," wherein, in general terms one or more days on are followed by one or more days off, *e.g.,* the p38 MAPK inhibitor may be administered for one day on, one day off; or two days on, one day off; or three days on, one day off, etc.; or it may be administered continuously, without a drug holiday. Optionally, the p38 MAPK inhibitor may be administered in an initial dose before or contemporaneously with administration of cancer immunotherapy (which, it will be understood, may be a unique or one-off dose not forming part of the above-described dosage schedule) followed by commencement of the above-described dosage schedule. For example, there may be provided an initial intravenous dose followed by a number of oral doses following an above-described dosage schedule (e.g., BID, such for example as BID for about 1 to about 50 days).

Administration of the p38 MAPK inhibitor may continue as long as necessary. In some embodiments, the p38 MAPK inhibitor may be administered for less than about 28, about 14, about 7, about 6, about 5, about 4, about 3, or about 2 days. In some embodiments, the p38 MAPK inhibitor may be administered for more than about 1, about 2, about 3, about 4, about 5, about 6, about 7, about 14, or about 28 days. In some cases, continuous dosing may be achieved and maintained as long as necessary. In some embodiments, the p38 MAPK inhibitor may be administered for about 1 to 50 days; preferably about 7 to 40 days; more preferably about 14 to 28 days, following administration of cancer immunotherapy. It is envisaged however that administration of a p38 MAP kinase inhibitor in accordance with the present disclosure should be an acute treatment, extending over a period of days or weeks. The treatment of the present disclosure is not intended to be chronic, continuing over a period of months or longer.

Thus, preferably, dosing of the p38 MAPK inhibitor may begin at about 4 days to about 2 hours prior to administration of cancer immunotherapy; and such dosing may continue until an end-point of about 15 days after administration of cancer immunotherapy.

Example 5 herein describes a murine model of BID dosing for up to 11 days, while Example 6 herein describes a murine model of BID dosing for up to 7 days.

The p38 MAPK inhibitor may be administered beginning about 1 to about 5, especially about 1 to about 4, such as about 1 to about 3 days before the onset of CRS. The p38 MAPK inhibitor may be administered until about 7 to about 28 days after the onset of CRS (i.e., and then stopped).

The selected dosage form and regime will depend upon a variety of factors including, for example, the activity of the p38 MAPK inhibitory compound(s) employed, the route of administration, the time of administration, the rate of elimination of the p38 MAPK inhibitory compound(s) employed, the rate and extent of absorption, the duration of the treatment, the formulation of the medicinal product containing the p38 MAP kinase inhibitor, the presence of other drugs, compounds and/or materials used in combination with the p38 MAPK inhibitor employed, the age, sex, weight, condition, general health and prior medical history of the patient being treated, and other such factors well known in the medical arts.

In general, a suitable dosage amount (such, for example, as a twice-daily dose) of a p38 MAPK inhibitor described herein is the amount of the compound which, in some embodiments, is the lowest dosage amount effective to produce a therapeutic and/or prophylactic effect.

The dosage amount may, in some embodiments, be the lowest dosage amount effective to obtain one or more of the following effects:
(i) reduced pro-inflammatory cytokine release by T cells (especially, reduced TNFα release by T cells);
(ii) reduced pro-inflammatory cytokine release by monocytes (especially, reduced IL-6 release by monocytes);
(iii) reduced pro-inflammatory cytokine release by endothelial cells (especially, reduced IL-6 release by endothelial cells; and, optionally, reduced IL-8 release by endothelial cells);
(iv) modulation of T cell signals 3 and/or 4 (especially, signal 4);
(v) modulation of T cell polarisation and/or reduction of T cell exhaustion (especially, reduced TIM3 and/or reduced LAG3 on T cells' surface);
(vi) maintained or increased T cell anti-cancer cell efficacy; and
(vii) maintained or increased proliferation of non-anergic T cells.

Most especially, it may be the lowest dosage amount effective to maintain or increase T cell anti-cancer cell efficacy.

An effective dosage amount generally depends upon the factors described herein. Generally, dosage amounts of the p38 MAPK inhibitor, when used for the indicated effects, range from about 10 mg to about 1400 mg per day, or about 10 mg to about 1300 mg per day, or about 10 mg to about 1200 mg per day, or about 20 mg to about 1120 mg per day.

Thus, the dosage amounts of the p38 MAPK inhibitor may be in a range of from about 10 mg per day to about 600 mg per day. For example, the dosage may be about 150 mg per day. The dosage may, for example, be about 300 mg per day.

The p38 MAPK inhibitor may optionally be delivered BID. Dosage amounts of the p38 MAPK inhibitor, when used for the indicated effects, may range from about 5 mg BID to about 1000 mg BID, or about 5 mg to about 700 mg BID (i.e., about 10 mg to about 1400 mg in total per day), or about 5 mg to about 650 mg BID, or about 5 mg to about 600 mg BID, such, for example, as about 10 mg to about 560 mg BID. In some embodiments, the dosage amount may be about 20 mg, about 80 mg, about 400 mg, about 600 mg, about 800 mg, or about 1120 mg per day. In some embodiments, the dosage amount may be about 10 mg, about 40 mg, about 200 mg, about 300 mg, about 400 mg, or about 560 mg BID. The dosage amount may especially be about 5 to about 1000 mg BID, especially about 5 to about 200 mg BID, such as about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195 or 200 mg BID; especially, about 70 or about 150 mg BID.

In some embodiments, for a human patient, a suitable dose would be about 0.001 to about 20 mg/kg/day, or about 0.001 to about 10 mg/kg/day, or about 0.001 to about 5 mg/kg/day.

As described hereinabove, 70 mg BID or 150 mg BID may be especially preferred when the p38 MAPK inhibitor is a compound of Formula II.

Actual dosage amounts of the p38 MAPK inhibitor may be varied so as to obtain an amount of the p38 MAPK inhibitor which is effective to achieve the desired therapeutic response for a given patient, composition and mode of administration, without being toxic to the patient. In some instances, dosage amounts below the lower limit of the disclosed ranges may be more than adequate, while in other cases still larger dosage amounts may be employed without causing any harmful side effects.

In some embodiments, an initial intravenous dose (e.g. about 10 mg to about 1000 mg) of the p38 MAPK inhibitor may be administered, for example but not exclusively contemporaneously with, or before (e.g. about 1 minute to 6 hours, such as about 20 to 60 minutes, before) administration of cancer immunotherapy; then subsequent doses of the p38 MAPK inhibitor may be administered orally (e.g. about 10 mg to about 560 mg of the p38 MAPK inhibitor may be administered orally, one to three times per day such for example as BID, for about 1 to 50 days).

In another aspect, provided herein is a p38 MAP kinase inhibitor of Formula II, or a pharmaceutically acceptable salt or solvate thereof, for use in the prevention or treatment of cancer immunotherapy-associated CRS in a human patient. The p38 MAP kinase inhibitor of Formula II, salt or solvate may suitably be administered orally BID, such as at 5-1000 mg BID, especially about 5 to about 200 mg BID, such as about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195 or 200 mg BID; especially, about 70 or about 150 mg BID.

In a second aspect of the present disclosure, there is provided a pharmaceutical composition comprising a p38 MAP kinase inhibitor for use in preventing or reducing the severity of signs or symptoms of cancer immunotherapy-associated CRS in a human patient, wherein the pharmaceutical composition is administered *in vivo* prior to the onset of CRS.

Suitably, the pharmaceutical composition may comprise a p38 MAP kinase inhibitor defined in accordance with the first aspect of the present disclosure, including the specific active agents described above.

In some embodiments, the pharmaceutical composition may be a pharmaceutical composition suitable for oral administration. Pharmaceutical compositions suitable for oral administration may be presented as discrete dosage forms, such as capsules, cachets, or tablets, or liquids or aerosol sprays each containing a predetermined amount of a p38 MAPK inhibitor as a powder or in granules, a solution, or a suspension in an aqueous or non-aqueous liquid, an oil-in-water emulsion, or a water-in-oil liquid emulsion. Such dosage forms may be prepared by any of the methods of pharmacy, but all methods include the step of bringing the p38 MAP kinase inhibitor into association with a carrier, which constitutes one or more ingredients. In general, the pharmaceutical compositions are prepared by uniformly and intimately admixing the p38 MAP kinase inhibitor with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine the p38 MAPK inhibitor in a free-flowing form such as powder or granules, optionally mixed with an excipient such as, but not limited to, a binder, a lubricant, an inert diluent, and/or a surface active or dispersing agent. Molded tablets may be made by molding in a suitable machine a mixture of the powdered compound moistened with an inert liquid diluent.

The present disclosure further encompasses anhydrous pharmaceutical compositions and dosage forms comprising p38 MAPK inhibitor. Anhydrous pharmaceutical compositions and dosage forms may be prepared using anhydrous or low moisture containing ingredients and low moisture or low humidity conditions. For example, pharmaceutical compositions and dosage forms which contain lactose may be made anhydrous if substantial contact with moisture and/or humidity during manufacturing, packaging, and/or storage is expected. An anhydrous pharmaceutical composition may be prepared and stored such that its anhydrous nature is maintained. Accordingly, anhydrous pharmaceutical compositions may be packaged using materials known to prevent exposure to water such that they may be included in suitable formulary kits. Examples of suitable packaging include, but are not limited to, hermetically sealed foils, plastic or the like, unit dose containers, blister packs, and strip packs.

A p38 MAPK inhibitor may be combined in an intimate admixture with a pharmaceutical carrier according to conventional pharmaceutical compounding techniques. The carrier may take a wide variety of forms depending on the form of preparation desired for administration. In preparing the pharmaceutical compositions for an oral dosage form, any of the usual pharmaceutical media may be employed as carriers, such as, for example, water, glycols, oils, alcohols, flavoring agents, preservatives, coloring agents, and the like in the case of oral liquid preparations (such as suspensions, solutions, and elixirs) or aerosols; or carriers such as starches, sugars, micro-crystalline cellulose, diluents, granulating agents, lubricants, binders, and disintegrating agents may be used in the case of oral solid preparations, in some embodiments without employing the use of lactose. For example, suitable carriers include powders, capsules, and tablets, with the solid oral preparations. In some embodiments, tablets may be coated by standard aqueous or non-aqueous techniques.

Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, microcrystalline cellulose, and mixtures thereof.

Examples of suitable fillers for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof.

Disintegrants may be used in the pharmaceutical compositions disclosed herein to provide tablets that disintegrate when exposed to an aqueous environment. The amount of disintegrant used may vary based upon the type of formulation and mode of administration, and may be readily discernible to those of ordinary skill in the art. For example, about 0.5 to about 15 weight percent of disintegrant, or about 1 to about 5 weight percent of disintegrant, may be used in the pharmaceutical composition. Disintegrants that may be used to form pharmaceutical compositions and dosage forms include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums or mixtures thereof.

Lubricants which may be used to form pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g.,* peanut oil, cottonseed oil, sunflower oil, sesame oil, olive oil, corn oil, and soybean oil), zinc stearate, ethyl oleate, ethylaureate, agar, or mixtures thereof. Additional lubricants include, for example, a syloid silica gel, a coagulated aerosol of synthetic silica, or mixtures thereof. A lubricant may optionally be added, in an amount of less than about 1 weight percent of the pharmaceutical composition.

When aqueous suspensions and/or elixirs are desired for oral administration, the p38 MAPK inhibitor therein may be combined with various sweetening or flavoring agents, coloring matter or dyes and, for example, emulsifying and/or suspending agents, together with such diluents as water, ethanol, propylene glycol, glycerin and various combinations thereof.

Tablet dosage forms as disclosed herein may be uncoated or coated by known techniques to delay disintegration and absorption in the gastrointestinal tract and thereby provide a sustained action over a longer period. For example, a time delay material such as glyceryl monostearate or glyceryl distearate may be employed. Formulations for oral use may also be presented as hard gelatin capsules wherein the p38 MAPK inhibitor is mixed with an inert solid diluent, for example, calcium carbonate, calcium phosphate or kaolin, or as soft gelatin capsules wherein the p38 MAPK inhibitor is mixed with water or an oil medium, for example, peanut oil, liquid paraffin or olive oil.

In some embodiments, the pharmaceutical composition may include one or more surfactants. Surfactants which may be used to form the pharmaceutical composition include, but are not limited to, hydrophilic surfactants, lipophilic surfactants, and mixtures thereof. A mixture of hydrophilic surfactants may be employed, a mixture of lipophilic surfactants may be employed, or a mixture of at least one hydrophilic surfactant and at least one lipophilic surfactant may be employed.

In some embodiments, the pharmaceutical composition may include a solubilizer to ensure good solubilization and/or dissolution of the p38 MAPK inhibitor and to minimize precipitation of the p38 MAPK inhibitor. A solubilizer may also be added to increase the solubility of the p38 MAPK inhibitor and/or other components, such as surfactants, or to maintain the pharmaceutical composition as a stable or homogeneous solution or dispersion.

Examples of suitable solubilizers include, but are not limited to, the following: alcohols and polyols, such as ethanol, isopropanol, butanol, benzyl alcohol, ethylene glycol, propylene glycol, butanediols and isomers thereof, glycerol, pentaerythritol, sorbitol, mannitol, transcutol, dimethyl isosorbide, polyethylene glycol, polypropylene glycol, polyvinylalcohol, hydroxypropyl methylcellulose and other cellulose derivatives, cyclodextrins and cyclodextrin derivatives; ethers of polyethylene glycols having an average molecular weight of about 200 to about 6000, such as tetrahydrofurfuryl alcohol PEG ether (glycofurol) or methoxy PEG; amides and other nitrogen-containing compounds such as 2-pyrrolidone, 2-piperidone, ε-caprolactam, N-alkylpyrrolidone, N-hydroxyalkylpyrrolidone, N-alkylpiperidone, N-alkylcaprolactam, dimethylacetamide and polyvinylpyrrolidone; esters such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, ε-caprolactone and isomers thereof, δ-valerolactone and isomers thereof, β-butyrolactone and isomers thereof; and other solubilizers known in the art, such as dimethyl acetamide, dimethyl isosorbide, N-methyl pyrrolidones, monooctanoin, diethylene glycol monoethyl ether, and water.

Mixtures of solubilizers may also be used. Examples include, but not limited to, triacetin, triethylcitrate, ethyl oleate, ethyl caprylate, dimethylacetamide, N-methylpyrrolidone, N-hydroxyethylpyrrolidone, polyvinylpyrrolidone, hydroxypropyl methylcellulose, hydroxypropyl cyclodextrins, ethanol, polyethylene glycol 200-100, glycofurol, transcutol, propylene glycol, and dimethyl isosorbide. In some embodiments, solubilizers include sorbitol, glycerol, triacetin, ethyl alcohol, PEG-400, glycofurol and propylene glycol.

The amount of solubilizer that may be included is not particularly limited. The amount of a given solubilizer may be limited to a bioacceptable amount, which may be readily determined by one of skill in the art. In some circumstances, it may be advantageous to include amounts of solubilizers far in excess of bioacceptable amounts, for example to maximize the concentration of the drug, with excess solubilizer removed prior to providing the pharmaceutical composition to a subject using conventional techniques, such as distillation or evaporation. Thus, if present, the solubilizer may be in a weight ratio of about 10%, 25%, 50%, 100%, or up to about 200% by weight, based on the combined weight of the drug, and other excipients. If desired, very small amounts of solubilizer may also be used, such as about 5%, 2%, 1% or even less. Typically, the solubilizer may be present in an amount of about 1% to about 100%, more typically about 5% to about 25% by weight.

The pharmaceutical composition may further include one or more pharmaceutically acceptable additives and excipients. Such additives and excipients include, without limitation, detackifiers, anti-foaming agents, buffering agents, polymers, antioxidants, preservatives, chelating agents, viscomodulators, tonicifiers, flavorants, colorants, oils, odorants, opacifiers, suspending agents, binders, fillers, plasticizers, lubricants, and mixtures thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and sorbic acid. Exemplary alcohol preservatives include, but are not limited to, ethanol, polyethylene glycol, phenol, phenolic compounds, bisphenol, chlorobutanol, hydroxybenzoate, and phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus, Phenonip, methylparaben, Germall 115, Germaben II, Neolone, Kathon, and Euxyl. In certain embodiments, the preservative is an anti-oxidant. In other embodiments, the preservative is a chelating agent.

Exemplary oils include, but are not limited to, almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and combinations thereof.

In addition, an acid or a base may be incorporated into the pharmaceutical composition to facilitate processing, to enhance stability, or for other reasons. Examples of pharmaceutically acceptable bases include amino acids, amino acid esters, ammonium hydroxide, potassium hydroxide, sodium hydroxide, sodium hydrogen carbonate, aluminum hydroxide, calcium carbonate, magnesium hydroxide, magnesium aluminum silicate, synthetic aluminum silicate, synthetic hydrocalcite, magnesium aluminum hydroxide, diisopropylethylamine, ethanolamine, ethylenediamine, triethanolamine, triethylamine, triisopropanolamine, trimethylamine, tris(hydroxymethyl)aminomethane (TRIS) and the like. Also suitable are bases that are salts of a pharmaceutically acceptable acid, such as acetic acid, acrylic acid, adipic acid, alginic acid, alkanesulfonic acid, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, uric acid, and the like. Salts of polyprotic acids, such as sodium phosphate, disodium hydrogen phosphate, and sodium dihydrogen phosphate may also be used. When the base is a salt, the cation may be any convenient and pharmaceutically acceptable cation, such as ammonium, alkali metals, alkaline earth metals, and the like. Examples may include, but not limited to, sodium, potassium, lithium, magnesium, calcium and ammonium.

Suitable acids are pharmaceutically acceptable organic or inorganic acids. Examples of suitable inorganic acids include hydrochloric acid, hydrobromic acid, hydriodic acid, sulfuric acid, nitric acid, boric acid, phosphoric acid, and the like. Examples of suitable organic acids include acetic acid, acrylic acid, adipic acid, alginic acid, alkanesulfonic acids, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, methanesulfonic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, uric acid and the like.

In some embodiments, provided herein are pharmaceutical compositions for parenteral administration, such as intravenous administration, containing a p38 MAPK inhibitor as disclosed herein, and a pharmaceutical excipient suitable for parenteral administration.

The forms in which the disclosed pharmaceutical compositions may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Aqueous solutions in saline are also conventionally used for injection. Ethanol, glycerol, propylene glycol, liquid polyethylene glycol, and the like (and suitable mixtures thereof), cyclodextrin derivatives, and vegetable oils may also be employed. The proper fluidity may be maintained, for example, by the use of a coating, such as lecithin, for the maintenance of the required particle size in the case of dispersion and by the use of surfactants. The prevention or attenuation of the action of microorganisms may be brought about by various antibacterial and antifungal agents, for example, parabens, chlorobutanol, phenol, sorbic acid, thimerosal, and the like.

A preferred mode of administration according to the present disclosure may be or involve intravenous infusion of a solution or dispersion of the p38 MAPK inhibitor, especially beginning about 1 to about 3 days before administration of cancer immunotherapy or the onset of CRS, thereby reaching a steady state blood plasma concentration of the p38 MAPK inhibitor by the time of administration of cancer immunotherapy or the onset of CRS. Thus, the present disclosure especially comprehends a pharmaceutical composition wherein a p38 MAPK inhibitor is incorporated in a powder for the preparation of such a solution or dispersion. The powder may be a vacuum-dried or freeze-dried (lyophilized) powder, comprising the p38 MAPK inhibitor and, optionally, suitable excipients and/or additives. Suitably the powder may contain a sufficient amount of at least one p38 MAP kinase inhibitor that when made up to form a solution for infusion, the concentration of active agent in the solution is suitable to achieve the required dosage amount per hour at an infusion rate of about 500-3000 mL/24h.

Sterile injectable solutions may be prepared by incorporating a p38 MAPK inhibitor as disclosed herein in the required amount in the appropriate solvent with various other ingredients as enumerated above, as appropriate, followed by filtered sterilization. Generally, dispersions are prepared by incorporating the sterilized p38 MAPK inhibitor into a sterile vehicle which contains the basic dispersion medium and the appropriate other ingredients from those enumerated above. In the case of sterile powders for the preparation of sterile injectable solutions, certain methods of preparation are vacuum-drying and freeze-drying techniques which yield a powder of the p38 MAPK inhibitor plus any additional ingredient from a previously sterile-filtered solution thereof.

The injectable formulations may be sterilized, for example, by filtration through a bacterial-retaining filter, or by incorporating sterilizing agents in the form of sterile solid compositions which may be dissolved or dispersed in sterile water or other sterile injectable medium prior to use. Injectable compositions may contain from about 0.1 to about 5% w/w of a p38 MAPK inhibitor as disclosed herein.

In some embodiments, provided herein are pharmaceutical compositions for controlled release administration containing a p38 MAPK inhibitor as disclosed herein, and a pharmaceutical excipient suitable for controlled release administration.

The p38 MAPK inhibitor may be administered by controlled release means or by delivery devices that are well known to those of ordinary skill in the art. Such dosage forms may be used to provide slow or controlled release of the p38 MAPK inhibitor using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled release formulations known to those of ordinary skill in the art, including those described herein, may be readily selected for use with the p38 MAPK inhibitors disclosed herein. Thus, the pharmaceutical compositions provided encompass single unit dosage forms suitable for oral administration such as, but not limited to, tablets, capsules, gelcaps, and caplets that are adapted for controlled release.

All controlled release pharmaceutical products have a common goal of improving drug therapy over that achieved by their non-controlled counterparts. In some embodiments, the use of a controlled release preparation in medical treatment is characterized by a minimum of drug substance being employed to cure or control the disease, disorder, or condition in a minimum amount of time. Advantages of controlled release formulations include extended activity of the drug, reduced dosage frequency, and increased subject compliance. In addition, controlled release formulations may be used to affect the time of onset of action or other characteristics, such as blood levels of the drug, and may thus affect the occurrence of side (e.g., adverse) effects.

In some embodiments, controlled release formulations are designed to initially release an amount of a p38 MAPK inhibitor as disclosed herein that promptly produces the desired therapeutic effect, and gradually and continually release other amounts of the p38 MAPK inhibitor to maintain this level of therapeutic or prophylactic effect over an extended period of time. In order to maintain this constant level of the compound in the body, the p38 MAPK inhibitor should be released from the dosage form at a rate that will replace the amount of drug being metabolized and excreted from the body. Controlled release of a p38 MAPK inhibitor may be stimulated by various conditions including, but not limited to, pH, temperature, enzymes, water, or other physiological conditions or compounds.

In certain embodiments, the pharmaceutical composition may be administered using intravenous infusion, an implantable osmotic pump, a transdermal patch, or other modes of administration. In one embodiment, a pump may be used. In another embodiment, polymeric materials may be used. In yet another embodiment, a controlled release system may be placed in a subject at an appropriate site determined by a practitioner of skill, *e.g.,* thus requiring only a fraction of the systemic dose.

In a third aspect of the present disclosure (disclosed but not claimed), there is provided a method of preventing or reducing the severity of cancer immunotherapy-associated CRS in a human patient, comprising administering to the patient a prophylactically effective amount of a p38 MAPK inhibitor prior to the onset of CRS.

Suitably, the method may comprise administering to the patient a prophylactically effective amount of a p38 MAPK inhibitor defined in accordance with the first aspect of the present disclosure, including the specific active agents described above.

In a fourth aspect of the present disclosure, there is provided a kit, comprising: (i) a pharmaceutical composition as defined in accordance with the second aspect of the present disclosure and (ii) a leaflet comprising instructions for the use of said pharmaceutical composition in preventing or reducing the severity of signs or symptoms of cancer immunotherapy-associated CRS in a human patient; wherein the p38 MAPK inhibitor is to be administered prior to the onset of CRS.

A preferred mode of administration according to the present disclosure may be intravenous infusion of a solution or dispersion of the p38 MAPK inhibitor, especially beginning about 1 to about 3 days before administration of cancer immunotherapy or the onset of CRS, thereby reaching a steady state blood plasma concentration of the p38 MAPK inhibitor by the time of administration of cancer immunotherapy or the onset of CRS. Thus, the present disclosure especially comprehends a kit including a pharmaceutical composition wherein a p38 MAPK inhibitor is incorporated in a powder for the preparation of such a solution or dispersion and a leaflet comprising printed instructions for using the powder in accordance with the methods disclosed herein. The powder may be a vacuum-dried or freeze-dried (lyophilized) powder, comprising the p38 MAPK inhibitor and, optionally, suitable excipients and/or additives.

Another preferred mode of administration according to the present disclosure may be repeated oral administration, especially BID, of the p38 MAPK inhibitor, especially beginning about 1 to about 3 days before administration of cancer immunotherapy or the onset of CRS, thereby reaching a steady state blood plasma concentration of the p38 MAPK inhibitor by the time of administration of cancer immunotherapy or the onset of CRS. Thus, the present disclosure especially comprehends a kit including a plurality of oral dosage forms of a p38 MAPK inhibitor for such administration and a leaflet comprising printed instructions for using the dosage forms in accordance with the methods disclosed herein.

In addition to the leaflet, the kit may comprise additional printed material, such, for example, as a discussion of clinical studies, listing of side effects, and the like. Such kits may also include information, such as scientific literature references, package insert materials, clinical trial results, and/or summaries of these and the like, which indicate or establish the activities and/or advantages of the pharmaceutical composition, and/or which describe dosing, administration, side effects, drug interactions, or other information useful to the healthcare provider.

In some embodiments, a memory aid may be provided with the kit, for example in the form of numbers next to individual dosage forms (e.g. tablets or capsules as disclosed herein) whereby the numbers correspond with the days of a dosage schedule during which the forms so specified should be ingested. Another example of such a memory aid is a calendar printed on a card, *e.g.,* as follows "First Week, Monday, Tuesday, . . . etc. . . . Second Week, Monday, Tuesday, . . . " etc. Other variations of memory aids will be readily apparent. A "daily dose" may be a single dosage form or several dosage forms to be taken on a given day (e.g. a dose specified as BID).

Suitable packaging and additional articles for use (*e.g*., measuring cup for liquid preparations, foil wrapping to minimize exposure to air, and the like) are known in the art and may be included in the kit. In other embodiments, kits may further comprise devices that are used to administer the pharmaceutical composition. Examples of such devices include, but are not limited to, syringes, drip bags, patches, and inhalers. Kits described herein may be provided, marketed and/or promoted to healthcare providers, including physicians, nurses, pharmacists, formulary officials, and the like. Kits may also, in some embodiments, be marketed directly to the consumer.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. The strength of the sheet is such that the tablets or capsules may be removed from the blister pack by manually applying pressure on the recesses whereby an opening is formed in the sheet at the place of the recess. The tablet or capsule may then be removed via said opening.

Kits may further comprise pharmaceutically acceptable vehicles that may be used to administer the pharmaceutical composition. For example, if the pharmaceutical composition is provided in a solid form that must be reconstituted for parenteral administration, the kit may comprise a sealed container of a suitable vehicle in which the p38 MAPK inhibitor may be dissolved to form a particulate-free sterile solution that is suitable for parenteral administration.

In a fifth aspect of the present disclosure (disclosed but not claimed), there is provided the use of a p38 MAP kinase inhibitor in the manufacture of a medicament for preventing or reducing the severity of signs or symptoms of cancer immunotherapy-associated CRS in a human patient, wherein the pharmaceutical composition is administered *in vivo* prior to the onset of CRS. Suitably, the p38 MAP kinase inhibitor may be defined in accordance with the first aspect of the present disclosure, including the specific active agents described above.

In a sixth aspect of the present disclosure (disclosed but not claimed), there is provided the use of a pharmaceutical composition comprising a p38 MAP kinase inhibitor in the manufacture of a medicament for preventing or reducing the severity of signs or symptoms of cancer immunotherapy-associated CRS in a human patient, wherein the pharmaceutical composition is administered *in vivo* prior to the onset of CRS. Suitably, the pharmaceutical composition may be defined in accordance with the third aspect of the present disclosure.

In a seventh aspect of the present disclosure (disclosed but not claimed), there is provided a method of reducing cytokine (especially, IL-6) release by monocytes owing to administration of cancer immunotherapy to a human patient, while maintaining or increasing the anti-cancer cell efficacy of the cancer immunotherapy, by administering a therapeutically or prophylactically effective amount of a p38 MAPK inhibitor to the patient. It will be appreciated that the reduction in cytokine release by monocytes, in methods according to the seventh aspect of the disclosure, is performed in the presence of cancer cells. Suitably, the p38 MAPK inhibitor may be defined in accordance with the first aspect of the present disclosure, including the specific active agents described above.

Optionally, the method further involves reducing cytokine (especially, TNFα) release by T cells. Additionally or alternatively, the method involves reducing cytokine (especially, IL-6) release by endothelial cells. Additionally or alternatively, the method involves modulation of T cell signals 3 and/or 4. Additionally or alternatively, the method involves maintaining or increasing T cell proliferation.

The method may suitably comprise prolonging the anti-cancer cell efficacy of T cells. The p38 MAP kinase inhibitor may modulate T cell polarisation. The p38 MAP kinase inhibitor may prevent or reduce T cell exhaustion. The p38 MAP kinase inhibitor may reduce one or more of TIM3 and LAG3 in T cells.

It will be appreciated that features described in relation to one aspect of the present disclosure may be incorporated into other aspects of the present disclosure.

### Description of the Drawings

Embodiments of the present disclosure are now described by way of example only with reference to the accompanying schematic drawings in which:
**FIG. 1** shows the structure of CD 19 CAR and mock CAR vectors used to transfect T293 cells (see Example 1);
**FIG. 2A** and **FIG. 2B** show flow cytometric confirmation of mock CAR T cell transduction and CD19 CAR T cell transduction (see Example 1);
**FIG. 3** shows mCherry fluorescence in stably transfected Raji (cancer) cells (see Example 1);
**FIG. 4** shows CD19 CAR T and mock CAR T cell tumour cell clearance at a range of CD19 CAR T (or mock CAR T) to tumour cell ratios, as determined by the proportion of cancer cells in culture after 28 and 48 hours (see Example 1);
**FIG. 5** shows CD19 CAR T and mock CAR T cell proliferation following incubation with tumour cells at a CD19 CAR T (or mock CAR T) to tumour cell ratio of 1:1 for 24, 48 and 96 hours (see Example 1);
**FIG. 6** shows the impact of p38 inhibitors on CAR T cell proliferation, in a CAR T : tumour cell co-culture (see Example 2);
**FIG. 7** shows the effect of p38 inhibition (at "high" dose only) on CAR T cell killing of tumour cells in a 24-hour coculture with a CAR T to cancer cell ratio of 1:1 (see Example 2);
**FIG. 8A** and **FIG. 8B** show CAR T cell activation and exhaustion as explored in the experiment described in Example 2;
**FIG. 9** shows the effect of p38 inhibition on supernatant levels of TNFα from a CAR T : tumour cell co-culture (see Example 2);
**FIG. 10** shows the effect of p38 inhibition on supernatant levels of IL-6 from THP-1 (monocyte) cells treated with "sCAR T" for 24 hours (see Example 3);
**FIG. 11A** and **FIG. 11B** show the effect of p38 inhibition on supernatant levels of IL-6 and IL-8 from HUVEC (endothelial) cells treated with "sCAR T" for 24 hours (see Example 3);
**FIGs. 12A-C** show the effect of p38 inhibition on RNA levels of IL-6, ICAM1 and VCAM1 in HUVEC cells treated with "sCAR T" for 24 hours (see Example 3);
**FIG. 13** is a schematic drawing of apparatus for the co-culture of the experiment described in Example 4;
**FIG. 14A** and **FIG. 14B** show the effect of p38 inhibition on levels of IL-6 and IL-8 in supernatant from HUVEC cells treated with "sCAR T mono" supernatant for 24 hours (see Example 4);
**FIG. 15** shows the effect of p38 inhibition on the RNA level of ICAM1 in HUVEC cells treated with "sCAR T mono" supernatant for 24 hours (see Example 4);
**FIG. 16** is a schematic timeline of the experiment described in Example 5;
**FIG. 17A** and **FIG. 17B** show the results of IVIS imaging of tumour cell fluorescence in the murine experiment described in Example 5;
**FIG. 18A** and **FIG. 18B** show murine CRS scores and CAR T cells/ml (see Example 5);
**FIG. 19** is a schematic timeline of the murine experiment described in Example 6;
**FIGs. 20A-C** show murine CRS scores in the murine experiment described in Example 6;
**FIG. 21** shows tumour cell proliferation during the murine experiment described in Example 6;
**FIGs. 22-29** show IFNγ **(****FIG. 22****),** TNF α **(****FIG. 23****),** IL-10 **(****FIG. 24****),** IL-6 **(****FIG. 25****),** IL-2 **(****FIG. 26****),** IL-4 **(****FIG. 27****),** IL-8 **(****FIG. 28****)** and MIP-1α **(****FIG. 29****)** levels during the murine experiment described in Example 6;
**FIG. 30A** shows mean CD19+ cancer cells per ml at days 9 and 10 during the murine experiment described in Example 6; and
**FIGs. 30B-30D** show mean hCD45+ cells/µl, hCD3+ cells/µl, and hCD4+ cells/µl at days 9 and 10 (day 9 only for **FIG. 30D****)** during the murine experiment described in Example 6.

### Detailed Description

While the subject-matter of the present disclosure is described and illustrated below with reference to particular embodiments, it will be appreciated by those skilled in the art that the subject-matter lends itself to many different variations not specifically illustrated herein. By way of example only, certain possible variations will now be described.

### Examples

The following Examples are provided to aid understanding of the subject-matter of the present disclosure and to assist in putting the subject-matter into practice. In particular, the Examples are intended to help understand the effect a p38 MAP kinase inhibitor may have on cellular responses to cancer immunotherapy which may be implicated in CRS.

CRS is studied in the present Examples on the basis of CRS induction by CAR T therapy or by anti-CD28 antibody (the latter being a T cell super-agonist, thus providing an excellent model for CRS induced by T cell activity); for which there exist relatively easily available research materials. Shared mechanisms mean it is reasonable to expect that the effect of a p38 MAP kinase inhibitor observed in the context of CRS induced by CAR T cell therapy or CRS induced by the T cell response to anti-CD28 antibody will extend to other cancer immunotherapies; in particular other cancer immunotherapies that comprehend the use of adoptive T cells or otherwise engage with T cells, as described herein. Such cancer immunotherapies may set in motion the signalling between T cells, monocytes and endothelial cells that is observed in CRS induced by CAR T cells or CRS induced by anti-CD28 antibody and which may be modulated by the p38 MAPK inhibitor of the present disclosure. Additionally or alternatively, the effect of a p38 MAPK inhibitor on signals 3 and/or 4 for T cells, and consequently on T cell survival, phenotype and memory, where beneficial in the context of CAR T or anti-CD28 antibody, can reasonably be expected to benefit other cancer immunotherapies, wherein T cells are involved.

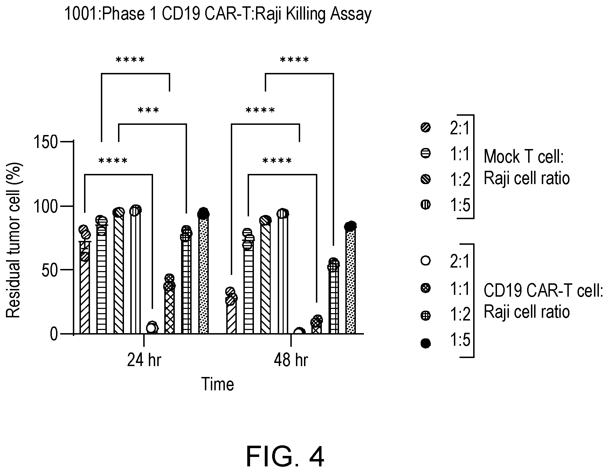

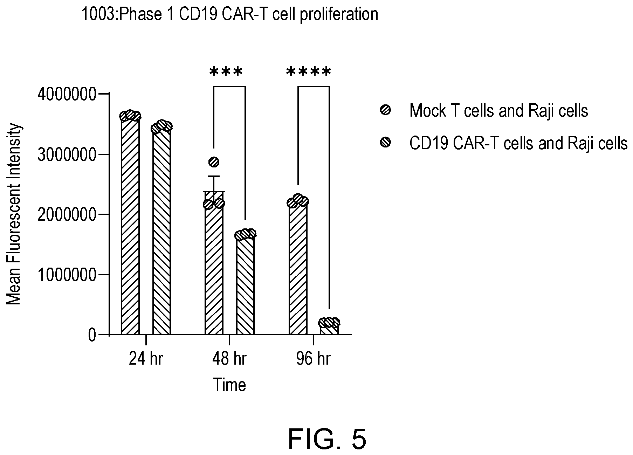

As a general statement, where applicable in the following Examples, statistical analyses were performed using GraphPad Prism version 10.1.2. A two-way ANOVA was used for data in **FIG. 4** and **FIG. 5****.** For all other figures a one-way ANOVA was fitted to the data and comparisons of interest were made using a Bonferroni test to adjust for multiple testing, using a 5% significance interval; p-values less than 0.05 were considered significant and are represented as follows in relevant Figures: *=p<0.05, **=p<0.01, ***=p<0.001, ****=p<0.0001. Where not graphically specified, significance values are in comparison to sCAR-T or CAR T only controls.

### Example 1 - Optimisation of CAR T cell : cancer cell co-culture ratio

The aim of Example 1 is to produce a co-culture of CAR T cells with cancer cells, having an optimised CAR T cell to cancer cell ratio and co-culture duration, for use in subsequent Examples. To summarise, the optimal ratio and duration are selected as 1:1 for a 24-hour co-culture based on CAR T cell expansion and cancer cell death relative to control.

T cells are engineered to express a CAR with specificity for the B-lymphocyte antigen CD19 ("CD19"; also known in the art as: CD19 molecule; B-Lymphocyte Surface Antigen B4; T-Cell Surface Antigen Leu-12; or CVID3). Transduction is confirmed via flow cytometry to demonstrate cell surface CAR expression (throughout the present Examples, flow cytometry is suitably carried out using an Agilent NovoCyte 3 cytometer, available from Agilent of 5301 Stevens Creek Blvd., Santa Clara, CA 95051, US).

The CAR sequence encodes a fusion protein composed of a leader sequence, CD19 specific single chain variable fragment, CD8α hinge, CD8α spacer and transmembrane domain, 4-1BB endodomain, CD3ζ endodomain, and T2A tag. An enhanced green fluorescent protein (EGFP) sequence is added to permit detection of CAR expression by fluorescence. The resultant cDNA is subcloned into a replication defective lentiviral or retroviral vector. To generate viral particles, triple transfection of vector, envelope and *gag-pol* genes are performed in 293T cells (an immortalized cell lined derived from human embryonic kidney cells carrying the SV40 origin of replication, available from the American Type Culture Collection). Thus, 293T cells are grown and transfected. Viral particles encoding for the CAR are harvested from their supernatant and are concentrated. Viral titre is measured; and virus is used to transduce activated T cells derived from human peripheral blood mononuclear cells (PBMCs) harvested from the peripheral blood of a healthy donor. T cells are expanded from the PBMCs in a medium containing IL-7 and IL-15, with treatment using CD3-and CD28-coupled beads. Thus, T cells are incubated in the presence of viral vector and the expression of the CAR is confirmed using flow cytometry.

"Mock" CAR T cells, having an irrelevant ectodomain (i.e., without antigen specificity for any cell in the culture) are used as a negative control.

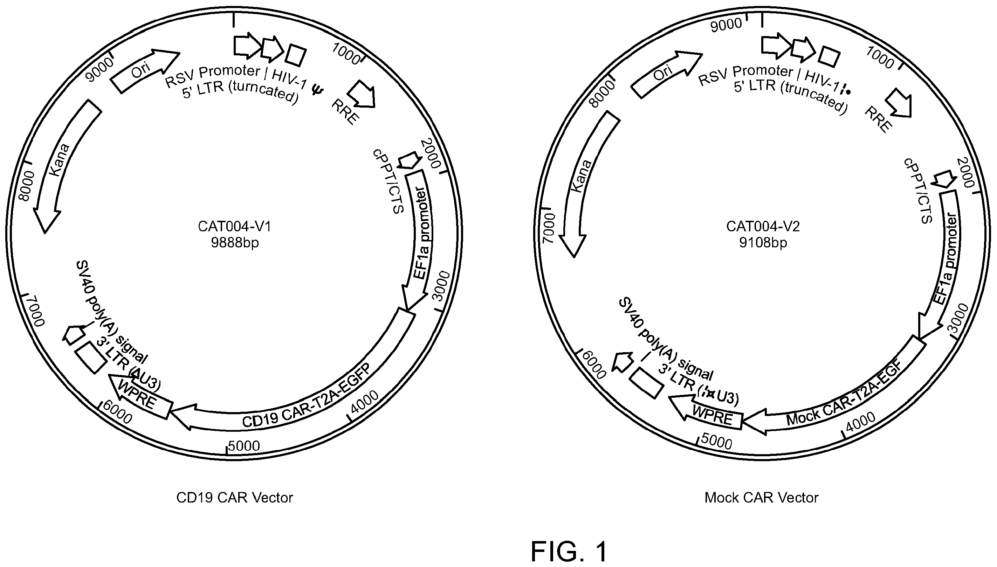

Schematic representations of the CD19 and mock CAR vectors are shown in **FIG. 1****.**

Cells are washed using phosphate buffered saline with a pH of 7.2. To confirm transduction, the co-expressed reporter gene enhanced green fluorescent protein (EGFP) is detected by fluorescence emission in flow cytometry; and CD19 specific CAR expression is confirmed by staining the cells with anti-CD19 antibody.

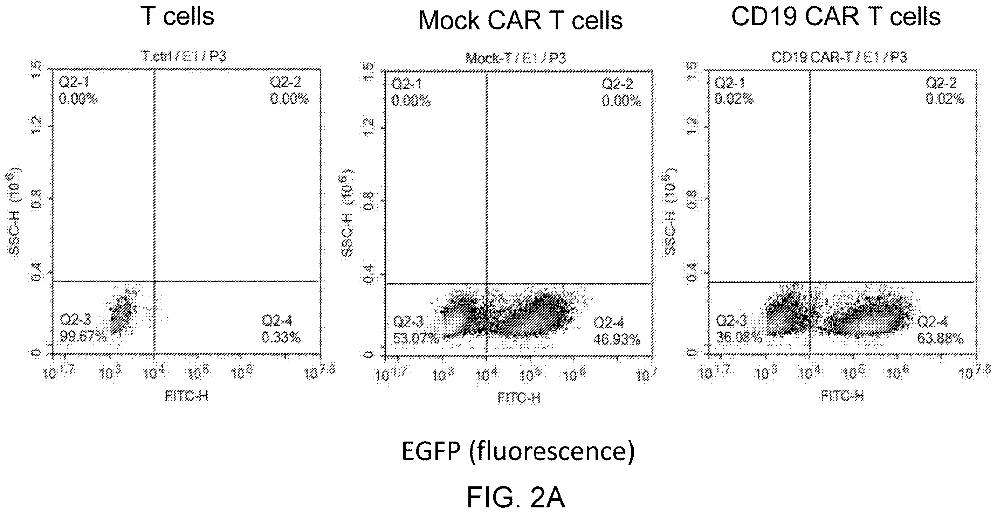

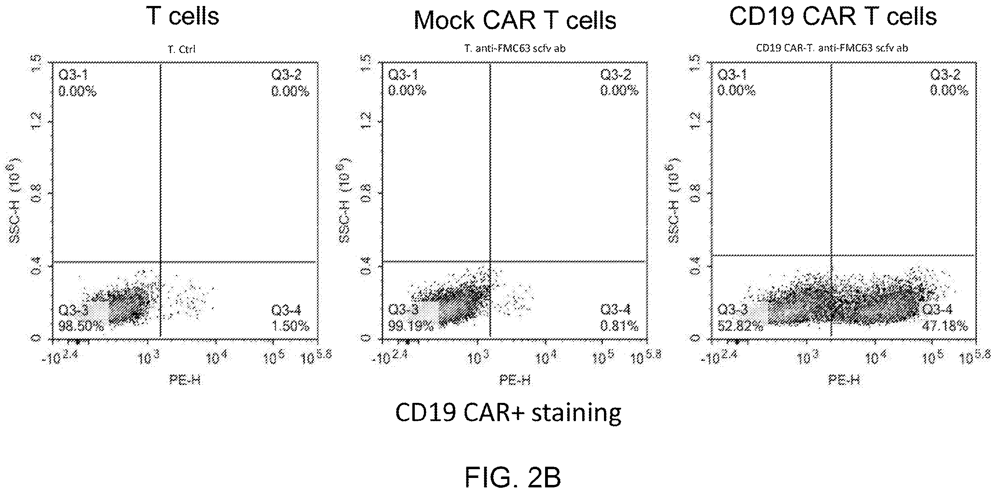

**FIG. 2A** and **FIG. 2B** respectively indicate cells' flow cytometry and staining results, confirming successful transduction.

(The process is repeated as required for the purposes of subsequent experiments. In each new transduction, gene transfer is confirmed using the described method and recorded in laboratory notebooks in a way that allows the relevant batch analysis to be linked to each subsequent experiment. Batches in this context refer to CAR T cells derived from the same donor and transduced as a unitary sample, which was subsequently subdivided as required for further experiments.)

Raji immortalized human B cell line CD19-expressing cancer cells (available from the American Type Culture Collection) are acquired and stable transfection of the Raji cell line is generated with an mCherry reporter protein to facilitate identification of the cancer cells within co-cultures by detection of the mCherry protein fluorescence by flow cytometry.

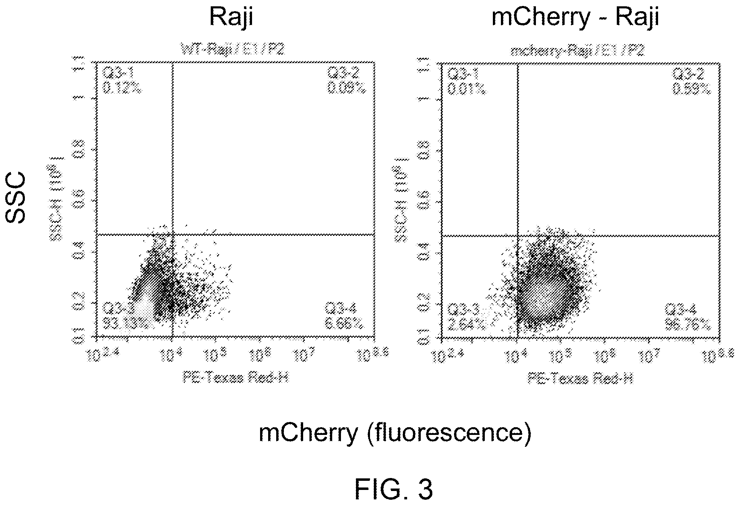

**FIG. 3** shows fluorescence of the mCherry tag in stably transfected Raji cells.

A co-culture of CAR T cells (or mock CAR T cells, for control) is then established with mCherry+ CD19-expressing cancer cells, in a liquid medium with a pH of 7.2, supplemented with nutrients and growth factors including L-arginine, L-glutamine and foetal bovine serum.

As shown in **Table 1** below, CAR T cells and cancer cells are incubated for 24 to 96 hours at 37 °C, 5% CO₂ in a humid atmosphere before cancer cell clearance is measured by flow cytometry. In this context, cancer cells may be detected as mCherry+ events and CAR T cells may be detected as EGFR+ events.

CO₂ levels are managed using a controlled pump system and air is filtered in a high-efficiency particulate absorbing (HEPA) filtration system. Ratios of CAR T cells (also referred to as effector cells) to tumour cells of 1:2, 1:1, 2:1 and 5:1 are investigated.

**Table 1 - CAR T cell: cancer cell ratios**

| **No.** | **Ratio CAR T cells: cancer cells** |
|---|---|
| 1 | 2:1 |
| 2 | 1:1 |
| 3 | 1:2 |
| 4 | 1:5 |

| | |
|---|---|
| *Duration of incubation is 24-96 hours at 37°C with 5% CO₂ at physiological pH.* | |

CAR T cell proliferation is confirmed by Cell Trace^{™} Violet staining of CAR T cells (staining agent having been added prior to addition of the T cells to the co-cultures with tumour cells). Staining may be observed, following co-culture, via flow cytometry.

Thus, **FIG. 4** shows CD19 CAR T (and mock CAR T) cell tumour cell killing at a range of effector to tumour cell ratios, as determined by the proportion of cancer cells in culture after 24 and 48 hours.

A ratio of CAR T cells to tumour cells of 1:1 was taken forward for investigation at different time points. Thus, **FIG. 5** shows CD19 CAR T and mock CAR T cell proliferation following incubation with tumour cells at an effector to tumour ratio of 1:1 for 24, 48 and 96 hours. (As shown in **FIG. 5****,** a decrease in fluorescence is related to the number of rounds of replication each cell, or progeny cell, has experienced during the co-culture.)

A ratio of CAR T cells to cancer cells of 1:1, co-cultured for 24 hours, is selected for use in subsequent experiments, by balancing two factors (in accordance with **FIG. 4** and **FIG. 5****):**
1. Firstly, a ratio at which T cells expand optimally as determined by Cell Trace^{™} Violet staining.
2. Secondly, a ratio at which a significantly larger frequency of tumour cell death is evident in the CD19 CAR T cell and tumour co-culture in comparison to the mock CAR T and tumour cell co-culture control.

A ratio of CAR T cells to cancer cells of 1:1, co-cultured for 24 hours, is expected to mimic successful CAR T treatment (or other cancer immunotherapy) in a clinical setting and thus to provide an optimal context for the assessment of CRS induction.

Supernatant isolated from the preferred ratio of CAR T cell and tumour cell co-culture (sCAR) of 1:1, co-cultured for 24 hours, is used for subsequent analysis and experimentation.

Supernatant (termed sCAR, or sCAR T) is harvested by isolating the media into a new container and exerting a centripetal force of 400G to sediment cells and debris. Cell- and debris-free supernatant is collected using a micropipette (for volumes of 0.01-2 ml) or a Pasteur pipette (for volumes over 2 ml).

All experiments using sCAR utilise a negative control of supernatant from the same 1:1 ratio of mock CAR T cells to tumour cells, also cultured for 24 hours (termed smCAR, or smCAR T).

In Example 3 below, sCAR (or smCAR) is added using a micropipette to an *in vitro* culture of monocyte cells of the human monocytic cell line THP-1, available from the American Type Culture Collection. The culture is incubated in a closed incubator at 37 °C with 5% CO₂ and a suitably humid atmosphere for approximately 24 hours, following which cytokine release is observed. p38 MAPK activation and cytokine release are confirmed by evaluation of the presence of IL-6 in THP-1 cells using an enzyme-linked immunosorbent assay (ELISA; for which a kit is suitably obtainable from Thermo Fisher Scientific of Kingfisher Dr, Swindon SN3 5BZ, UK).

In like manner and also in Example 3 below, sCAR (or smCAR) is added to endothelial (HUVEC) cells, available from the American Type Culture Collection, and using an equivalent protocol to the treatment of THP-1 cells (but with individually optimised conditions, such as the concentration of sCAR used), following which IL-6 release and IL-8 release are observed. Pro-inflammatory cytokines IL-6 and IL-8 are measured using an enzyme-linked immunosorbent assay (ELISA) and IL-6, ICAM1 and VCAM1 are measured by quantitative PCR (QPCR, for which a kit is suitably obtainable from Thermo Fisher Scientific of Kingfisher Dr, Swindon SN3 5BZ, UK).

### Example 2 - Addition of p38 MAP kinase inhibitor to CAR T cell/cancer cell co-culture

Example 2 investigates the effect of several p38 MAPK inhibitors, namely UR-13870, Ralimetinib and Acumapimod, on the CAR T cells in the described co-culture of cancer cells and CAR T cells; especially, their effect on features that contribute to CAR T cell anti-cancer cell efficacy and on features that contribute to the onset of CRS, such as the secretion of cytokines. Several concentrations of the p38 MAPK inhibitors were tested, as shown in **Table 2.** Each of UR-13870, Ralimetinib and Acumapimod are highly selective for p38, ensuring no (or minimal) off-target effects.

**Table 2 - p38 MAPK inhibitors**

| **p38 MAPK inhibitor** | **Concentration** | **Concentration Description** |
|---|---|---|
| UR-13870 | 128 nM | Low |
| *6-bis(4-fluorophenyl)-5-(4-pyridinyl)-2Hpyrazolo[3,4-b]pyridine-2-propanol* | 256 nM | Medium |
| (The compound of Formula II) | 1024 nM | High |
| Acumapimod | 100 nM | Low |
| *3-[5-amino-4-(3-cyanobenzoyl)pyrazol-1-yl]-N-cyclopropyl-4-methylbenzamide* | 500 nM | Medium |
| | 5 µM | High |
| Ralimetinib | 50 nM | Low |
| *5-[2-tert-Butyl-4-(4-fluorophenyl)-1H-imidazol-5-yl]-3-(2,2-dimethylpropyl)imidazo[4,5-b]pyridin-2-amine* | 500 nM | Medium |
| | 5 µM | High |

UR-13870, Ralimetinib or Acumapimod are each added to a culture of CD19+ cancer cells (using a micropipette to transfer the correct volume to achieve the desired final concentration in **Table 2).**

CAR T cells are then added to the same culture 5-10 minutes later. In brief, CAR T cell concentrations per ml are determined and used to aliquot the desired number of CAR T cells into the culture to achieve the described 1:1 CAR T to cancer cell ratio.

The co-culture containing CAR T cells and tumour cells is swirled gently to ensure homogenous dispersion of cells throughout the vessel. The resultant co-culture is incubated for 24 to 96 hours in a closed incubator at 37 °C with 5% CO₂ and a suitably humid atmosphere.

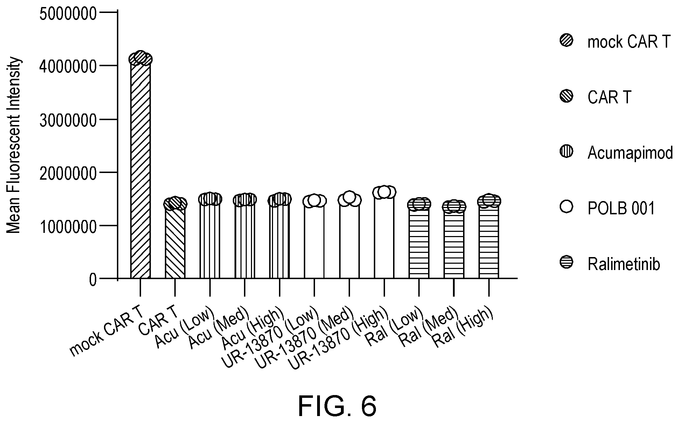

CAR T cell proliferation is confirmed by Cell Trace^{™} Violet staining observed via flow cytometry, as shown in **FIG. 6. FIG. 6** shows CD19 CAR T (and mock CAR T) cell-induced tumour cell death, at a range of CAR T to tumour cell ratios, as determined by the proportion of cancer cells remaining in the culture after 28 and 48 hours. Advantageously, UR-13870, Ralimetinib and Acumapimod each maintain CAR T cell proliferation. These results indicate that the p38 MAP kinase inhibitors may modulate post-activation events involved, in particular, in signal 4; having an impact on post-activation T cell phenotype, persistence and function.

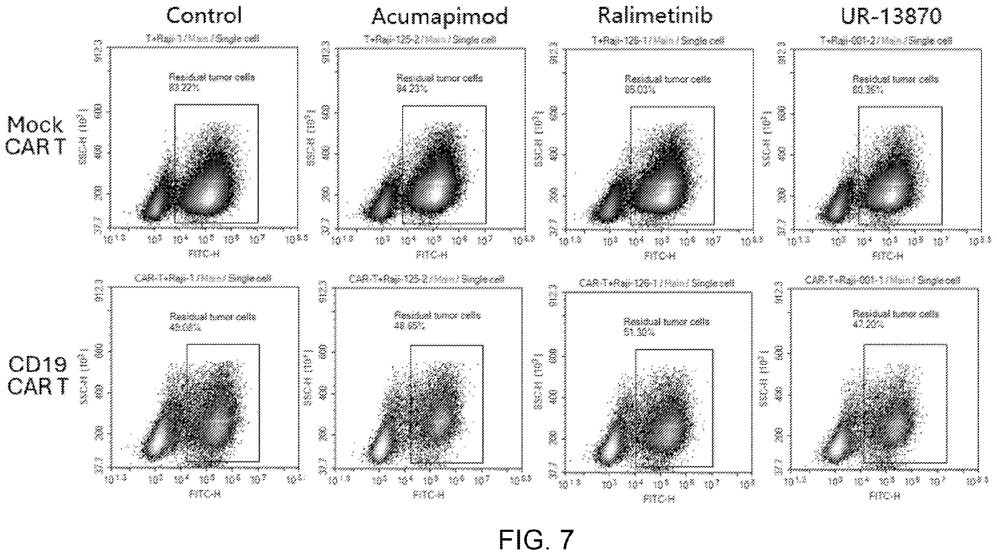

Tumour cell death is observed by examining relative CAR T cell: tumour cell numbers by flow cytometry following 24 hour co-culture, as shown in **FIG. 7****.** Thus, **FIG. 7** shows the effect of p38 inhibition (high dose only) on CAR T cell killing of tumour cells in a 24-hour co-culture with a CAR T to cancer cell ratio of 1:1. Advantageously, UR-13870, Ralimetinib and Acumapimod maintain CAR T cell-induced cancer cell death, as determined by the percentage of residual tumour cells in the co-culture.

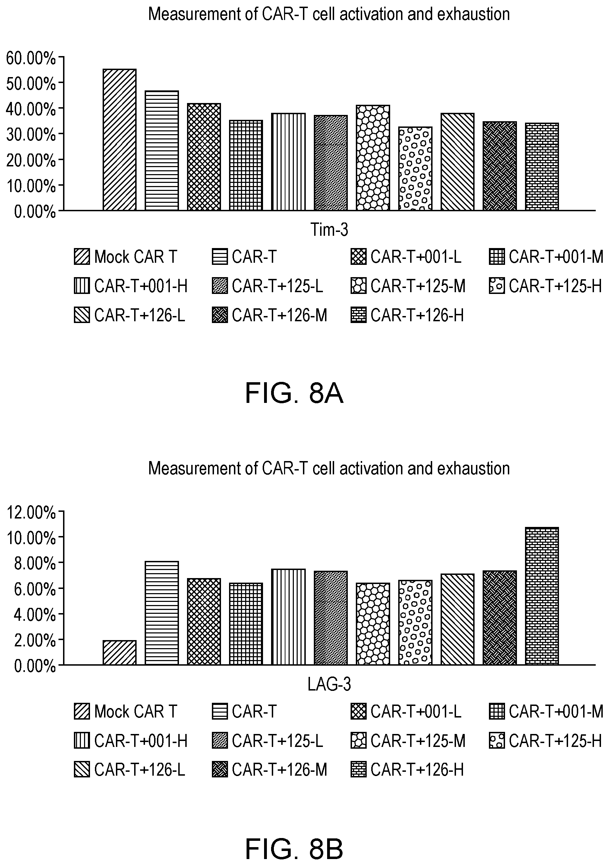

T cell markers are observed via flow cytometry, as shown in **FIG. 8A** and **FIG. 8B****.** Both T cell surface markers T cell immunoglobulin and mucin domain-containing protein 3 (TIM3/CD366) and Lymphocyte Activation Gene 3 (LAG3/CD223) are associated with T cell exhaustion and disease progression during immunotherapy treatment. Advantageously, all of UR-13870, Ralimetinib and Acumapimod reduce both the number of TIM3+ CAR T cells, and the number of LAG3+ CAR T cells. The reduction of TIM3 and LAG3 indicates that each of UR-13870, Ralimetinib and Acumapimod modulate T cell polarisation (signal 2), by preventing the induction of an exhausted T cell phenotype. For LAG3+, the results for Ralimetinib at a 5 µM concentration appear to have been affected by experimental failure, and these may be disregarded accordingly.

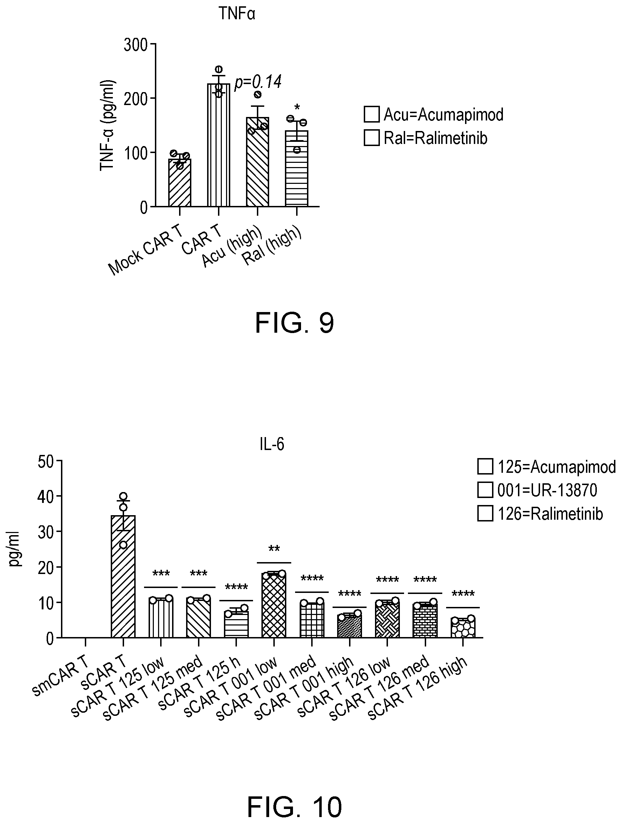

Cytokine release by T cells is also quantified by TNFα enzyme-based immunosorbent assays of co-culture supernatant, as shown in **FIG. 9****.** Ralimetinib and Acumapimod reduced TNF-α secretion following treatment. Data for UR-13870 were discarded due to failures in the UR-13870 experimental procedure.

### Example 3 - Addition of p38 MAP kinase inhibitor and sCART to (i) THP-1 monocytes and (ii) HUVEC endothelial cells

This experiment investigates the effect of each of UR-13870, Acumapimod and Ralimetinib on (i) monocytes and (ii) endothelial cells, which are stimulated with supernatant obtained from the described co-culture of cancer cells and CAR T cells (sCAR; or, in the case of control, smCAR), thus mimicking the environment of (i) monocytes and (ii) endothelial cells *in vivo* for a patient exposed to CAR T therapy. (See Example 1 for details.)

### (i) Monocytes

sCAR (or smCAR), produced as described in Example 1 above, is added to a monoculture of THP-1 monocytes in the presence of UR-13870, Ralimetinib or Acumapimod at each of the concentrations shown in **Table 2** above at 37 °C, 5% CO₂ and a suitably humid environment for 24 hours.

Supernatant levels of IL-6 from the THP-1 monocyte monoculture with sCAR (or smCAR) are determined by ELISA. The results are displayed in **FIG. 10****,** which shows that each of UR-13870, Ralimetinib and Acumapimod significantly reduce IL-6 secretion by THP-1 monocytes.

### (ii) Endothelial cells

sCAR (or smCAR), produced as described in Example 1 above, is added to a monoculture of HUVEC endothelial cells in the presence of UR-13870, Ralimetinib or Acumapimod at each of the concentrations shown in **Table 2** above, at 37 °C, 5% CO₂ and a suitably humid environment for 24 hours.

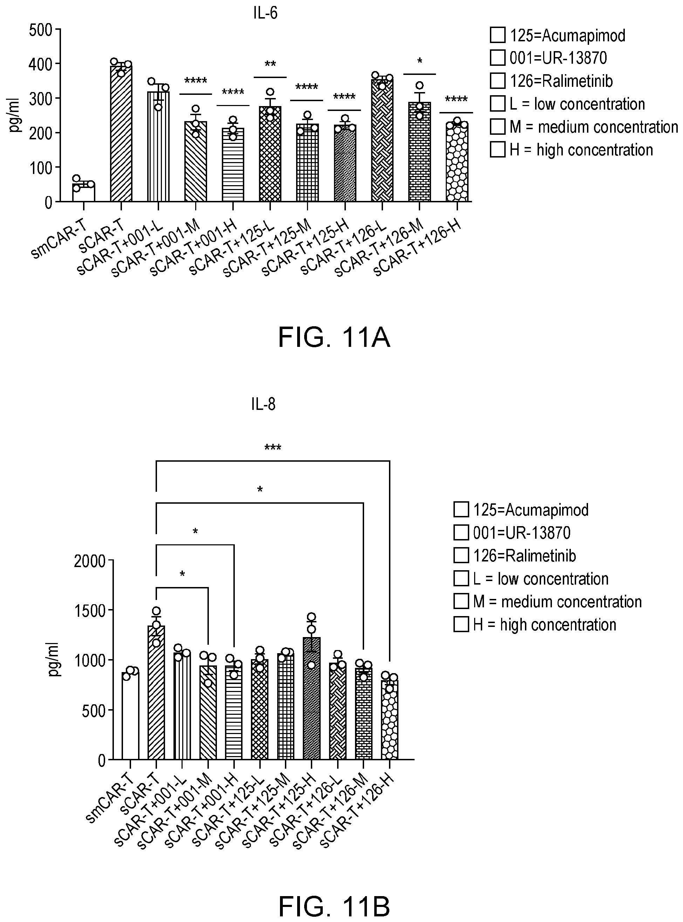

Supernatant levels of IL-6 and IL-8 from the HUVEC endothelial cell monoculture with sCAR (or smCAR) are determined by ELISA. As shown in **FIG. 11A****,** each of UR-13870, Ralimetinib and Acumapimod significantly reduce IL-6 secretion by HUVEC cells, IL-8 is also reduced, as shown in **FIG. 11B****.**

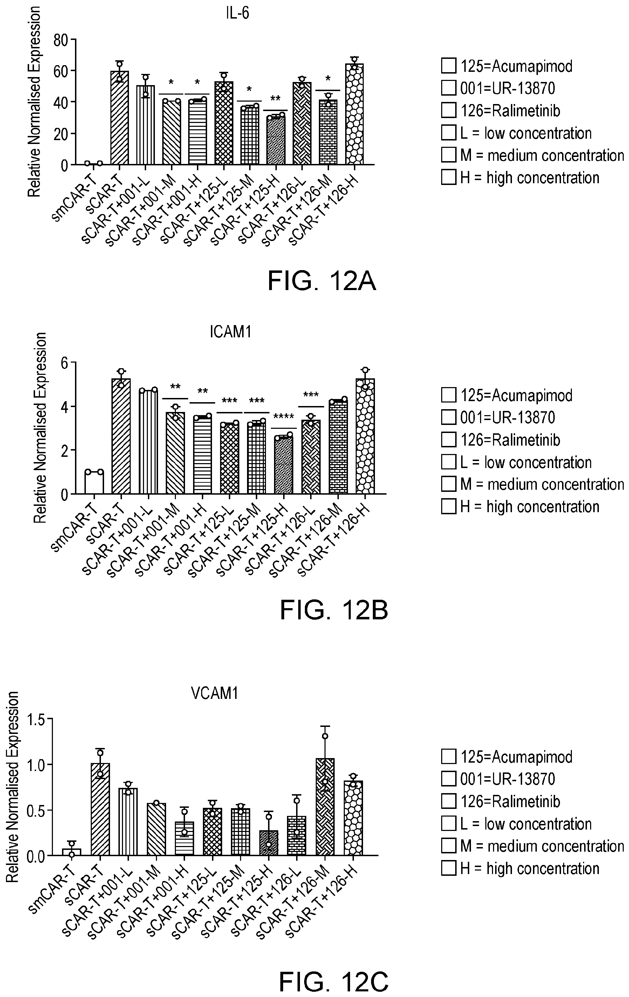

Endothelial cell activation is also evaluated by QPCR analysis of VCAM1, ICAM1 and IL-6, shown in **FIGS 12A-C**. Each of UR-13870, Ralimetinib and Acumapimod significantly reduce IL-6 and ICAM1 mRNA levels. VCAM1 mRNA levels are also reduced, albeit non-significantly.

### Example 4 - Effect of supernatant obtained from co-culture of monocytes with CAR T cells and cancer cells in the presence of p38 MAP kinase inhibitor, on endothelial cells

This experiment investigates the effect of each of UR-13870, Acumapimod and Ralimetinib on endothelial cells, which are stimulated with supernatant from an *in vitro* co-culture of cancer cells, CAR T cells and monocytes, thus providing a further approximation of the environment of endothelial cells *in vivo* for a patient exposed to CAR T therapy, and the effect thereupon of p38 MAPK inhibitors.

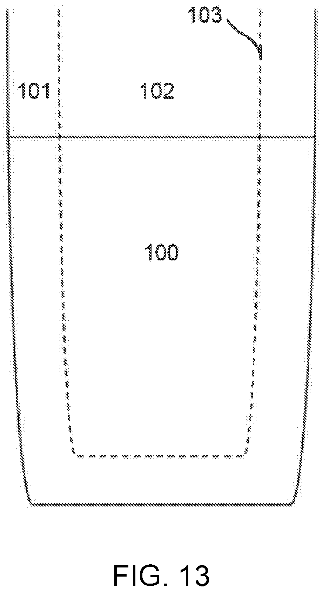

With reference to **FIG. 13** of the accompanying drawings, a co-culture (100) is established using a standard cell culture transwell dish, including a first compartment (101), containing CAR T cells and cancer cells (at a 1:1 ratio), which is separated from a second compartment (102), containing monocytes, by a semi-permeable membrane (103) composed of tissue-culture treated polyethylene terephthalate with a 0.4 µm pore size. The membrane is permeable to solubilised factors but does not allow cells to traverse from one compartment to the other.

Each of UR-13870, Acumapimod and Ralimetinib are administered to the co-culture following a procedure analogous to that described in Example 2 above (at the concentrations as shown in **Table 2** above).

The co-culture is incubated at 37 °C, 5% CO₂ and a suitably humid environment for 24 hours. On completion, the transwell insert is removed and supernatant is isolated. Cells and debris are removed from the supernatant by exposing the container to a centripetal force of 400 G and gently pipetting the supernatant into a new container without disturbing the cell pellet at the bottom of the previous container.

The resultant supernatant from the co-culture, termed sCART-mono, is then added using a micropipette to a HUVEC endothelial cell monoculture in the presence of UR-13870, Acumapimod or Ralimetinib at the respective concentrations as shown in **Table 2** above, 30 minutes after addition of the p38 MAPK inhibitors. Prior to adding the p38 inhibitors and sCART-mono, endothelial cells are incubated overnight at 37 °C and 5% CO₂ in a suitably humid environment to allow the cells to adhere fully to the tissue culture dish.

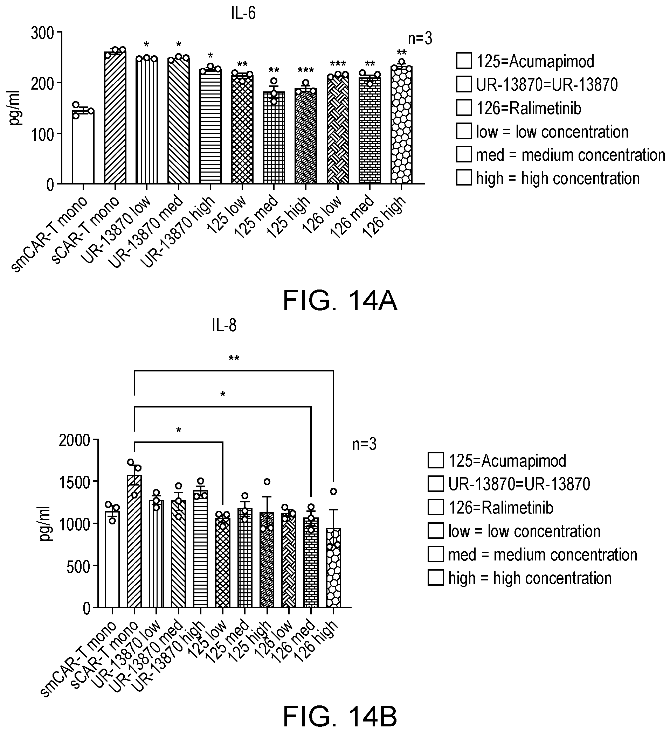

Supernatant levels of IL-6 and IL-8 are determined following 24 hr treatment with sCART-mono and the p38 MAPK inhibitors by ELISA. Results are shown in **FIG. 14A** and **FIG. 14B****.** UR-13870, Acumapimod and Ralimetinib reduced IL-6 levels in supernatant and show a (albeit non-significant) downward trend in IL-8 levels in supernatant.

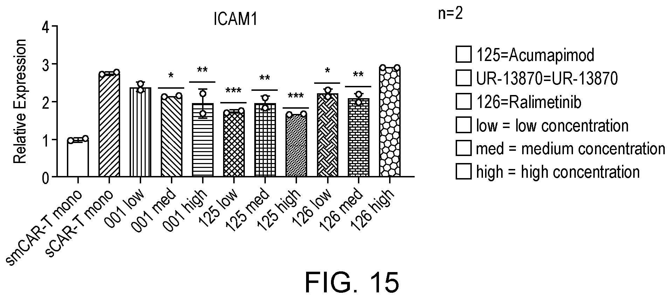

Endothelial cell activation is also evaluated by QPCR analysis of ICAM1, as shown in **FIG. 15****.** Reduced endothelial cell activation is evidenced by reduced ICAM1 expression in response to treatment with UR-13870, Acumapimod and Ralimetinib.

### Example 5 - In vivo (murine) model using CAR T for CRS induction

Example 6 investigates the effect of p38 MAPK inhibition on CRS induced by CAR T in cancerous mice, as well as the effect of p38 MAPK inhibition on the CAR T cells themselves.

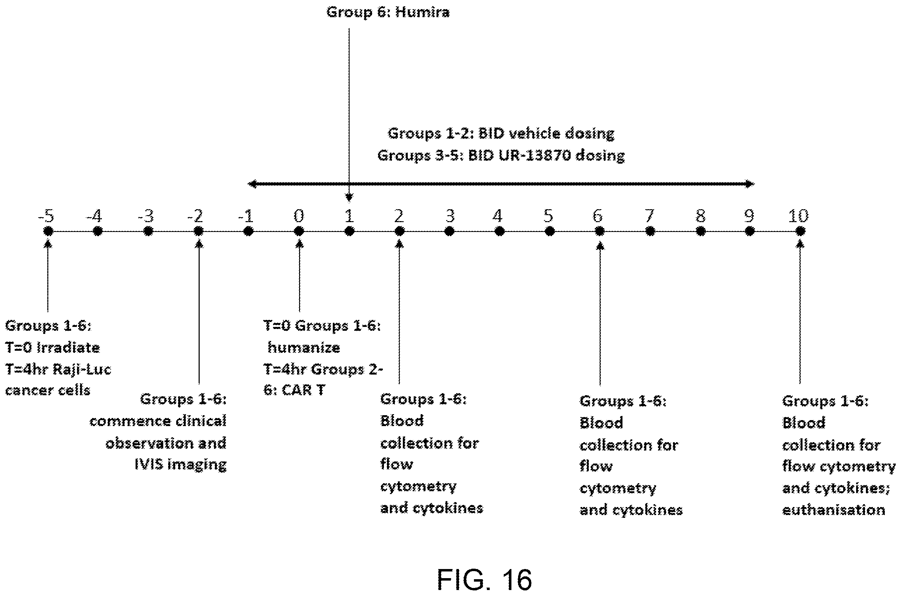

An outline of the timeline of the model is provided in **FIG. 16****;** it commences on Study Day -5 and ends on Study Day 10.

The timing of the model is designed to assess the effect of administering a p38 MAPK inhibitor, UR-13870 (highly selective for p38, thus with no or minimal off-target effects) *prior* to the onset of CRS in the studied PBMC-humanized NSG-MHC I/II double knock-out mice.

Thus, on Study Day -5, 30 female 6- to 8-week-old NSG-(KbDb)null (IA)null mice are irradiated at 100 cGy. 4 hours later, on the same day (Study Day -5) they are each injected, via tail vein, with 0.25x10⁶ Raji-Luc CD19-expressing cancer cells (a Raji wild-type cell line with stable and high expression of luciferase, enabling detection of cancer burden by fluorescence).

On Study Day -2, clinical observation commences for all 30 mice (including body weight monitoring) and all 30 mice are imaged using *in vivo* fluorescence imaging (IVIS). For IVIS, mice are intraperitoneally injected with 150 mg/kg D-Luciferin. 10 minutes after the D-Luciferin injection, mice are imaged dorsally and ventrally (auto exposure) using an IVIS Lumina system (obtainable from PerkinElmer of Chalfont Road, Seer Green, Beaconsfield, HP9 2FX, UK). Region of interest (ROI) are drawn for each mouse to capture its whole body, and total flux (p/s) is reported. IVIS imaging of all 30 mice continues on a biweekly basis throughout the study, thereby monitoring cancer cell burden. Daily clinical observation continues throughout the study, including observation of CRS, CRS being scored as follows:
0 = normal activity
1 = normal activity, hunched +/- piloerection
2 = hunched with reduced activity but will still move around the cage without continued stimulation.
3 = Not moving unless being stimulated; Will move when touched but will stop moving soon after hand is removed.
4 = moribund (non-responsive to touch)
25 of the mice are injected intraperitoneally, on Study Day 0, with 5 x10⁶ anti-CD19 CAR T cells in phosphate-buffered saline.

As a control, a group consisting of 5 of the mice ("Group 1") are not administered CAR T cells. Beginning on Study Day -1 and ending on Study Day 9, Group 1 receive treatment with 0.5% (w:v) HPMC, 0.1% (w:v) Tween 80 in sWFI (vehicle/control), administered BID at 5 mL/kg.

The 25 mice administered CAR T cells are divided into groups of 5 mice per group. Administration of a control drug (Humira), vehicle or UR-13870 is carried out in accordance with the following protocol:
Group 2 - 0.5% (w:v) HPMC, 0.1% (w:v) Tween 80 in sWFI (hereafter referred to as 'vehicle'), 5 mL/kg BID, beginning on Study Day -1 and ending on Study Day 9;
Group 3 - UR-13870 (formulated in vehicle), 2 mg/kg, twice daily (BID) beginning on Study Day -1 and ending on Study Day 9;
Group 4 - UR-13870 (formulated in vehicle), 10 mg/kg, twice daily (BID) beginning on Study Day -1 and ending on Study Day 9;
Group 5 - UR-13870 (formulated in vehicle), 25 mg/kg, twice daily (BID) beginning on Study Day -1 and ending on Study Day 9; and
Group 6 - Humira (formulated in PBS pH 7.2) (further, positive control) 5 mg/kg, once on Study Day 1.

Dosing of UR-13870 or vehicle is by subcutaneous injection. Subcutaneous injection sites are rotated for each dose (rotating among right flank, left flank, right inguinal region, left inguinal region). For UR-13870 and vehicle, AM and PM (BID) doses are scheduled at least 6 hours apart. Dosing of Humira is by intraperitoneal injection and occurs once, approximately 24 hours after CAR T administration.

On Study Day -1, after the first dose of UR-13870 is administered to Groups 2-6 but 4 hours before CAR T cells are administered to Groups 2-6, mice in Groups 1-6 are humanised by IV dosing with 10 x10⁶ human peripheral blood mononuclear cells (PBMCs).

On Study Days 2, 6 and 10, blood is collected from each of the 30 mice in Groups 1-6 (a 50-300 µl blood sample from each mouse, each time), for flow cytometry analysis, including analysis of CAR T cells per µl. It should be noted that, where relevant to BID dosed groups, blood collection is carried out between AM and PM dosing.

All mice in Groups 1-6 are euthanized by CO₂ asphyxiation on Study Day 10.

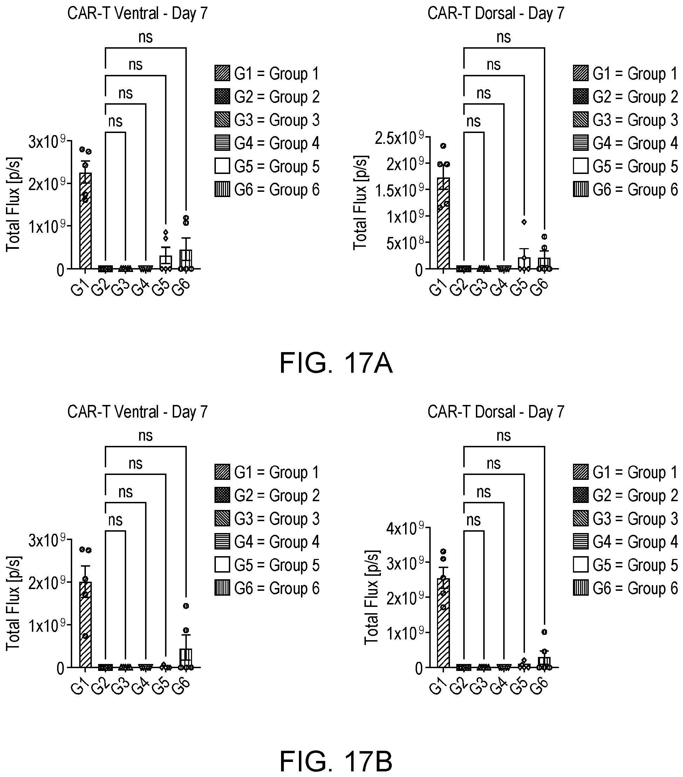

CAR T cell therapy remained efficacious in the presence of UR-13870, as indicated by tumour burden (as measured by IVIS) on day 7 and day 9, see **FIG. 17A** and **FIG. 17B** (nor does Humira).

CAR T cells successfully expanded and CAR T cell expansion was successfully maintained in the presence of UR-13870 (and in the presence of the positive control, Humira) (as measured by flow cytometry), see **FIG. 18A****.** In fact at day 10, high dose UR-13870 significantly enhanced the number of CAR T cells in circulation, see **FIG. 18A****.**

CRS scores were mild during the experiment (as indicated by CRS scores of 1 even in group 2) therefore for this model, the anti-inflammatory effect of neither UR-13870 nor Humira were parameterizable by CRS scoring of impact on cytokine levels in peripheral blood: see **FIG. 18B****.**

### Example 6 - In vivo (murine) model using anti-CD28 antibody for CRS induction

Example 6 investigates the effect of p38 MAPK inhibition on CRS induced by anti-CD 28 antibody (a T cell super-agonist) activation of native T cells in cancerous mice, as well as on the T cells themselves.

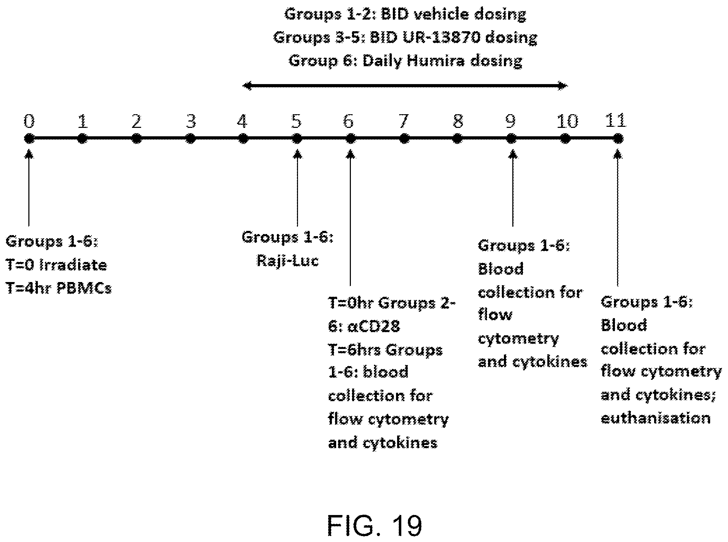

An outline of the timeline of the model is provided in **FIG. 19****;** it commences on Study Day 0 and ends on Study Day 10. It assesses the effect of administering a p38 MAPK inhibitor, UR-13870 (highly selective for p38, thus with no or minimal off-target effects) on anti-CD28 toxicity in PBMC humanized NSG-MHC I/II double knock-out mice, when the p38 MAPK inhibitor is administered *prior* to activation of native T cells by anti-CD28 antibody. It thus models the effect of administering a p38 MAP kinase inhibitor *prior* to the onset of CRS in accordance with the present disclosure.

Anti-CD28 antibody acts as a super-agonist of T cells (i.e., strongly activates T cells) resulting in T cell-mediated tumour cell death, with a rapid consequent release of cytokines and knock-on effects for monocytes and endothelial cells; thus modelling CRS induced by various different types of T cell-involving cancer immunotherapy.

Thus, on Study Day 0, 30 female 6- to 8-week-old NSG-(KbDb)null (IA)null mice are irradiated at 100 cGy. Approximately 4 hours later, on the same day (Study Day 0) all mice are humanised by IV dosing with 15 x10⁶ human peripheral blood mononuclear cells (PBMCs).

On Study Day 5, all mice are intravenously dosed with 0.25x10⁶ Raji-Luc CD19-expressing cancer cells (a Raji wild-type cell line with stable and high expression of luciferase, enabling detection of cancer burden by fluorescence). Approximately 4 hours later, mice are imaged using *in vivo* fluorescence imaging (IVIS). For IVIS, mice are IP injected with 150 mg/kg D-Luciferin. 10 minutes after the D-Luciferin injection, mice are imaged dorsal and ventral; auto exposure) using an IVIS Lumina system (obtainable from PerkinElmer of Chalfont Road. Seer Green, Beaconsfield, HP9 2FX, UK). Regions of interest (ROI) are drawn for each mouse to capture its whole body, and total flux (p/s) is reported. Tumour burden continues to be monitored by IVIS twice per week, starting on Study Day 5, until the end of the study. Daily clinical observation continues throughout the study, including observation of CRS, CRS being scored as follows:
0 = normal activity
1 = normal activity, hunched +/- piloerection
2 = hunched with reduced activity but will still move around the cage without continued stimulation.
3 = Not moving unless being stimulated; Will move when touched but will stop moving soon after hand is removed.
4 = moribund (non-responsive to touch)

On Study Day 6, 25 of the 30 mice are treated with 1mg/kg anti-CD28.

As a control, a group consisting of the 5 remaining mice ("Group 1") are not administered anti-CD28. Beginning on Study Day 4 and ending on Study Day 10, Group 1 receive treatment with 0.5% (w:v) HPMC, 0.1% (w:v) Tween 80 in sWFI (vehicle/control), administered BID at 5 mL/kg.

The 25 mice administered anti-CD28 are divided into groups of 5 mice per group. Administration of a control drug (Humira), vehicle or UR-13870 is carried out in accordance with the following protocol:
Group 2 - Vehicle only, BID, beginning on Study Day 4 and ending on Study Day 10;
Group 3 - UR-13870 (formulated in vehicle), 2 mg/kg, twice daily (BID) beginning on Study Day 4 and ending on Study Day 10;
Group 4 - UR-13870 (formulated in vehicle), 10 mg/kg, twice daily (BID) beginning on Study Day 4 and ending on Study Day 10;
Group 5 - UR-13870 (formulated in vehicle), 25 mg/kg, twice daily (BID) beginning on Study Day 4 and ending on Study Day 10; and
Group 6 - Humira (formulated in PBS) (further, positive control) 5 mg/kg, once daily beginning on Study Day 4 and ending on Study Day 10.

Dosing of UR-13870 or vehicle is by subcutaneous injection. Subcutaneous injection sites are rotated for each dose (rotating among right flank, left flank, right inguinal region, left inguinal region). For UR-13870 and vehicle, AM and PM (BID) doses are scheduled at least 6 hours apart. Dosing of Humira is by intraperitoneal injection.

On Study Days 6, 9 and 10, blood is collected from each of the 30 mice in Groups 1-6 (a 50-200 µl blood sample from each mouse, each time), for flow cytometry analysis, including analysis of CD19+ cancer cells per ml; and cytokine measurement. It should be noted that, where relevant to BID dosed groups, blood collection is carried out between AM and PM dosing.

All mice in Groups 1-6 are euthanized by CO₂ asphyxiation on Study Day 10.

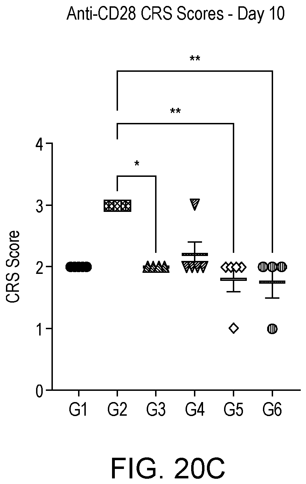

UR-13870 significantly reduces CRS scores (as does Humira, confirming positive control). Scores across the study are shown in **FIG. 20A****;** day 9 and 10 scores are shown in **FIG. 20B** and **FIG. 20C** respectively.

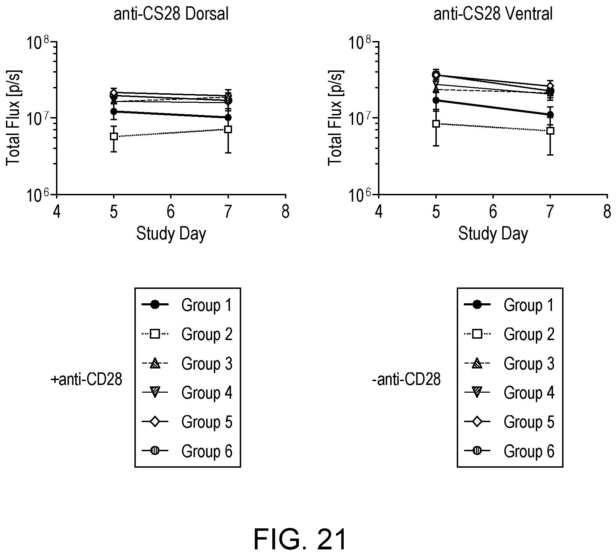

All experimental arms show consistent tumour burden across measurements taken from Day 5 to Day 7 (mid-dosing regime with UR-13870 and Humira), indicating UR-13870 does not increase tumour cell proliferation and maintains T cell mediated cancer cell death (and nor does Humira). See **FIG. 21****.** This suggests the p38 MAP kinase inhibitor is effective to prevent CRS without unduly attenuating the anti-cancer cell efficacy of the T cells in the presence of cancer cells.

Proinflammatory cytokines are measured from blood serum samples using Luminex cytokine analysis (Millipore Sigma Cat#HCYTA-60k, Human IFNγ, TNF, IL-10, IL-6, IL-4, IL-2, MIP-1α and IL-8), according to manufacturer's guidelines and analysed on a BioRad BioPlex200 Luminex plate reader (kit available from Sigma Aldrich/ Merck Life Science UK Limited of The Old Brickyard, New Rd, Gillingham, SP8 4XT, UK).

T cell phenotypes are observed from blood samples using flow cytometry to measure cytotoxicity and polarization (by observation of hCD45, hCD3 and hCD4). Peak cytokines are seen at day 9 for all cytokines tested except for IL-8 which continued to rise at day 10.

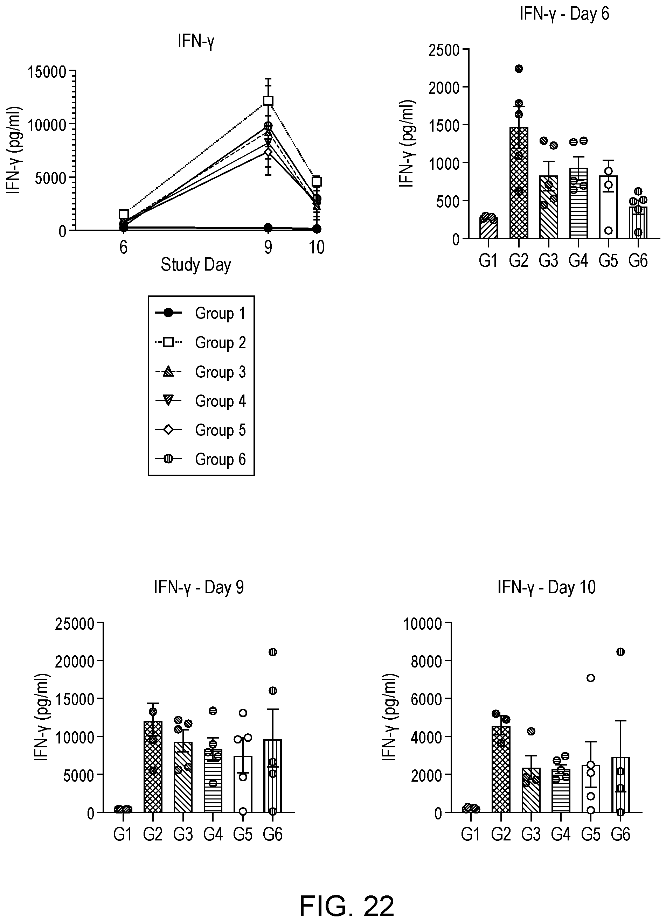

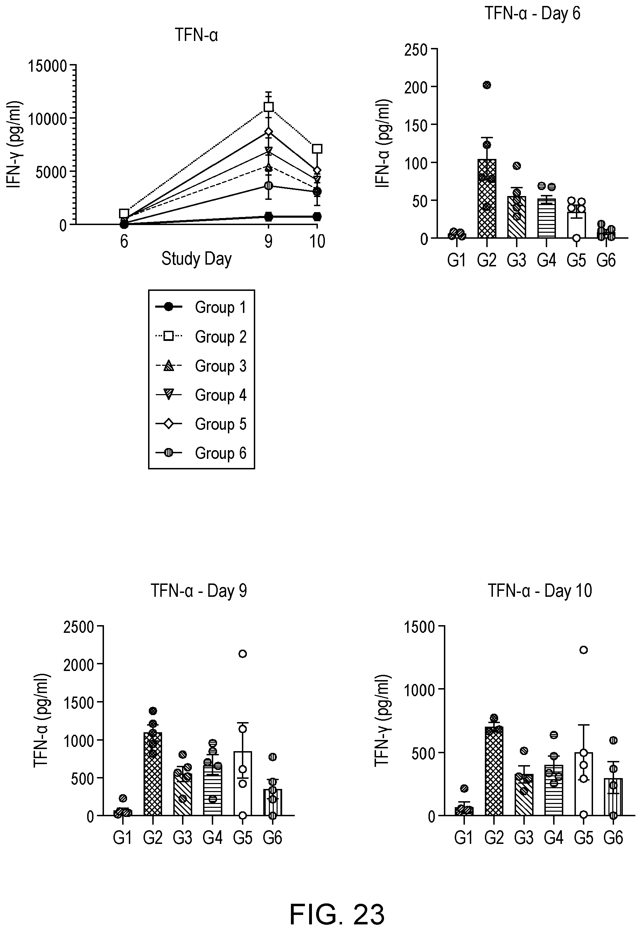

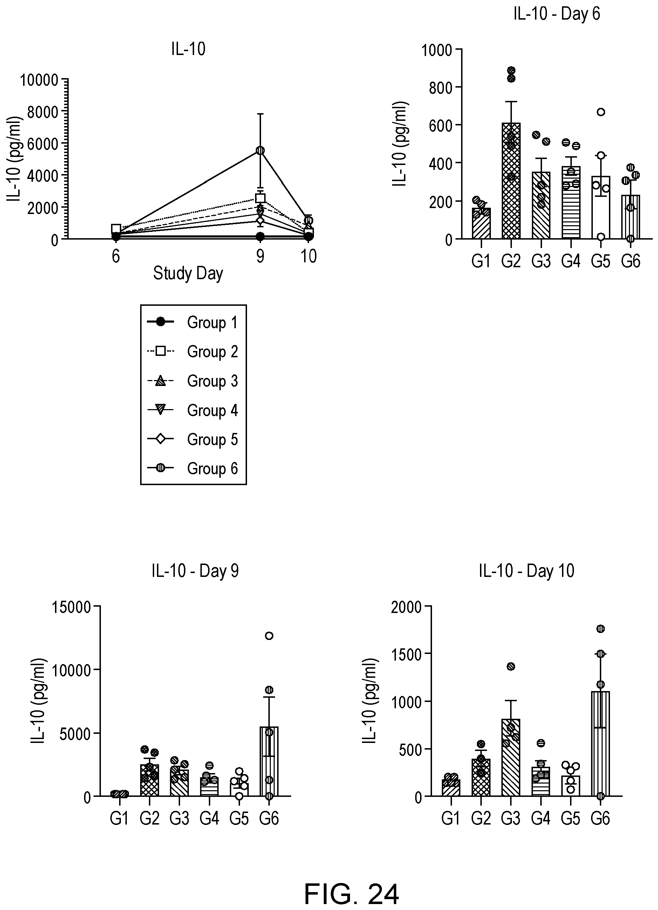

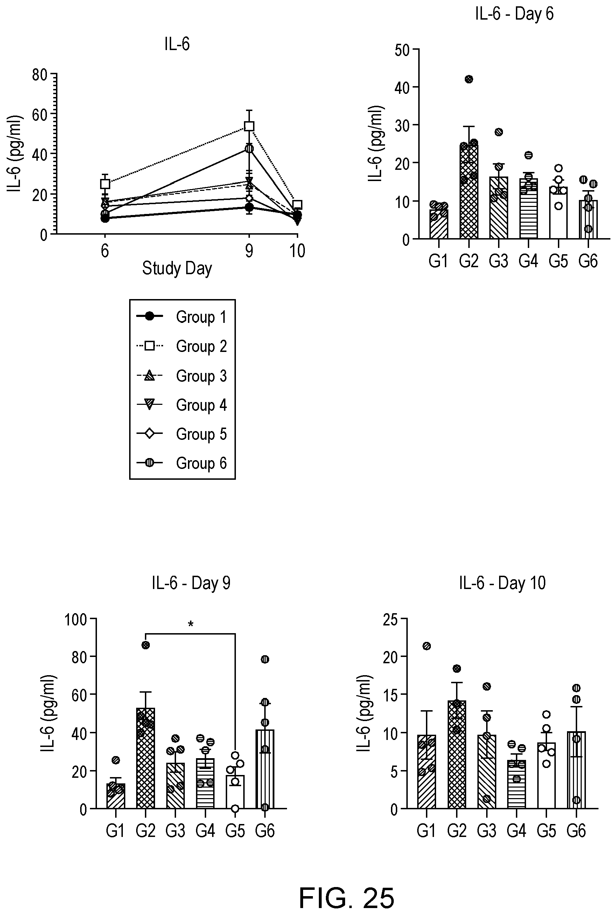

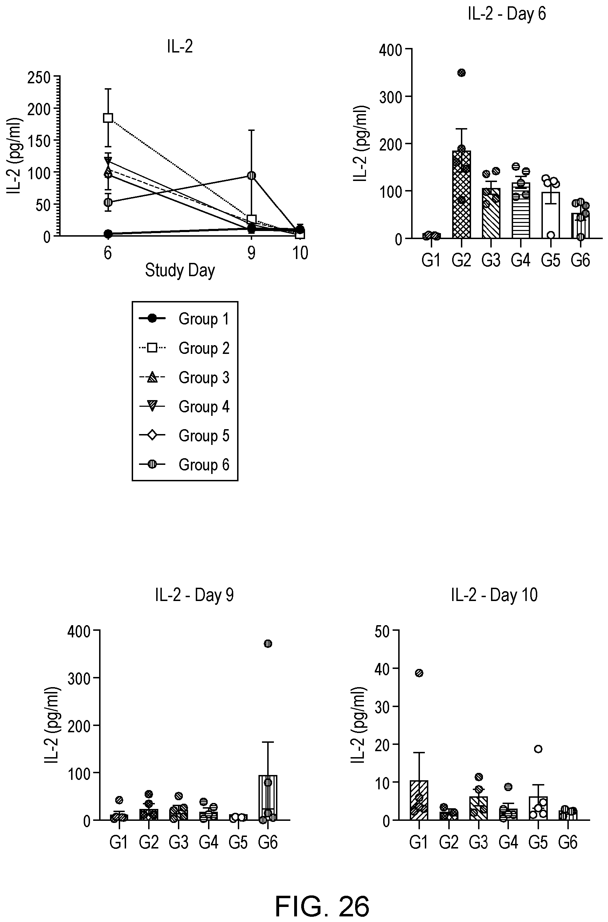

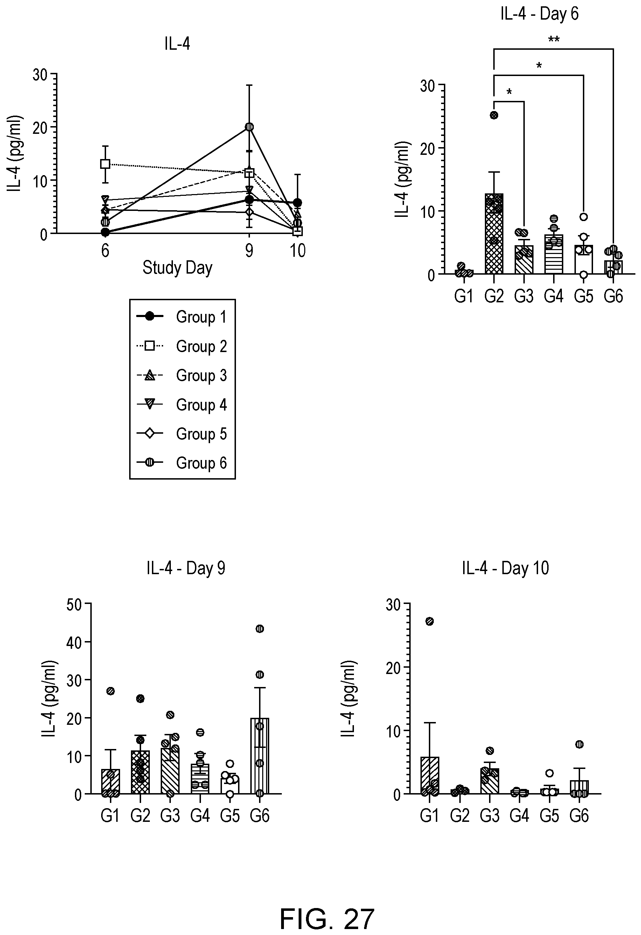

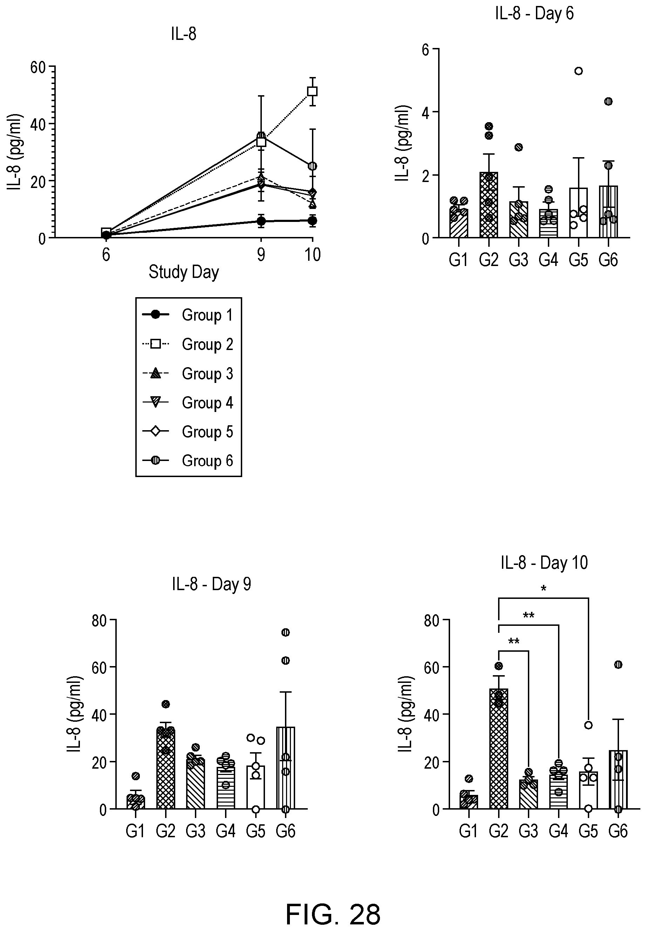

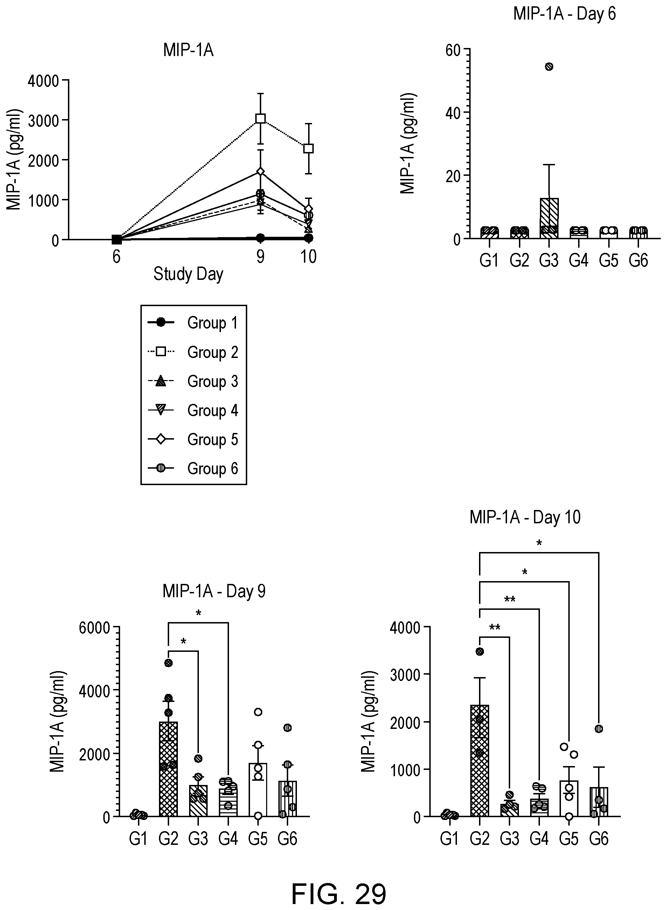

UR-13870 (and Humira) reduced key cytokines, including IFNγ **(****FIG. 22****),** TNF α **(****FIG. 23****),** IL-10 **(****FIG. 24****),** IL-6 **(****FIG. 25****),** IL-2 **(****FIG. 26****),** IL-4 **(****FIG. 27****),** IL-8 **(****FIG. 28****)** and MIP-1α **(****FIG. 29****).** This encompasses cytokines released by all of T cells, monocytes and endothelial cells.

Counts of CD19+ cancer cells per ml supported observations that neither UR-13870 or Humira increased proliferation of tumour cells. Furthermore, a downward trend was seen in mean CD19+ cancer cells per ml following treatment with UR-13870 compared to CD28 stimulation only at days 9 and 10 (especially at day 10; a similar trend was observed for Humira at day 10 only) **(****FIG. 30A****).**

Neither UR-13870 or Humira had a significant impact on hCD45+ cells/µl, hCD45+ hCD3+ cells/µl, or hCD45+ CD3+ hCD4+ cells/µl **(****FIGs. 30B****-D;** with serial gates on the data, in that order). This suggests that p38 MAP kinase inhibition may increase T cell anti-cancer efficacy.

1 animal in group 2 was euthanised on day 8 and 1 animal each from groups 2, 3 and 6 were euthanised on day 9, due to acute effects of CD28 antibody and tumour burden.

Various effects on T cells in the presence of cancer cells, as shown in Examples 2 to 6 (including upon administration of a p38 MAP kinase inhibitor *in vivo* beginning prior to anti-CD28 antibody administration to mice, modelling administration prior to CRS onset in humans) may be summarised in the following non-limiting Table 3:

**Table 3**

| **No.** | **Effect** | **Commentary on results** |
|---|---|---|
| 1 | Reduced pro-inflammatory cytokine release by T cells. | Effective reduction. |
| 2 | Modulation of T cell signals 3 and/or 4. | Indirectly evidenced by T proliferation and anti-cancer cell efficacy (especially suggestive of beneficial effect of p38 MAPK inhibition on signal 4). |
| 3 | Modulation of T cell polarisation and/or reduction of T cell exhaustion. | Evidenced by TIM3 and LAG3 reduction. |
| 4 | Maintained or increased T cell anti-cancer cell efficacy. | At least maintained throughout; increase appears *in vivo* in Example 6. |
| 5 | Maintained or increased proliferation of T cells. | At least maintained throughout; increase appears *in vivo* in in Example 6. |

Meanwhile, beneficial effects on monocytes and endothelial cells, which may assist in the reducing or preventing CRS, may be summarised in the following non-limiting **Table 4:**

**Table 4**

| **No.** | **Effect** | **Commentary on results** |
|---|---|---|
| 6 | Reduced pro-inflammatory cytokine release by monocytes. | Yes, especially IL-6 *in vitro* and a range of cytokines *in vivo.* |
| 7 | reduced pro-inflammatory cytokine release by endothelial cells. | Yes, especially IL-6 (and IL-8) *in vitro* and a range of cytokines *in vivo.* |

### Example 7 - Administration to a patient

About 4 days to about 2 hours prior to the administration of cancer immunotherapy (such, for example, as the adoptive transfer of CAR T cells) to a patient in need thereof, the patient begins an oral dosage regime of about 5 mg BID to about 1000 mg BID of a p38 MAPK inhibitor, such as UR-13870 (the compound of Formula II); for example, about 70 mg BID or about 150 mg BID (depending on the age, sex, weight, condition, general health and prior medical history of the patient being treated) of UR-13870 (the compound of Formula II).

For up to about 15 days following the administration of cancer immunotherapy (which occurs in a single dose, e.g., adoptive transfer of CAR T cells), the patient continues the oral BID dosage regime of the p38 MAPK inhibitor, such as UR-13870. For example, an oral dose containing about 70 mg or about 150 mg of UR-13870 is administered BID.

Throughout, the patient is monitored by a healthcare provider for fever, hypotension and hypoxia. The patient may be provided with supportive care if needed, including analgesics and/or antipyretics. CRS grading during monitoring is in accordance with American Society for Transplantation and Cellular Therapy Consensus Guidelines (2019) as set out in **Table 5.**

**Table 5**

| **CRS Parameter** | **Grade 1** | **Grade 2** | **Grade 3** | **Grade 4** |
|---|---|---|---|---|
| Fever^{†} | Temperature ≥ 38°C | Temperature ≥ 38°C | Temperature ≥ 38°C | Temperature ≥ 38°C |
| with either: | | | | |
| Hypotension^{†} | None | Not requiring vasopressors | Requiring one vasopressor with or without vasopressin | Requiring multiple vasopressors |
| and/or: * | | | | |
| Hypoxia^{†} | None | Requiring low-flow nasal cannula (≤ 6 L/min) | Requiring high-flow nasal cannula (> 6 L/min), facemask, non-rebreather mask, or Venturi mask | Requiring positive pressure (e.g.: CPAP, BiPAP, intubation and mechanical ventilation) |

| | | | | |
|---|---|---|---|---|
| *^{†}Not attributable to any other cause.* **CRS grade is determined by the more severe event.* | | | | |

Prevention or reduction of the severity of signs or symptoms of CAR T cell therapy-associated CRS in the patient may be ascertained by observation of attenuation or prevention of one or more of the symptoms and signs set out in Table 5. There may be observed a diminishing or averting of fever. There may be observed a delay in the onset of fever (e.g., for one, two, three, four or five days). There may be observed maintenance of a rectal, ear or temporal artery temperature of below about 38 °C, such, for example, as during a period of one, two, three or more weeks following the administration of CAR T cell therapy. Hypotension, hypoxia and/or organ dysfunction may be averted or at least attenuated. Death may be averted. The time period during which the above-described desirable effects are observed may last for one week, two weeks, three weeks or longer. It will be appreciated that while patient malaise induced by CRS may be attenuated or averted, there is no guarantee of patient malaise induced by other causes (for example, an underlying cancer) being attenuated or averted.

## Claims

1. A p38 MAP kinase inhibitor for use in preventing or reducing the severity of signs or symptoms of cancer immunotherapy-associated cytokine release syndrome ("CRS") in a human patient, wherein the p38 MAP kinase inhibitor is administered prior to the onset of CRS.

2. A p38 MAP kinase inhibitor for use according to claim 1, wherein preventing or reducing the severity of signs or symptoms of cancer immunotherapy-associated CRS in a human patient is or comprises preventing or reducing the severity of fever.

3. A p38 MAP kinase inhibitor for use according to claim 1 or claim 2, wherein the cancer immunotherapy is: a T cell-engaging therapy; TIL therapy; CAR NK cell therapy; a cancer vaccine; a T cell checkpoint modulator therapy; or a CAR T cell therapy.

4. A p38 MAP kinase inhibitor for use according to claim 3, wherein the T cell-engaging therapy is selected from BiTE therapy, therapy with another bispecific antibody, CiTE therapy, SMITe therapy and TriKE therapy.

5. A p38 MAP kinase inhibitor for use according to any one of the preceding claims, wherein the p38 MAP kinase inhibitor inhibits the release from monocytes of pro-inflammatory mediators including one or more of: IFNγ, IL-1β, IL-2, IL-6, IL-8, IL-10, IL-12, IL-18, CCL2, CCL5, CXCL10, MCP-1, MIP-1β, GM-CSF, VEGF and TNFα; and/or wherein the p38 MAP kinase inhibitor inhibits the release from endothelial cells of pro-inflammatory mediators including one or more of: IL-1β, IL-2, IL-6, IL-8, IL-10, CCL2, CCL5, CXCL10, IL-18, TNFα, MCP-1, MIP-1β, IP10 and GM-CSF.

6. A p38 MAP kinase inhibitor for use according to any one of the preceding claims, wherein the p38 MAP kinase inhibitor is an inhibitor of p38α MAP kinase and/or p38 β MAP kinase.

7. A p38 MAP kinase inhibitor for use according to any one of the preceding claims, wherein the p38 MAP kinase inhibitor is of Formula I, or a pharmaceutically acceptable salt or solvate thereof: 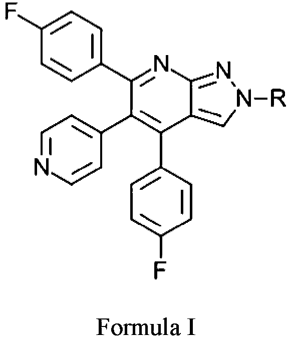 wherein R is C₁₋₃ alkyl, optionally substituted by one or more halo, NR¹R² or hydroxyl, and R¹ and R² are independently H, halo or C₁₋₃ alkyl, optionally substituted by one or more F.

8. A p38 MAP kinase inhibitor for use according toclaim 7, wherein the p38 MAP kinase inhibitor is of Formula II, or a pharmaceutically acceptable salt or solvate thereof:

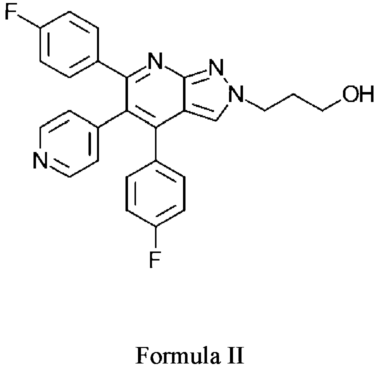

9. A p38 MAP kinase inhibitor for use according to any one of the preceding claims, wherein the p38 MAP kinase inhibitor is administered prior to administration of the cancer immunotherapy.

10. A p38 MAP kinase inhibitor for use according to any one of the preceding claims, wherein the p38 MAP kinase inhibitor is administered beginning about 4 days to about 2 hours before administration of the cancer immunotherapy, or about 1 to about 3 days before the onset of CRS, or
wherein the p38 MAP kinase inhibitor is administered over a period of about 7 to about 28 days; or wherein the p38 MAP kinase inhibitor is administered orally BID.

11. A p38 MAP kinase inhibitor for use according to claim 10, wherein the p38 MAP kinase inhibitor is administered beginning about 4 days to about 2 hours before administration of the cancer immunotherapy, or about 1 to about 3 days before the onset of CRS, and wherein a steady state blood plasma concentration of the p38 MAPK inhibitor is reached by the time of administration of the cancer immunotherapy, or by the time of the onset of CRS.

12. A p38 MAP kinase inhibitor for use according to any one of the preceding claims, wherein the p38 MAP kinase inhibitor prevents an infusion-related hypersensitivity response or reduces the severity of an infusion-related hypersensitivity response.

13. A p38 MAP kinase inhibitor for use according to any one of the preceding claims, wherein the p38 MAP kinase inhibitor is administered orally at about 5 mg BID to about 1000 mg BID.

14. A p38 MAP kinase inhibitor for use according to any one of the preceding claims, wherein the p38 MAP kinase inhibitor is administered at a dosage of about 10 mg BID to about 200 mg BID, such as at a dosage of 30 mg BID, 70 mg BID, 110 mg BID or 150 mg BID.

15. A p38 MAP kinase inhibitor for use according to any one of the preceding claims, wherein the p38 MAP kinase inhibitor is administered beginning about 4 days to about 2 hours before administration of the cancer immunotherapy, until an end-point of about 15 days after administration of the cancer immunotherapy.

16. A p38 MAP kinase inhibitor of Formula II, or a pharmaceutically acceptable salt or solvate thereof: 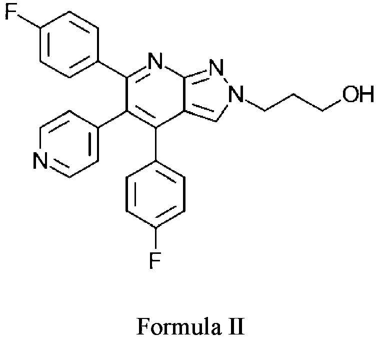 for use in the prevention or treatment of cancer immunotherapy-associated CRS in a human patient.

17. A pharmaceutical composition comprising a p38 MAP kinase inhibitor or a pharmaceutically acceptable salt or solvate thereof, for use in preventing or reducing the severity of signs or symptoms of cancer immunotherapy-associated cytokine release syndrome ("CRS") in a human patient, wherein the pharmaceutical composition is administered *in vivo* prior to the onset of CRS.

## Patentansprüche

1. Ein p38-MAP-Kinase-Inhibitor zur Verwendung bei der Verhinderung oder Verringerung der Schwere von Anzeichen oder Symptomen von mit Krebsimmuntherapie verbundenem Zytokin-Freisetzungssyndrom ("CRS") bei einem menschlichen Patienten, wobei der p38-MAP-Kinase-Inhibitor vor dem Auftreten von CRS verabreicht wird.

2. Ein p38-MAP-Kinase-Inhibitor zur Verwendung gemäß Anspruch 1, wobei die Verhinderung oder Verringerung der Schwere von Anzeichen oder Symptomen von mit Krebsimmuntherapie verbundenem CRS bei einem menschlichen Patienten das Verhindern oder Verringern der Schwere von Fieber ist oder umfasst.

3. Ein p38-MAP-Kinase-Inhibitor zur Verwendung gemäß Anspruch 1 oder Anspruch 2, wobei die Krebsimmuntherapie ist: T-Zell-Engager-Therapie, TIL-Therapie, CAR-NK-Zell-Therapie, ein Tumorvakzin, eine T-Zell-Checkpoint-Modulator-Therapie oder eine CAR-T-Zell-Therapie.

4. Ein p38-MAP-Kinase-Inhibitor zur Verwendung gemäß Anspruch 3, wobei die T-Zell-Engager-Therapie ausgewählt ist aus BiTE-Therapie, einer Therapie mit einem weiteren bispezifischen Antikörper, CiTE-Therapie, SMITe-Therapie und TriKE-Therapie.

5. Ein p38-MAP-Kinase-Inhibitor zur Verwendung gemäß einem der vorhergehenden Ansprüche, wobei der p38-MAP-Kinase-Inhibitor die Freisetzung aus Monozyten proinflammatorischer Mediatoren inhibiert, einschließlich einem oder mehreren von: IFNγ, IL-1β, IL-2, IL-6, IL-8, IL-10, IL-12, IL-18, CCL2, CCL5, CXCL10, MCP-1, MIP-1β, GM-CSF, VEGF und TNFα, und/oder wobei der p38-MAP-Kinase-Inhibitor die Freisetzung aus Endothelzellen proinflammatorischer Mediatoren inhibiert, einschließlich einem oder mehrere von: IL-β, IL-2, IL-6, IL-8, IL-10, CCL2, CCL5, CXCL10, IL-18, TNFα, MCP-1, MIP-1β, IP10 und GM-CSF.

6. Ein p38-MAP-Kinase-Inhibitor zur Verwendung gemäß einem der vorhergehenden Ansprüche, wobei der p38-MAP-Kinase-Inhibitor ein Inhibitor von p38-α-MAP-Kinase und/oder p38-β-MAP-Kinase ist.

7. Ein p38-MAP-Kinase-Inhibitor zur Verwendung gemäß einem der vorhergehenden Ansprüche, wobei der p38-MAP-Kinase-Inhibitor die Formel I besitzt oder ein pharmazeutisch annehmbares Salz oder Solvat davon ist: 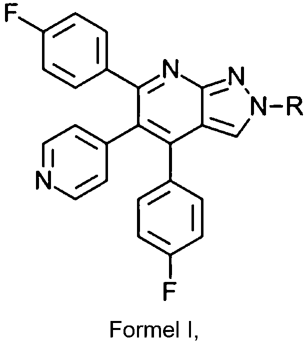 wobei R C₁₋₃-Alkyl ist, gegebenenfalls durch ein oder mehrere Halogene, NR¹R² oder Hydroxyl substituiert, und R¹ und R² unabhängig H, Halogen oder C₁₋₃-Alkyl, unabhängig durch ein oder mehrere F substituiert, sind.

8. Ein p38-MAP-Kinase-Inhibitor zur Verwendung gemäß Anspruch 7, wobei der p38-MAP-Kinase-Inhibitor die Formel II besitzt oder ein pharmazeutisch annehmbares Salz oder Solvat davon ist:

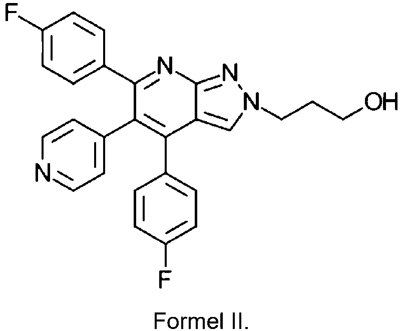

9. Ein p38-MAP-Kinase-Inhibitor zur Verwendung gemäß einem der vorhergehenden Ansprüche, wobei der p38-MAP-Kinase-Inhibitor vor der Verabreichung der Krebsimmuntherapie verabreicht wird.

10. Ein p38-MAP-Kinase-Inhibitor zur Verwendung gemäß einem der vorhergehenden Ansprüche, wobei der p38-MAP-Kinase-Inhibitor erstmalig etwa 4 Tage bis etwa 2 Stunden vor der Verabreichung der Krebsimmuntherapie oder etwa 1 bis etwa 3 Tage vor dem Auftreten von CRS verabreicht wird, oder
wobei der p38-MAP-Kinase-Inhibitor über einen Zeitraum von etwa 7 bis etwa 28 Tagen verabreicht wird, oder wobei der p38-MAP-Kinase-Inhibitor oral BID verabreicht wird.

11. Ein p38-MAP-Kinase-Inhibitor zur Verwendung gemäß Anspruch 10, wobei der p38-MAP-Kinase-Inhibitor erstmalig etwa 4 Tage bis etwa 2 Stunden vor der Verabreichung der Krebsimmuntherapie oder etwa 1 bis etwa 3 Tage vor dem Auftreten von CRS verabreicht wird, und wobei zum Zeitpunkt der Verabreichung der Krebsimmuntherapie oder zum Zeitpunkt des Auftretens von CRS eine Steady-State-Blutplasmakonzentration des p38-MAPK-Inhibitors erreicht wird.

12. Ein p38-MAP-Kinase-Inhibitor zur Verwendung gemäß einem der vorhergehenden Ansprüche, wobei der p38-MAP-Kinase-Inhibitor eine infusionsbedingte Überempfindlichkeitsreaktion verhindert oder die Schwere einer infusionsbedingten Überempfindlichkeitsreaktion verringert.

13. Ein p38-MAP-Kinase-Inhibitor zur Verwendung gemäß einem der vorhergehenden Ansprüche, wobei der p38-MAP-Kinase-Inhibitor oral in einer Dosierung von etwa 5 mg BID bis etwa 1000 mg BID verabreicht wird.

14. Ein p38-MAP-Kinase-Inhibitor zur Verwendung gemäß einem der vorhergehenden Ansprüche, wobei der p38-MAP-Kinase-Inhibitor in einer Dosierung von etwa 10 mg BID bis etwa 200 mg BID, wie z.B. in einer Dosierung von 30 mg BID, 70 mg BID, 110 mg BID oder 150 mg BID, verabreicht wird.

15. Ein p38-MAP-Kinase-Inhibitor zur Verwendung gemäß einem der vorhergehenden Ansprüche, wobei der p38-MAP-Kinase-Inhibitor erstmalig etwa 4 Tage bis etwa 2 Stunden vor der Verabreichung der Krebsimmuntherapie verabreicht wird, bis zu einem Endpunkt von etwa 15 Tagen nach Verabreichung der Krebsimmuntherapie.

16. Ein p38-MAP-Kinase-Inhibitor der Formel II oder ein pharmazeutisch annehmbares Salz oder Solvat davon: 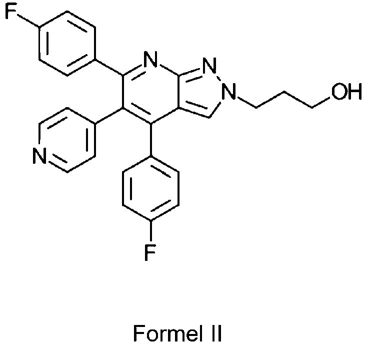 zur Verwendung bei der Verhinderung oder Behandlung von mit Krebsimmuntherapie verbundenem CRS bei einem menschlichen Patienten.

17. Eine pharmazeutische Zusammensetzung, die einen p38-MAP-Kinase-Inhibitor oder ein pharmazeutisch annehmbares Salz oder Solvat davon umfasst, zur Verwendung bei der Verhinderung oder Verringerung der Schwere von Anzeichen oder Symptomen von mit Krebsimmuntherapie verbundenem Zytokin-Freisetzungssyndrom ("CRS") bei einem menschlichen Patienten, wobei die pharmazeutische Zusammensetzung *in vivo* vor dem Auftreten von CRS verabreicht wird.

## Revendications

1. Inhibiteur de la p38 MAP kinase pour une utilisation dans la prévention ou la réduction de la gravité des signes ou symptômes du syndrome de relargage de cytokines (CRS pour « cytokine release syndrome ») associé à l'immunothérapie anticancéreuse chez un patient humain, l'inhibiteur de la p38 MAP kinase étant administré avant l'apparition du CRS.

2. Inhibiteur de la p38 MAP kinase pour une utilisation selon la revendication 1, la prévention ou la réduction de la gravité des signes ou symptômes du CRS associé à l'immunothérapie anticancéreuse chez un patient humain consistant en ou comprenant la prévention ou la réduction de la gravité de la fièvre.

3. Inhibiteur de la p38 MAP kinase pour une utilisation selon la revendication 1 ou la revendication 2, l'immunothérapie anticancéreuse étant : une thérapie mobilisant les cellules T ; une thérapie TIL ; une thérapie par cellules CAR-NK ; un vaccin anticancéreux ; une thérapie par modulateur de point de contrôle des cellules T ; ou une thérapie par cellules CAR-T.

4. Inhibiteur de la p38 MAP kinase pour une utilisation selon la revendication 3, la thérapie mobilisant les cellules T étant choisie parmi la thérapie BiTE, la thérapie avec un autre anticorps bispécifique, la thérapie CiTE, la thérapie SMITe et la thérapie TriKE.

5. Inhibiteur de la p38 MAP kinase pour une utilisation selon l'une quelconque des revendications précédentes, l'inhibiteur de la p38 MAP kinase inhibant le relargage de médiateurs pro-inflammatoires à partir des monocytes, comprenant un ou plusieurs parmi : IFNγ, IL-1β, IL-2, IL-6, IL-8, IL-10, IL-12, IL-18, CCL2, CCL5, CXCL10, MCP-1, MIP-1β, GM-CSF, VEGF et TNFα; et/ou l'inhibiteur de la p38 MAP kinase inhibant le relargage de médiateurs pro-inflammatoires à partir des cellules endothéliales, comprenant un ou plusieurs parmi : IL-1β, IL-2, IL-6, IL-8, IL-10, CCL2, CCL5, CXCL10, IL-18, TNFα, MCP-1, MIP-1β, IP10 et GM-CSF.

6. Inhibiteur de la p38 MAP kinase pour une utilisation selon l'une quelconque des revendications précédentes, l'inhibiteur de la p38 MAP kinase étant un inhibiteur de la p38α MAP kinase et/ou de p38β MAP kinase.

7. Inhibiteur de la p38 MAP kinase pour une utilisation selon l'une quelconque des revendications précédentes, l'inhibiteur de la p38 MAP kinase répondant à la Formule I ou étant l'un des ses sels ou solvates pharmaceutiquement acceptables : 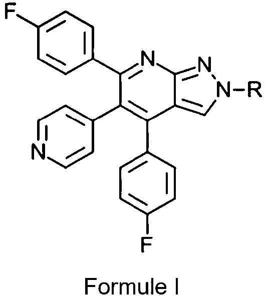 dans laquelle R représente un groupe alkyle en C₁ à C₃, facultativement substitué par un ou plusieurs groupes halogéno, NR¹R² ou hydroxyle, et R¹ et R² représentent indépendamment H, un groupe halogéno ou alkyle en C₁ àC₃, facultativement substitué par un ou plusieurs F.

8. Inhibiteur de la p38 MAP kinase pour une utilisation selon la revendication 7, l'inhibiteur de la p38 MAP kinase répondant à la Formule II ou étant l'un des ses sels ou solvates pharmaceutiquement acceptables :

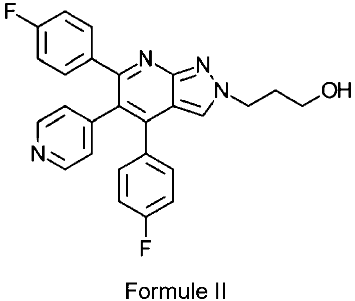

9. Inhibiteur de la p38 MAP kinase pour une utilisation selon l'une quelconque des revendications précédentes, l'inhibiteur de la p38 MAP kinase étant administré avant l'administration de l'immunothérapie anticancéreuse.

10. Inhibiteur de la p38 MAP kinase pour une utilisation selon l'une quelconque des revendications précédentes, l'inhibiteur de la p38 MAP kinase étant administré en commençant environ 4 jours à environ 2 heures avant l'administration de l'immunothérapie anticancéreuse, ou environ 1 à environ 3 jours avant l'apparition du CRS, ou
l'inhibiteur de la p38 MAP kinase étant administré sur une période d'environ 7 à environ 28 jours ; ou l'inhibiteur de la p38 MAP kinase étant administré par voie orale BID.

11. Inhibiteur de la p38 MAP kinase pour une utilisation selon la revendication 10, l'inhibiteur de la p38 MAP kinase étant administré en commençant entre environ 4 jours et environ 2 heures avant l'administration de l'immunothérapie anticancéreuse, ou entre environ 1 et environ 3 jours avant l'apparition du CRS, et une concentration plasmatique à l'état d'équilibre de l'inhibiteur de la p38 MAP kinase étant atteinte au moment de l'administration de l'immunothérapie anticancéreuse, ou au moment de l'apparition du CRS.

12. Inhibiteur de la p38 MAP kinase pour une utilisation selon l'une quelconque des revendications précédentes, l'inhibiteur de la p38 MAP kinase prévenant une réaction d'hypersensibilité liée à la perfusion ou réduisant la gravité d'une réaction d'hypersensibilité liée à la perfusion.

13. Inhibiteur de la p38 MAP kinase pour une utilisation selon l'une quelconque des revendications précédentes, environ 5 mg BID à environ 1000 mg BID de l'inhibiteur de la p38 MAP kinase étant administrés par voir orale.

14. Inhibiteur de la p38 MAP kinase pour une utilisation selon l'une quelconque des revendications précédentes, l'inhibiteur de la p38 MAP kinase étant administré à une posologie d'environ 10 mg BID à environ 200 mg BID, par exemple à une posologie de 30 mg BID, 70 mg BID, 110 mg BID ou 150 mg BID.

15. Inhibiteur de la p38 MAP kinase pour une utilisation selon l'une quelconque des revendications précédentes, l'inhibiteur de la p38 MAP kinase étant administré en commençant environ 4 jours à environ 2 heures avant l'administration de l'immunothérapie anticancéreuse jusqu'à un point final environ 15 jours après l'administration de l'immunothérapie anticancéreuse.

16. Inhibiteur de la p38 MAP kinase de Formule II, ou un des ses sels ou solvates pharmaceutiquement acceptables : 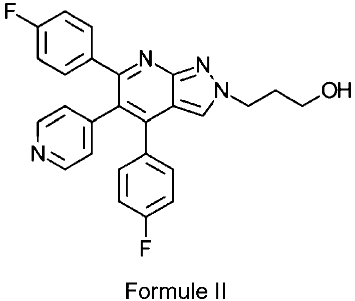 pour une utilisation dans la prévention ou le traitement du CRS associé à l'immunothérapie anticancéreuse chez un patient humain.

17. Composition pharmaceutique comprenant un inhibiteur de la p38 MAP kinase ou un des ses sels ou solvates pharmaceutiquement acceptables, pour une utilisation dans la prévention ou la réduction de la gravité des signes ou symptômes du syndrome de relargage de cytokines (CRS pour « cytokine release syndrome ») associé à l'immunothérapie anticancéreuse chez un patient humain, la composition pharmaceutique étant administrée in *vivo* avant l'apparition du CRS.
